# EUROPÄISCHE PATENTSCHRIFT

(11) **EP 1 599 455 B1**
(45) Veröffentlichungstag und Bekanntmachung des Hinweises auf die Patenterteilung: **01.07.2009**
(21) Anmeldenummer: 04712503.4
(22) Anmeldetag: 19.02.2004
(51) Int. Cl.: C07D 263/32, C07D 413/14, A61K 31/421, A61K 31/422, A61P 3/10, A61P 9/10

(54) **4-(3- (2-PHENYL-OXAZOL-4-YLMETHOXY)-CYCLOHEXYLOXY)-BUTANSÄURE DERIVATE UND VERWANDTE VERBINDUNGEN ALS PPAR MODULATOREN ZUR BEHANDLUNG VON TYP 2 DIABETES UND ATHEROSKLEROSE**
4-(3- (2-PHENYL-OXAZOL-4-YLMETHOXY)-CYCLOHEXYLOXY)-BUTANE ACID DERIVATIVES AND RELATED COMPOUNDS AS PPAR MODULATORS FOR TREATING DIABETES OF TYPE 2 AND ATHEROSCLEROSIS
DERIVES D'ACIDE 4-(3-(2-PHENYL-OXAZOL-4-YLMETHOXY)-CYCLOHEXYLOXY)-BUTANOIQUE ET COMPOSES APPARENTES EN TANT QUE MODULATEURS DE PPAR DANS LE TRAITEMENT DU DIABETE DE TYPE 2 ET DE L'ATHEROSCLEROSE

(30) Priorität: 27.02.2003 DE 10308355
(43) Veröffentlichungstag der Anmeldung: 30.11.2005
(73) Patentinhaber: Sanofi-Aventis Deutschland GmbH, 65929 Frankfurt am Main (DE)
(72) Erfinder: STAPPER, Christian, 55518 Mainz (DE); KEIL, Stefanie, 65719 Hofheim (DE); GLOMBIK, Heiner, 65719 Hofheim (DE); FALK, Eugen, 60529 Frankfurt (DE); GOERLITZER, Jochen, 60594 Frankfurt am Main (DE); GRETZKE, Dirk, 60596 Frankfurt (DE); SCHAEFER, Hans-Ludwig, 65239 Hochheim (DE); WENDLER, Wolfgang, 65618 Selters (DE)
(86) Internationale Anmeldenummer: PCT/EP2004/001586
(87) Internationale Veröffentlichungsnummer: WO 2004/076428

(56) Entgegenhaltungen:
- WO-A-00/64876
- WO-A-02/059098

## Beschreibung

Die Erfindung betrifft Aryl-cycloalkyl substituierte Alkansäurederivate sowie deren physiologisch verträgliche Salze.

Es sind bereits strukturähnliche Verbindungen zur Behandlung von Hyperlipidämie und Diabetes im Stand der Technik beschrieben (WO 2000/64876).

Der Erfindung lag die Aufgabe zu Grunde, Verbindungen zur Verfügung zu stellen, die eine therapeutisch verwertbare Modulation des Lipid- und/oder Kohlehydratstoffwechsels erlauben und sich somit zur Prävention und/oder Behandlung von Krankheiten wie Typ 2 Diabetes und Atherosklerose sowie deren vielfältigen Folgeerkrankungen eignen.

Überraschenderweise wurde eine Serie von Verbindungen gefunden, die die Aktivität von PPAR Rezeptoren modulieren. Insbesondere eignen sich die Verbindungen zur Aktivierung von PPARalpha und PPARgamma, wobei das Ausmaß der relativen Aktivierung je nach Verbindungen unterschiedlich sein kann.

Die Erfindung betrifft daher Verbindungen der Formel I 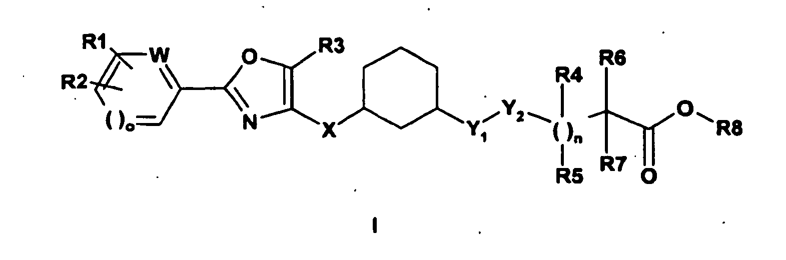 worin bedeuten:
- R1, R2: unabhängig voneinander H, F, Cl, Br, CF₃, OCF₃, (C1-C6)-Alkyl, O-(C1-C6)-Alkyl, SCF₃, SF₅, OCF₂-CHF₂, (C6-C10)-Aryl, (C6-C10)-Aryloxy, OH, NO₂; oder
- R1 und R2: zusammengenommen mit dem Phenyl-, Pyridin-, 1-H-Pyrrol-, Thiophen- oder Furan-Ring kondensiertes, teilweise oder nicht gesättigtes bicyclisches (C6-C10)-Aryl, (C5-C11)-Heteroaryl;
- R3: H, (C1-C6)-Alkyl, (C3-C8)-Cycloalkyl, (C1-C3)-Alkyl-(C3-C8)-Cycloalkyl, Phenyl, (C1-C3)-Alkyl-Phenyl, (C5-C6)-Heteroaryl, (C1-C3)-Alkyl-(C5-C6)-Heteroaryl oder (C1-C3)-Alkyl, das durch F ganz oder teilweise substituiert ist;
- o: 0 oder 1;
- W: CH, N, falls o = 1;
- W: O, S, NR9, falls o = 0;
- X: (C1-C6)-Alkandiyl, wobei in der Alkandiylgruppe ein oder mehrere Kohlenstoffatome durch Sauerstoffatome ersetzt sein können;
- Y1: O;
- Y2: CR12R13, SO, SO2;
- n: 0 - 2;
- R4: H, F, (C1-C6)-Alkyl;
- R5: H, F, (C1-C6)-Alkyl;
- R6: H, (C1-C6)-Alkyl; oder F, falls n ungleich 0;
- R7: H, (C1-C6)-Alkyl, (C2-C6)-Alkenyl, (C2-C6)-Alkinyl, O-(C1-C6)-Alkyl, O-(C2-C6)-Alkenyl, O-(C2-C6)-Alkinyl, (C3-C8)-Cycloalkyl, Phenyl, (C5-C11)-Heteroaryl, O-(C3-C8)-Cycloalkyl, O-Phenyl, die substituiert sein können durch OH, NR10R11, (C1-C6)-Alkyl, (C2-C6)-Alkenyl, (C2-C6)-Alkinyl, O-(C3-C8)-Cycloalkyl, O-Phenyl, O-(C5-C11)-Heteroaryl, und wobei (C3-C8)-Cycloalkyl, Phenyl, (C5-C11)-Heteroaryl zusätzlich substituiert sein können durch (C1-C6)-Alkyl gegebenenfalls ganz oder teilweise substituiert durch F, O-(C1-C6)-Alkyl gegebenenfalls ganz oder teilweise substituiert durch F, Cl, Br, J, OH, NR10R11, CO-(C1-C6)-Alkyl, CO-(C6-C10)-Aryl, CO-(C1-C6)-Alkyl-(C6-C10)-Aryl, CO-(C5-C11)-Heteroaryl, C(O)-O-(C1-C6)-Alkyl, C(O)-O-(C1-C6)-Alkyl-(C6-C10)-Aryl, C(O)-O-(C6-C10)-Aryl, C(O)-O-(C5-C11)-Heteroaryl, SO2-(C1-C6)-Alkyl, SO2-(C1-C6)-Alkyl-(C6-C10)-Aryl, SO2-(C1-C6)-Alkyl-SO2-(C1-C6)-alkyl, SO2-(C6-C10)Aryl, SO2-(C5-C11)-Heteroaryl;
- R6 und R7: zusammen mit dem sie tragenden C-Atom (C3-C8)-Cycloalkyl;
- R8: H, (C1-C6)-Alkyl;
- R9: H, (C1-C6)-Alkyl, das gegebenenfalls substituiert ist durch Phenyl;
- R10: H, (C1-C6)-Alkyl, das gegebenenfalls substituiert ist durch Phenyl;
- R11: H, (C1-C6)-Alkyl, das gegebenenfalls substituiert ist durch Phenyl;
- R12: H, (C1-C6)-Alkyl;
- R13: H, (C1-C6)-Alkyl;
sowie deren physiologisch verträgliche Salze.

Bevorzugt sind Verbindungen der Formel I worin bedeuten:
- X: (C1-C6)-Alkandiyl, wobei in der Alkandiylgruppe das C1- oder C2-Kohlenstoffatom (zum Cyclohexandiyl-Ring) durch Sauerstoffatom ersetzt ist;
sowie deren physiologisch verträgliche Salze.

Besonders bevorzugt sind die Verbindungen der Formel I, worin ein oder mehrere Reste die folgenden Bedeutungen haben:
- R1, R2: unabhängig voneinander H, F, CF3, (C1-C6)-Alkyl, O-(C1-C6)-Alkyl, Phenyl, oder
- R1 und R2: zusammengenommen mit dem Phenylring = Naphthyl;
- R3: (C1-C6)-Alkyl;
- W: CH, falls o = 1;
- X: (CH2)O, CH2-O-CH2;
- Y1: O;
- Y2: CH2;
- n: 0, 1;
- R4: H;
- R5: H;
- R6: H;
- R7: H, (C1-C6)-Alkyl, O-(C1-C6)-Alkyl, (C1-C6)-Alkyl-O-(C1-C6)-Alkyl, O-(C2-C6)-Alkenyl, O-(C2-C6)-Alkinyl, CH2NR10R11, wobei Alkyl, O-Alkyl und Alkenyl substituiert sein kann durch Phenyl oder (C5-C6)-Heteroaryl, die wiederum substituiert sein können durch (C1-C6)-Alkyl, O-(C1-C6)-Alkyl, CF3;
- R6 und R7: zusammen mit dem sie tragenden C-Atom (C3-C6)-Cycloalkyl;
- R8: H;
- R10: (C1-C6)-Alkyl;
- R11: (C1-C6)-Alkyl, das durch Phenyl substituiert ist;
sowie deren physiologisch verträgliche Salze.

Die Alkyl-, Alkenyl, Alkinyl-Reste in den Substituenten R1, R2, R3, R4, R5 , R6, R7, R8, R9, R10, R11, R12 und R13 können sowohl geradkettig wie verzweigt sein.

Unter Aryl wird ein aromatisches carbocyclisches mono- oder bicyclisches RingSystem verstanden, das 6 bis 10 Atome im Ring oder in den Ringen enthält.

Heteroaryl ist ein mono- oder bicyclisches aromatisches Ringsystem mit 4 bis 11 Ringgliedern, worin mindestens ein Atom im Ringsystem ist ein Heteroatom aus der Reihe N, O und S.

Die Verbindungen der Formel I enthalten mindestens zwei Assymmetriezentren und können darüber hinaus mehr enthalten. Daher können die Verbindungen der Formel I in Form ihrer Racemate, racemischen Mischungen, reinen Enantiomere, Diastereomere und Diastereomer-Mischungen vorliegen. Die vorliegende Erfindung umfasst alle diese isomeren Formen der Verbindungen der Formel I. Diese Isomeren Formen können, wenn auch zum Teil nicht expressis verbis beschrieben, nach bekannnten Methoden erhalten werden.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der erfindungsgemäßen Verbindungen sind Salze anorganischer Säuren, wie Salzsäure, Bromwasserstoff-, Phosphor-, Metaphosphor-, Salpeter- und Schwefelsäure sowie organischer Säuren, wie z.B. Essigsäure, Benzolsulfon-, Benzoe-, Zitronen-, Ethansulfon-, Fumar-, Glucon-, Glykol-, Isethion-, Milch-, Lactobion-, Malein-, Äpfel-, Methansulfon-, Bernstein-, p-Toluolsulfon- und Weinsäure. Geeignete pharmazeutisch verträgliche basische Salze sind Ammoniumsalze, Alkalimetallsalze (wie Natrium- und Kaliumsalze) und Erdalkalisalze (wie Magnesium- und Calciumsalze), Trometamol (2-Amino-2-hydroxymethyl-1,3-propandiol), Diethanolamin, Lysin oder Ethylendiamin.

Salze mit einem nicht pharmazeutisch verträglichen Anion, wie zum Beispiel Trifluoracetat, gehören ebenfalls in den Rahmen der Erfindung als nützliche Zwischenprodukte für die Herstellung oder Reinigung pharmazeutisch verträglicher Salze und/oder für die Verwendung in nicht-therapeutischen, zum Beispiel in-vitro-Anwendungen.

Der hier verwendete Begriff "physiologisch funktionelles Derivat" bezeichnet jedes physiologisch verträgliche Derivat einer erfindungsgemäßen Verbindung der Formel I, z.B. einen Ester, der bei Verabreichung an einen Säuger, wie z.B. den Menschen, in der Lage ist, (direkt oder indirekt) eine Verbindung der Formel I oder einen aktiven Metaboliten hiervon zu bilden.

Zu den physiologisch funktionellen Derivaten zählen auch Prodrugs der erfindungsgemäßen Verbindungen, wie zum Beispiel in H. Okada et al., Chem. Pharm. Bull. 1994, 42, 57-61 beschrieben. Solche Prodrugs können in vivo zu einer erfindungsgemäßen Verbindung metabolisiert werden. Diese Prodrugs können selbst wirksam sein oder nicht.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Nachfolgend beziehen sich alle Verweise auf "Verbindung(en) gemäß Formel I" auf Verbindung(en) der Formel I, wie vorstehend beschrieben, sowie ihre Salze und Solvate wie hierin beschrieben.

### Verwendung

Diese Erfindung bezieht sich weiterhin auf die Verwendung von Verbindungen der Formeln I und ihren pharmazeutischen Zusammensetzungen als PPAR-Rezeptor-Liganden. Die erfindungsgemäßen PPAR-Rezeptor-Liganden eignen sich als Modulatoren der Aktivität der PPAR Rezeptoren.
Peroxisomen-Proliferatoren-Aktivierte Rezeptoren (PPAR) sind durch Liganden aktivierbare Transkriptionsfaktoren, die zur Klasse der Kern-Hormon-Rezeptoren gehören. Es existieren drei PPAR Isoformen, PPARalpha, PPARgamma und PPARdelta, die von verschiedenen Genen kodiert werden (Peroxisome proliferator-activated receptor (PPAR): structure, mechanisms of activation and diverse functions: Motojima K, Cell Struct Funct. 1993 Oct; 18(5): 267-77).
Von PPARgamma existieren zwei Varianten, PPARgamma₁ und gamma₂, die das Ergebnis eines alternativen Einsatzes von Promotoren und einer differenziellen mRNA-Spleißung (Vidal-Puig et al. J. Clin. Invest., 97:2553-2561, 1996) sind. Die verschiedenen PPAR Rezeptoren besitzen eine unterschiedliche Gewebsverteilung und modulieren unterschiedliche physiologische Funktionen. Die PPAR-Rezeptoren spielen eine Schlüsselrolle bei unterschiedlichen Aspekten der Regulation einer Vielzahl von Genen, deren Genprodukte direkt oder indirekt am Lipid- und Kohlehydratstoffwechsel entscheidend beteiligt sind. So spielen z. B. PPARalpha Rezeptoren bei der Regulation des Fettsäurekatabolismus oder des Lipoproteinstoffwechsels in der Leber eine wichtige Rolle, während PPARgamma zum Beispiel bei der Regulation der Fettzelldifferenzierung entscheidend beteiligt ist. Darüberhinaus sind PPAR Rezeptoren aber auch an der Regulation vieler weiterer physiologischer Prozesse, auch solcher die nicht direkt mit dem Kohlehydrat- oder Lipidstoffwechsel in Verbindung stehen, beteiligt. Die Aktivität der unterschiedlichen PPAR Rezeptoren kann durch verschiedene Fettsäuren, Fettsäurederivate sowie synthetische Verbindungen in unterschiedlichem Ausmaß moduliert werden. Entsprechende Reviews über Funktionen, physiologische Wirkung und Pathophysiologie siehe: Joel Berger et al., Annu. Rev. Med. 2002, 53, 409 - 435; Timothy Wilson et al. J. Med. Chem., 2000, Vol. 43, No. 4, 527-550; Steven Kliewer et al., Recent Prog Horm Res. 2001; 56: 239-63.
Die vorliegende Erfindung betrifft Verbindungen der Formel I, die sich zur Modulierung der Aktivität von PPAR Rezeptoren eignen, insbesondere der Aktivität von PPARalpha und PPARgamma. Je nach Profil der Modulation sind die Verbindungen der Formel I zur Behandlung, Kontrolle und Prohylaxe nachfolgend beschriebener Indikationen, sowie einer Reihe anderer, damit verbundener pharmazeutischer Anwendungen geeignet (siehe z.B. Joel Berger et al., Annu. Rev. Med. 2002, 53, 409 - 435; Timothy Wilson et al. J. Med. Chem., 2000, Vol. 43, No. 4, 527-550; Steven Kliewer et al., Recent Prog Horm Res. 2001; 56: 239-63; Jean-Charles Fruchart, Bart Staels and Patrick Duriez: PPARS, Metabolic Disease and Arteriosclerosis, Pharmacological Research, Vol. 44, No. 5, 2001; Sander Kersten, Beatrice Desvergne & Walter Wahli: Roles of PPARs in health and disease, NATURE, VOL 405 ,25 MAY 2000 ; Ines Pineda Torra, Giulia Chinetti, Caroline Duval, Jean-Charles Fruchart and Bart Stapels: Peroxisome proliferator-activated receptors: from transcriptional control to clinical practice, Curr Opin Lipidol 12: 2001, 245-254).
Besonders geeignet sind solche Verbindungen zur Behandlung und/oder Prävention von
1.
   - Störungen des Fettsäurestoffwechsels und Glucoseverwertungsstörungen
   - Störungen, bei denen Insulin Resistenz eine Rolle spielt
2. Diabetes mellitus, insbesondere Typ 2 Diabetes einschließlich der Verhinderung der damit verbundenen Folgeerkrankungen.
   Besondere Aspekte sind dabei die
   - Hyperglykämie,
   - Verbesserung der Insulinresistenz,
   - Verbesserung der Glucose-Toleranz,
   - Schutz der β-Zellen der Bauchspeicheldrüse
   - Verhinderung makro- und mikrovaskulärer Erkrankungen
3. Dyslipidämien und deren Folgen, wie z.B. Atherosklerose, koronare Herzkrankheit, zerebrovaskuläre Erkrankungen etc, insbesondere solche (aber nicht beschränkt auf), die durch einen oder mehrerer folgender Faktoren charakterisiert sind:
   - hohe Plasma-Triglycerid-, hohe postprandiale Plasma-Triglycerid-Konzentrationen
   - niedrige HDL-Cholesterin Konzentration
   - niedrige ApoA Lipoprotein-Konzentrationen
   - hohe LDL-Cholesterin Konzentrationen
   - kleine dichte LDL-Cholesterin Partikel
   - hohe ApoB Lipoprotein-Konzentrationen
4. Verschiedene andere Zuständen, die mit dem Metabolischen Syndrom assoziert sein können, sind wie:
   - Adipositas (Fettsucht), einschließlich abdominale Adipositas
   - Thrombosen, Stadien von Hyperkoagulabilität und Thromboseneigung (arteriell und venös)
   - Hoher Blutdruck
   - Herzinsuffizienz, wie z.B. (aber nicht beschränkt auf) bei Zustand nach Myokardinfarkt, hypertensive Herzerkrankung oder Kardiomyopathie
5. weitere Krankheiten oder Zustände bei welchen zum Beispiel entzündliche Reaktionen oder die Zelldifferenzierung eine Rolle spielt sind:
   - Atherosklerose wie z.B. (aber nicht beschränkt auf) Koronarsklerose einschl. Angina pectoris oder Herzinfarkt, Hirnschlag
   - Vaskuläre Restenose oder Reverschluß
   - Chronisch entzündliche Darmerkrankungen, wie z.B. Morbus Crohn und Colitis ulcerosa
   - Pankreatitis
   - Andere entzündliche Zustände
   - Retinopathie
   - Fettzell-Tumore (adipose cell tumors)
   - Fettzell-Karzimone, wie z.B. Liposarkome
   - solide Tumoren und Neoplasien, wie z.B. (aber nicht beschränkt auf) Karzinome des Magen-Darm Traktes, der Leber, der Gallenwege und des Pankreas, endokrine Tumore, Karzinome der Lunge, der Niere und hamableitenden Organe, des Genitaltraktes, Prostata-Karzinome etc.
   - akute und chronische myeloprolifeative Erkrankungen und Lymphome
   - Angiogenese
   - Neurodegenerative Erkrankungen
   - Alzheimersche Krankheit
   - Multiple Sklerose
   - Morbus Parkinson
   - Erythemato-squamöse Dermatosen, wie z.B. Psoriasis (Schuppenflechte)
   - Akne vulgaris
   - Andere Hautkrankheiten und dermatologische Zustände, die durch PPAR moduliert werden
   - Ekzeme und Neurodermitis
   - Dermatitiden, wie z.B. seborrhoische Dermatitis oder Lichtdermatitis
   - Keratitis und Keratosen, wie z.B. seborrhoische Keratosen, senile Keratosen, aktinische Keratose, photo-induzierte Keratosen oder Keratosis follicularis
   - Keloide und Keloid-Prophylaxe
   - Warzen, einschließlich Kondylomata oder Kondylomata acuminata
   - Human papilloma viral (HPV) Infektionen, wie z.B. venerische Papillomata, virale Warzen, wie z.B. Molluscum contagiosum, Leukoplakie
   - Papulöse Dermatosen, wie z.B. Lichen planus
   - Hautkrebs, wie z.B. Basalzellkarzinome, Melanome oder kutane T-Zell Lymphome
   - Lokalisierte, benigne epidermale Tumore, wie z.B. Keratoderma, epidermale Naevi
   - Frostbeulen
   - Hoher Blutdruck
   - Syndrome X
   - Syndrom der polyzystischen Ovarien (PCOS)
   - Asthma
   - Osteoarthritis
   - Lupus erythematodes (LE) oder entzündliche rheumatische Erkrankungen, wie z.B. Rheumatoide Arthritis
   - Vaskulitis
   - Auszehrung (Kachexie)
   - Gicht
   - Ischämie/Reperfusions Syndrom
   - Akutes respiratorisches Distress Syndrom (ARDS) ("Schocklunge")

### Galenik

Die Menge einer Verbindung gemäß Formel I, die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im Bereich von 0,001 mg bis 100 mg (typischerweise von 0,01 mg und 50 mg) pro Tag pro Kilogramm Körpergewicht, z.B. 0,1 -10 mg/kg/Tag. Eine intravenöse Dosis kann z.B. im Bereich von 0,001 mg bis 1,0 mg/kg liegen, die geeigneterweise als Infusion von 10 ng bis 100 ng pro Kilogramm pro Minute verabreicht werden kann. Geeignete Infusionslösungen für diese Zwecke können z.B. von 0,1 ng bis 10 mg, typischerweise von 1 ng bis 10 mg pro Milliliter, enthalten. Einzeldosen können z.B. von 1 mg bis 10 g des Wirkstoffs enthalten. Somit können Ampullen für Injektionen beispielsweise von 1 mg bis 100 mg, und oral verabreichbare Einzeldosisformulierungen, wie zum Beispiel Tabletten oder Kapseln, können beispielsweise von 0.05 bis 1000 mg, typischerweise von 0,5 bis 600 mg enthalten. Zur Therapie der oben genannten Zustände können die Verbindungen gemäß Formel I selbst als Verbindung verwendet werden, vorzugsweise liegen sie jedoch mit einem verträglichen Träger in Form einer pharmazeutischen Zusammensetzung vor. Der Träger muss natürlich verträglich sein, in dem Sinne, dass er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel I. Die erfindungsgemäßen pharmazeutischen Zusammensetzungen können nach einer der bekannten pharmazeutischen Methoden hergestellt werden, die im wesentlichen darin bestehen, dass die Bestandteile mit pharmakologisch verträglichen Träger- und/oder Hilfsstoffen gemischt werden.

Erfindungsgemäße pharmazeutische Zusammensetzungen sind solche, die für orale, rektale, topische, perorale (z.B. sublinguale) und parenterale (z.B. subkutane, intramuskuläre, intradermale oder intravenöse) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel I abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Polyvinalacetatphthalat, Hydroxypropylmethylcellulosephthalat und anionische Polymere von Methacrylsäure und Methacrylsäuremethylester.

Geeignete pharmazeutische Zubereitungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel I enthalten; als Pulver oder Granulate; als Lösung oder Suspension in einer wässrigen oder nicht-wässrigen Flüssigkeit; oder als eine Öl-in-Wasser- oder Wasser-in Öl-Emulsion. Diese Zusammensetzungen können, wie bereits erwähnt, nach jeder geeigneten pharmazeutischen Methode zubereitet werden, die einen Schritt umfasst, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpresst oder geformt wird, gegebenenfalls mit einem oder mehreren zusätzlichen Bestandteilen. Gepresste Tabletten können durch Tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel I mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Geeignete pharmazeutische Zusammensetzungen für die parenterale Verabreichung umfassen vorzugsweise sterile wässrige Zubereitungen einer Verbindung gemäß Formel I, die vorzugsweise isotonisch mit dem Blut des vorgesehenen Empfängers sind. Diese Zubereitungen werden vorzugsweise intravenös verabreicht, wenngleich die Verabreichung auch subkutan, intramuskulär oder intradermal als Injektion erfolgen kann. Diese Zubereitungen können vorzugsweise hergestellt werden, indem die Verbindung mit Wasser gemischt wird und die erhaltene Lösung steril und mit dem Blut isotonisch gemacht wird. Injizierbare erfindungsgemäße Zusammensetzungen enthalten im allgemeinen von 0,1 bis 5 Gew.-% der aktiven Verbindung.

Geeignete pharmazeutische Zusammensetzungen für die rektale Verabreichung liegen vorzugsweise als Einzeldosis-Zäpfchen vor. Diese können hergestellt werden, indem man eine Verbindung gemäß Formel I mit einem oder mehreren herkömmlichen festen Trägem, beispielsweise Kakaobutter, mischt und das entstehende Gemisch in Form bringt.

Geeignete pharmazeutische Zusammensetzungen für die topische Anwendung auf der Haut liegen vorzugsweise als Salbe, Creme, Lotion, Paste, Spray, Aerosol oder Öl vor. Als Träger können Vaseline, Lanolin, Polyethylenglycole, Alkohole und Kombinationen von zwei oder mehreren dieser Substanzen verwendet werden. Der Wirkstoff ist im allgemeinen in einer Konzentration von 0,1 bis 15 Gew.-% der Zusammensetzung vorhanden, beispielsweise von 0,5 bis 2%.

Auch eine transdermale Verabreichung ist möglich. Geeignete pharmazeutische Zusammensetzungen für transdermale Anwendungen können als einzelne Pflaster vorliegen, die für einen langzeitigen engen Kontakt mit der Epidermis des Patienten geeignet sind. Solche Pflaster enthalten geeigneterweise den Wirkstoff in einer gegebenenfalls gepufferten wässrigen Lösung, gelöst und/oder dispergiert in einem Haftmittel oder dispergiert in einem Polymer. Eine geeignete Wirkstoff-Konzentration beträgt ca. 1% bis 35%, vorzugsweise ca. 3% bis 15%. Als eine besondere Möglichkeit kann der Wirkstoff, wie beispielsweise in Pharmaceutical Research, 2(6): 318 (1986) beschrieben, durch Elektrotransport oder lontophorese freigesetzt werden.

Die Verbindungen der Formeln I zeichnen sich durch günstige Wirkungen auf Stoffwechselstörungen aus. Sie beeinflussen den Fett- und Zuckerstoffwechsel positiv, sie senken insbesondere den Triglyceridspiegel und sind zur Prävention und Behandlung von Typ II Diabetes und Arteriosklerose sowie deren vielfältigen Folgeerkrankungen geeignet.

### Kombinationen mit anderen Medikamenten

Die erfindungsgemäßen Verbindungen können allein oder in Kombination mit einer oder mehreren weiteren pharmakologisch wirksamen Substanzen verabreicht werden, die beispielsweise günstige Wirkungen auf Stoffwechselstörungen oder damit häufig assoziierte Erkrankungen haben. Solche Medikamente sind zum Beispiel
1. Blutzuckersenkende Medikamente, Antidiabetika,
2. Wirkstoffe zur Behandlung von Dyslipidemien,
3. Antiatherosklerotische Medikamente,
4. Antiadiposita,
5. Antiinflammatorische Wirkstoffe
6. Wirkstoffe zur Behandlung von malignen Tumoren
7. Antithrombotische Wirkstoffe
8. Wirkstoffe zur Behandlung von Bluthochdruck
9. Wirkstoffe zur Behandlung von Herzinsuffizienz sowie
10.Wirkstoffe zur Behandlung und/oder Prävention von Komplikationen, die von Diabetes verursacht werden oder mit Diabetes assoziiert sind.

Sie können mit den erfindungsgemäßen Verbindungen der Formel I insbesondere zur synergistischen Wirkungsverbesserung kombiniert werden. Die Verabreichung der Wirkstoffkombination kann entweder durch getrennte Gabe der Wirkstoffe an den Patienten oder in Form von Kombinationspräparaten, worin mehrere Wirkstoffe in einer pharmazeutischen Zubereitung vorliegen, erfolgen.

### Beispielhaft seien genannt:

### Antidiabetika

Geeignete Antidiabetika sind z.B. die in der Roten Liste 2001, Kapitel 12 oder USP Dictionary of USAN and International Drug Names, US Pharmacopeia, Rockville 2003, offenbart. Antidiabetika umfassen alle Insuline und Insulinderivate, wie z.B. Lantus^{®} (siehe www.lantus.com) oder Apidra^{®}, sowie andere schnell wirkende Insuline (siehe US 6,221,633), GLP-1-Rezeptor Modulatoren, wie in WO 01/04146 beschrieben, oder auch wie z.B. diejenigen, die in WO 98/08871 von Novo Nordisk A/S offenbart wurden. Die oral wirksamen hypoglykämischen Wirkstoffe umfassen vorzugsweise Sulphonylfhamstoffe, Biguanidine, Meglitinide, Oxadiazolidindione, Thiazolidindione, Glukosidase-Inhibitoren, Glukagon-Antagonisten, orale GLP-1-Agonisten, DPP-IV Inhibitoren, Kaliumkanalöffner, wie z.B. diejenigen, die in WO 97/26265 und WO 99/03861 offenbart wurden, Insulin-Sensitizer, Inhibitoren von Leberenzymen, die an der Stimulation der Glukoneogenese und/oder Glykogenolyse beteiligt sind, Modulatoren der Glukoseaufnahme, den Fettstoffwechsel verändernde Verbindungen, die zur Veränderung der Lipidzusammensetzung des Blutes führen, Verbindungen, die die Nahrungsmitteleinnahme oder Nahrungsmittelaufnahme verringern, PPAR - und PXR-Modulatoren und Wirkstoffe, die auf den ATP-abhängigen Kaliumkanal der Betazellen wirken.

Bei einer Ausführungsform der Erfindung werden die Verbindungen der Formel I in Kombination mit Insulin verabreicht.

Bei einer Ausführungsform der Erfindung werden die Verbindungen der Formel I in Kombination mit Substanzen, die Einfluss haben auf die hepatische Glukoseproduktion, wie z.B. Glycogen Phosphorylase Inhibitoren (siehe: WO 01/94300, WO 02/096864, WO 03/084923, WO 03/084922, WO 03/104188) Bei einer Ausführungsform werden die Verbindungen der Formel I in Kombination mit einem Sulphonylharnstoff, wie z.B. Tolbutamid, Glibenclamid, Glipizid oder Glimepirid verabreicht.
Bei einer Ausführungsform werden die Verbindungen der Formel I in Kombination mit einem Wirkstoff verabreicht, der auf den ATP-abhängigen Kaliumkanal der Betazellen wirkt, wie z.B. Tolbutamid, Glibenclamid, Glipizid, Glimepirid oder Repaglinid.
Bei einer Ausführungsform werden die Verbindungen der Formel I in Kombination mit einem Biguanid, wie z.B. Metformin, verabreicht.

Bei wieder einer Ausführungsform werden die Verbindungen der Formel I in Kombination mit einem Meglitinid, wie z.B. Repaglinid, verabreicht.
Bei einer Ausführungsform werden die Verbindungen der Formel I in Kombination mit einem Thiazolidindion, wie z.B., Ciglitazon, Pioglitazon, Rosiglitazon oder den in WO 97/41097 von Dr. Reddy's Research Foundation offenbarten Verbindungen, insbesondere 5-[[4-[(3,4-Dihydro-3-methyl-4-oxo-2-chinazolinylmethoxy]-phenyl]methyl]-2,4-thiazolidindion, verabreicht.
Bei einer Ausführungsform werden die Verbindungen der Formel I in Kombination mit einem DPPIV Inhibitor, wie z.B. in WO98/19998, WO99/61431, WO99/67278, WO99/67279, WO01/72290, WO 02/38541, WO03/040174 beschrieben, insbesondere P 93/01 (1-Cyclopentyl-3-methyl-1-oxo-2-pentanammonium chlorid), P-31/98, LAF237 (1-[2-[3-Hydroxyadamant-1-ylamino)acetyl]pyrrolidin-2-(S)-carbonitril), TS021 ((2S, 4S)-4-Fluoro-1-[[(2-hydroxy-1,1-dimethylethyl)amino]-acetyl]-pyrrolidin-2-carbonitril monobenzenesulfonat)

Bei einer Ausführungsform der Erfindung werden die Verbindungen der Formel I in Kombination mit einem PPARgamma Agonist, wie z.B. Rosiglitazon, Pioglitazon, verabreicht.

Bei einer Aufführungsform werden die Verbindungen der Formel I in Kombination mit Verbindungen mit inhibitorischer Wirkung auf SGLT-1 und/oder 2, wie z.B. in PCT/EP03/06841, PCT/EP03/13454 und PCT/EP03/13455 direkt oder indirekt offenbart, verabreicht.

Bei einer Ausführungsform werden die Verbindungen der Formel I in Kombination mit einem α-Glukosidase-Inhibitor, wie z.B. Miglitol oder Acarbose, verabreicht.
Bei einer Ausführungsform werden die Verbindungen der Formel I in Kombination mit mehr als einer der vorstehend genannten Verbindungen, z.B. in Kombination mit einem Sulphonylharnstoff und Metformin, einem Sulphonylharnstoff und Acarbose, Repaglinid und Metformin, Insulin und einem Sulphonylharnstoff, Insulin und Metformin, Insulin und Troglitazon, Insulin und Lovastatin, etc. verabreicht.

### Lipidmodulatoren

Bei einer Ausführungsform der Erfindung werden die Verbindungen der Formel I in Kombination mit einem HMGCoA-Reduktase Inhibitor wie Lovastatin , Fluvastatin, Pravastatin, Simvastatin, Ivastatin, Itavastatin, Atorvastatin, Rosuvastatin verabreicht.

Bei einer Ausführungsform der Erfindung werden die Verbindungen der Formel I in Kombination mit einem Gallensäureresorptionsinhibitor (siehe z.B. US 6,245,744, US 6,221,897, US 6,277,831, EP 0683 773, EP 0683 774) verabreicht.

Bei einer Ausführungsform der Erfindung werden die Verbindungen der Formel I in Kombination mit einem polymeren Gallensäureadsorber, wie z.B. Cholestyramin, Colesevelam, verabreicht.

Bei einer Ausführungsform der Erfindung werden die Verbindungen der Formel I in Kombination mit einem Cholesterinresorptionsinhibitor, wie z.B. in WO 0250027 beschrieben, oder Ezetimibe, Tiqueside, Pamaqueside verabreicht.

Bei einer Ausführungsform der Erfindung werden die Verbindungen der Formel I in Kombination mit einem LDL-Rezeptorinduktor (siehe z.B. US 6,342,512) verabreicht.

Bei einer Ausführungsform werden die Verbindungen der Formel I in Kombination mit Ballaststoffen, vorzugsweise unlöslichen Ballaststoffen (siehe z.B. Carob/ Caromax^{®} (Zunft H J; et al., Carob pulp preparation for treatment of hypercholesterolemia, ADVANCES IN THERAPY (2001 Sep-Oct), 18(5), 230-6)); Caromax ist ein Carob enthaltendes Produkt der Fa. Nutrinova, Nutrition Specialties & Food Ingrediens GmbH, Industriepark Höchst, 65926 Frankfurt / Main) verabreicht. Die Kombination mit Caromax^{®} kann in einer Zubereitung erfolgen, oder durch getrennte Gabe von Verbindungen der Formel I und Caromax^{®}. Caromax^{®} kann dabei auch in Form von Lebensmitteln, wie z.B. in Backwaren oder Müsliriegeln, verabreicht werden.

Bei einer Ausführungsform der Erfindung werden die Verbindungen der Formel I in Kombination mit einem PPARalpha Agonist verabreicht.

Bei einer Ausführungsform der Erfindung werden die Verbindungen der Formel I in Kombination mit einem gemischten PPAR alpha/gamma Agonisten, wie z.B. AZ 242 (Tesaglitazar, (S)-3-(4-[2-(4-Methansulfonyloxyphenyl)ethoxy]phenyl)-2-ethoxypropionsäure), BMS 298585 (N-[(4-Methoxyphenoxy)carbonyl]-N-[[4-[2-(5-methyl-2-phenyl-4-oxazolyl)ethoxy]phenyl]methyl]glycin) oder wie in WO 99/62872, WO 99/62871, WO 01/40171, WO 01/40169, WO96/38428, WO 01/81327, WO 01/21602, WO 03/020269, WO 00/64888 oder WO 00/64876 beschrieben, verabreicht.

Bei einer Ausführungsform der Erfindung werden die Verbindungen der Formel I in Kombination mit einem Fibrat, wie z.B. Fenofibrat, Gemfibrozil, Clofibrat, Bezafibrat, verabreicht.

Bei einer Ausführungsform der Erfindung werden die Verbindungen der Formel I in Kombination mit Nicotinsäure bzw. Niacin verabreicht.

Bei einer Ausführungsform der Erfindung werden die Verbindungen der Formel I in Kombination mit einem CETP-Inhibitor, z.B. CP- 529, 414 (Torcetrapib), verabreicht.

Bei einer Ausführungsform der Erfindung werden die Verbindungen der Formel I in Kombination mit einem ACAT-Inhibitor verabreicht

Bei einer Ausführungsform der Erfindung werden die Verbindungen der Formel I in Kombination mit einem MTP-Inhibitor, wie z.B. Implitapide, verabreicht.

Bei einer Ausführungsform der Erfindung werden die Verbindungen der Formel I in Kombination mit einem Antioxidanz verabreicht.

Bei einer Ausführungsform der Erfindung werden die Verbindungen der Formel I in Kombination mit einem Lipoprotein-Lipase Inhibitor, verabreicht.

Bei einer Ausführungsform der Erfindung werden die Verbindungen der Formel I in Kombination mit einem ATP-Citrat-Lyase Inhibitor verabreicht.

Bei einer Ausführungsform der Erfindung werden die Verbindungen der Formel I in Kombination mit einem Squalen Synthetase Inhibitor verabreicht

Bei einer Ausführungsform der Erfindung werden die Verbindungen der Formel I in Kombination mit einem Lipoprotein(a) Antagonist verabreicht.

### Antiobesita

Bei einer Ausführungsform der Erfindung werden die Verbindungen der Formel I in Kombination mit einem Lipase Inhibitor, wie z.B. Orlistat, verabreicht.

Bei einer Ausführungsform ist der weitere Wirkstoff Fenfluramin oder Dexfenfluramin. Bei noch einer Ausführungsform ist der weitere Wirkstoff Sibutramin.

Bei einer weiteren Ausführungsform werden die Verbindungen der Formel I in Kombination mit CART-Modulatoren (siehe "Cocaine-amphetamine-regulated transcript influences energy metabolism, anxiety and gastric emptying in mice" Asakawa, A, et al., M.:Hormone and Metabolic Research (2001), 33(9), 554-558), NPY-Antagonisten z.B. Naphthalin-1-sulfonsäure {4-[(4-amino-quinazolin-2-ylamino)-methyl]-cyclohexylmethyl}- amid; hydrochlorid (CGP 71683A)), MC4-Agonisten (z.B. 1-Amino-1,2,3,4-tetrahydro-naphthalin-2-carbonsäure [2-(3a-benzyl-2-methyl-3-oxo-2,3,3a,4,6,7-hexahydro-pyrazolo[4,3-c]pyridin-5-yl)-1-(4-chloro-phenyl)-2-oxo-ethyl]-amid; (WO 01/91752)), Orexin-Antagonisten (z.B. 1-(2-Methyl-benzoxazol-6-yl)-3-[1,5]naphthyridin-4-yl-hamstoff; hydrochloride (SB-334867-A)), H3-Agonisten (3-Cyclohexyl-1-(4,4-dimethyl-1,4,6,7-tetrahydro-imidazo[4,5-c]pyridin-5-yl)-propan-1- on Oxalsäuresalz (WO 00/63208)); TNF-Agonisten, CRF-Antagonisten (z.B. [2-Methyl-9-(2,4,6-trimethyl-phenyl)-9H-1,3,9-triaza-fluoren-4-yl]-dipropyl-amin (WO 00/66585)), CRF BP-Antagonisten (z.B. Urocortin), Urocortin-Agonisten, β3-Agonisten (z.B. 1-(4-Chloro-3-methanesulfonylmethyl-phenyl)-2-[2-(2,3-dimethyl-1H-indol-6-yloxy)-ethylamino]-ethanol; hydrochloride (WO 01/83451)), MSH (Melanocyt-stimulierendes Hormon)-Agonisten, CCK-A Agonisten (z.B. {2-[4-(4-Chloro-2,5-dimethoxy-phenyl)-5-(2-cyclohexyl-ethyl)-thiazol-2-ylcarbamoyl]-5,7-dimethyl-indol-1-yl}-acetic acid Trifluoressigsäuresalz (WO 99/15525)); Serotonin-Wiederaufnahme-Inhibitoren (z.B. Dexfenfluramine), gemischte Sertonin- und noradrenerge Verbindungen (z.B. WO 00/71549), 5HT-Agonisten z.B. 1-(3-Ethyl-benzofuran-7-yl)-piperazin Oxalsäuresalz (WO 01/09111), Bombesin-Agonisten, Galanin-Antagonisten, Wachstumshormon (z.B. humanes Wachstumshormon), Wachstumshormon freisetzende Verbindungen (6-Benzyloxy-1-(2-diisopropylamino-ethylcarbamoyl)-3,4-dihydro-1H-isoquinoline-2-carboxylic acid tert-butyl ester (WO 01/85695)), TRH-Agonisten (siehe z.B. EP 0 462 884) entkoppelnde Protein 2- oder 3-Modulatoren, Leptinagonisten (siehe z.B. Lee, Daniel W.; Leinung, Matthew C.; Rozhavskaya-Arena, Marina; Grasso, Patricia. Leptin agonists as a potential approach to the treatment of obesity. Drugs of the Future (2001), 26(9), 873-881), DA-Agonisten (Bromocriptin, Doprexin), Lipase/Amylase-Inhibitoren (z.B. WO 00/40569), PPAR-Modulatoren (z.B. WO 00/78312), RXR-Modulatoren oder TR-β-Agonisten) verabreicht.

Bei einer Ausführungsform der Erfindung ist der weitere Wirkstoff Leptin.

Bei einer Ausführungsform ist der weitere Wirkstoff Dexamphatamin, Amphetamin, . Mazindol oder Phentermin.
Bei einer Ausführungsform werden die Verbindungen der Formel I in Kombination mit Medikamenten mit Wirkungen auf das Herz-Kreislauf- und das Blutgefäß-System, wie verabreicht, wie z.B. ACE-Hemmer (z.B. Ramipril), Medikamente, die auf das Angiotensin-Renin-System wirken, Calcium-Antagonisten, Beta-Blocker etc.

Bei einer Ausführungsform werden die Verbindungen der Formel I in Kombination mit anti-entzündlich wirkenden Medikamenten verabreicht.

Bei einer Ausführungsform werden die Verbindungen der Formel in Kombination mit Medikamenten, die zur Krebstherapie und Krebsprävention eingesetzt werden, verabreicht.

Es versteht sich, dass jede geeignete Kombination der erfindungsgemäßen Verbindungen mit einer oder mehreren der vorstehend genannten Verbindungen und wahlweise einer oder mehreren weiteren pharmakologisch wirksamen Substanzen als unter den Schutzbereich der vorliegenden Erfindung fallend angesehen wird.

Die Wirksamkeit der Verbindungen wurde wie folgt getestet:

### Bestimmung von EC50-Werten von PPAR-Agonisten im zellulären PPARalpha -Test Prinzip

Für die Analyse der Wirkstärke von Substanzen, die an humanes PPARalpha binden und es in agonistischer Weise aktivieren, wird eine stabil transfizierte HEK-Zellinie (HEK= human embryo kidney) benutzt, die hier als PPARalpha-Reporterzellinie bezeichnet wird. Sie enthält zwei genetische Elemente, ein Luziferase-Reporterelement (pdeltaM-GAL4-Luc-Zeo) und ein PPARalpha-Fusionsprotein (GR-GAL4-humanPPARalpha-LBD), welches die Expression des Luziferase-Reporterelementes in Abhängigkeit eines PPARalpha-Liganden vermittelt. Das stabil und konstitutiv exprimierte Fusionsprotein GR-GAL4-humanPPARalpha-LBD bindet im Zellkern der PPARalpha-Reporterzellinie über den GAL4-Proteinanteil an die GAL4-DNA-Bindungsmotife 5'-oberhalb des Luziferase-Reporterelementes, das im Genom der Zellinie integriert ist. Ohne Zugabe eines PPARalpha-Liganden ist die Expression des Luziferase-Reportergens nur gering, sofern im Test fettsäuredepletiertes fötales Kälberserum (cs-FKS) verwendet wird. PPARalpha-Liganden binden und aktivieren das PPARalpha-Fusionsprotein und bewirken darüber die Expression des Luciferasereportergens. Die gebildete Luziferase lässt sich über ein entsprechendes Substrat mittels Chemilumineszenz nachweisen.

### Konstruktion der Zelllinie

Die PPARalpha-Reporterzellinie wurde in 2 Stufen hergestellt: Zuerst wurde das Luziferase-Reporterelement konstruiert und stabil in HEK-Zellen transfiziert. Dazu wurden fünf Bindungsstellen des Hefe-Transkriptionsfaktors GAL4 (jeweils 5'-CGGAGTACTGTCCTCCGAG-3') 5'-oberhalb eines 68 bp langen minimalen MMTV-Promotors (Genbank-Accession # V01175) einkloniert. Der minimale MMTV-Promotorabschnitt enthält eine CCAAT-Box und ein TATA-Element, um eine effiziente Transkription durch die RNA-Polymerase II zu ermöglichen. Die Klonierung und Sequenzierung des GAL4-MMTV-Konstruktes erfolgte analog wie bei Sambrook J. et. al. beschrieben (Molecular cloning, Cold Spring Harbor Laboratory Press, 1989). Danach wurde 3'-unterhalb des GAL4-MMTV-Elementes das gesamte Luziferasegen von Photinus pyralis (Genbank-Accession # M15077) einkloniert. Nach der Sequenzierung wurde das aus fünf GAL4-Bindungsstellen, MMTV-Promotor und Luziferasegen bestehende Luziferase-Reporterelement in ein Zeozin-Resistenz vermittelndes Plasmid umkloniert, um zu dem Plasmid pdeltaM-GAL4-Luc-Zeo zu gelangen. Dieser Vektor wurde nach den Angaben in Ausubel, F.M. et al. (Current protocols in molecular biology, Vol. 1-3, John Wiley & Sons, Inc., 1995) in HEK-Zellen transfiziert. Danach wurde unter Verwendung von zeozinhaltigem Medium (0,5 mg/ml) ein geeigneter stabiler Zellklon selektioniert, der eine möglichst niedrige Basalexpression des Luziferasegens zeigte.
In einem zweiten Schritt wurde das PPARalpha-Fusionsprotein (GR-GAL4-humanPPARalpha-LBD) in den beschriebenen stabilen Zellklon eingebracht. Dazu wurde zunächst die für die N-terminalen 76 Aminosäuren des Glukocorticoid-Rezeptors (Genbank-Accession # P04150) kodierende cDNA mit dem für die Aminosäuren 1-147 des Hefetranskriptionsfaktors GAL4 (Genbank-Accession # P04386) kodierenden cDNA-Abschnitt verknüpft. Am 3'-Ende dieses GR-GAL4-Konstruktes wurde die cDNA der Ligandenbindungsdomäne des humanen PPARalpha-Rezeptors einkloniert (Aminosäuren S167-Y468; Genbank-Accession # S74349). Das so hergestellte Fusionskonstrukt (GR-GAL4-humanPPARalpha-LBD) wurde in das Plasmid pcDNA3 (Firma Invitrogen) umkloniert, um darin eine konstitutive Expression durch den Cytomegalovirus-Promotor zu ermöglichen. Dieses Plasmid wurde mit einer Restriktionsendonuklease linearisiert und stabil in den bereits beschriebenen, das Luziferase-Reporterelement enthaltenden Zellklon transfiziert. Durch Selektion mit Zeozin (0,5 mg/ml) und G418 (0,5 mg/ml) wurde die fertige PPARalpha-Reporterzellinie isoliert, die ein Luziferase-Reporterelement enthält und konstitutiv das PPARalpha-Fusionsprotein (GR-GAL4-humanPPARalpha-LBD) exprimiert.

### Durchführung des Tests

Die Aktivität von PPARalpha-Agonisten wird in einem 3-Tagestest bestimmt, der nachfolgend beschrieben ist:

### Tag 1

Die PPARalpha-Reporterzellinie wird bis zu einer 80 %-igen Konfluenz in DMEM-Medium (# 41965-039, Invitrogen) kultiviert, das mit folgenden Zusätzen versetzt ist: 10% cs-FKS (fötales Kälberserum; #SH-30068.03, Hyclone), 0,5 mg/ml Zeozin (#R250-01, Invitrogen), 0,5 mg/ml G418 (#10131-027, Invitrogen), 1% Penicillin-Streptomycin-Lösung (#15140-122, Invitrogen) und 2 mM L-Glutamin (#25030-024, Invitrogen). Die Kultivierung erfolgt in Standard-Zellkulturflaschen (# 353112, Becton Dickinson) in einem Zellkulturbrutschrank bei 37°C in Anwesenheit von 5% CO₂. Die zu 80% konfluenten Zellen werden einmal mit 15 ml PBS gewaschen (#14190-094, Invitrogen), mit 3 ml Trypsinlösung (#25300-054, Invitrogen) für 2 min bei 37°C behandelt, in 5 ml des beschriebenen DMEM-Mediums aufgenommen und in einem Zellzählgerät gezählt. Nach der Verdünnung auf 500.000 Zellen/ml werden jeweils 35.000 Zellen pro well einer 96 well-Mikrotiterplatte mit klarem Plastikboden (#3610, Coming Costar) ausgesät. Die Platten werden für 24 h in einem Zellkulturbrutschrank bei 37°C und 5% CO₂ inkubiert.

### Tag 2

Zu testende PPARalpha-Agonisten werden in einer Konzentration von 10 mM in DMSO gelöst. Diese Stocklösung wird in DMEM-Medium (#41965-039, Invitrogen) verdünnt, das mit 5 % cs-FKS (#SH-30068.03, Hyclone), 2 mM L-Glutamin (#25030-024, Invitrogen) und den bereits beschriebenen Antibiotika (Zeozin, G418, Penicillin und Streptomycin) versetzt ist.
Testsubstanzen werden in 11 verschiedenen Konzentrationen im Bereich von 10 µM bis 100 pM getestet. Potentere Verbindungen werden in Konzentrationsbereichen von 1 µM bis 10 pM oder zwischen 100 nM und 1 pM geprüft.
Das Medium der an Tag 1 ausgesäten PPARalpha-Reporterzellinie wird vollständig abgesaugt und die in Medium verdünnten Testsubstanzen sofort zu den Zellen zugegeben. Die Verdünnung und Zugabe der Substanzen erfolgt mit einem Roboter (Beckman FX). Das Endvolumen der in Medium verdünnten Testsubstanzen beträgt 100 µl pro well einer 96 well-Mikrotiterplatte. Die DMSO-Konzentration in dem Test beträgt unter 0.1 % v/v, um zelltoxische Effekte des Lösungsmittels zu vermeiden.

Jede Platte wurde mit einem Standard PPARalpha-Agonisten belegt, der ebenfalls in 11 verschiedenen Konzentrationen verdünnt wird, um die Funktionsfähigkeit des Tests in jeder Einzelplatte nachzuweisen. Die Testplatten werden für 24 h in einem Brutschrank bei 37 °C und 5 % CO₂ inkubiert.

### Tag3

Die mit den Testsubstanzen behandelten PPARalpha-Reporterzellen werden aus dem Brutschrank entnommen und das Medium abgesaugt. Zur Lyse der Zellen werden 50 µl Bright Glo Reagens (Firma Promega) pro well einer 96-well Mikrotiterplatte zupipettiert. Nach einer 10 minütigen Inkubation im Dunkeln bei Raumtemperatur werden die Mikrotiterplatten im Lumineszenzmeßgerät (Trilux der Firma Wallac) gemessen. Die Messzeit pro well einer Mikrotiterplatte beträgt 1 sec.

### Auswertung

Die Rohdaten des Lumineszenzmeßgerätes werden in ein Microsoft Excel-File transferiert. Dosis-Wirkungskurven und EC50-Werte von PPAR-Agonisten werden mit dem Program XL.Fit nach Vorgabe des Herstellers (Firma IDBS) berechnet.

Die PPARalpha-EC50-Werte für die Verbindungen der Beispiele 1 bis 91 liegen in diesem Assay im Bereich von 0.6nM bis >10 µM.

Die Ergebnisse für die Aktivität einiger erfindungsgemäßer Verbindungen der Formel I sind in der folgenden Tabelle I angegeben:

**Tabelle I**

| **Beispiel Nr.** | **EC50 PPARalpha [nM]** |
|---|---|
| 1 | 41 |
| 5 | 69 |
| 8 | 24 |
| 18 | 1,5 |
| 22 | 43 |
| 25 | 6,2 |
| 35 | 84 |
| 36 | 14 |
| 47 | 0,8 |
| 52 | 22 |
| 60 | 26 |
| 81 | 0,6 |
| 93 | 78 |

Aus der Tabelle I ist ersichtlich, dass die erfindungsgemäßen Verbindungen der Formel I den PPARalpha-Rezeptor aktivieren und damit zum Beispiel analog zu klinisch verwendeten Fibraten im Organismus eine Triglyceridsenkung bewirken (siehe z.B. J.-Ch. Fruchard et al.,: PPARS, Metabolic Disease and Atherosclerosis, Pharmacological Research, Vol. 44, No. 5, 2001; S. Kersten et al.: Roles of PPARs in health and disease, NATURE, VOL 405 ,25 MAY 2000 ;I. Pineda et al.: Peroxisome proliferator-activated receptors: from transcriptional control to clinical practice,Curr Opin Lipidol 12: 2001, 245-254).

### Bestimmung von EC50-Werten von PPAR-Agonisten im zellulären PPARgamma -Test

### Prinzip

Zur Bestimmung der zellulären PPARgamma Aktivität von PPAR-Agonisten wird ein transientes Transfektionssystem eingesetzt. Es basiert auf der Verwendung eines Luziferase-Reporterplasmides (pGL3basic-5xGAL4-TK) und eines PPARgamma-Expressionsplasmides (pcDNA3-GAL4-humanPPARgammaLBD). Beide Plasmide werden vorübergehend (= transient) in humane embryonale Nierenzellen (HEK-Zellen) transfiziert. In diesen Zellen wird dann das Fusionsprotein GAL4-humanPPARgammaLBD exprimiert, das an die GAL4-Bindungsstellen des Reporterplasmides bindet. In Anwesenheit eines PPARgamma-aktiven Liganden wird durch das aktivierte Fusionsprotein GAL4-humanPPARgammaLBD die Expression des Luziferase-Reportergens induziert, was sich nach Zugabe eines Luziferasesubtrates in Form eines Chemilumineszenzsignals nachweisen lässt. Im Unterschied zur stabil transfizierten PPARalpha-Reporterzellinie werden beim zellulären PPARgamma-Test die beiden Komponenten (Luziferase-Reporterplasmid und PPARgamma-Expressionsplasmid) transient in HEK-Zellen transfiziert, weil die stabile und permanente Expression des PPARgamma-Fusionsproteins zelltoxisch ist.

### Konstruktion der Plasmide

Das Luziferase-Reporterplasmid pGL3basic-5xGAL4-TK basiert auf dem Vektor pGL3basic der Firma Promega. Zur Herstellung des Reporterplasmides wurden fünf Bindungsstellen des Hefe-Transkriptionsfaktors GAL4 (jede Bindungsstelle mit der Sequenz 5'-CTCGGAGGACAGTACTCCG-3'), 5'-obefialb zusammen mit einem 160 bp langen Thymidinkinase-Promotorabschnitt (Genbanlc-Accession # AF027128) in pGL3basic einkloniert. 3'-unterhalb des Thymidinkinasepromotors liegt das gesamte Luziferasegen von Photinus pyralis (Genbank-Accession # M15077), welches bereits Bestandteil des verwendeten Plasmides pGL3basic ist. Die Klonierung und Sequenzierung des Reporterplasmides pGL3basic-5xGAL4-TK erfolgte analog wie bei Sambrook J. et. al. beschrieben (Molecular cloning, Cold Spring Harbor Laboratory Press, 1989).
Zur Herstellung des PPARgamma-Expressionsplasmides pcDNA3-GAL4-humanPPARgammaLBD wurde in das Plasmid pcDNA3 (Firma Invitrogen) 3'-unterhalb des Cytomegalovirus-Promotors zunächst die für die Aminosäuren 1-147 des Hefetranskriptionsfaktors GAL4 (Genbank-Accession # P04386) kodierende cDNA einkloniert. 3'-unterhalb der GAL4-DNA-Bindungsdomäne wurde anschließend die cDNA der Ligandenbindungsdomäne (LBD) des humanen PPARgamma-Rezeptors kloniert (Aminosäuren I152-Y475; Accession # g1480099). Klonierung und Sequenzierung des PPARgamma-Expressionsplasmides pcDNA3-GAL4-humanPPARgammaLBD erfolgten wiederum analog wie bei Sambrook J. et. al. beschrieben (Molecular cloning, Cold Spring Harbor Laboratory Press, 1989). Neben dem Luziferase-Reporterplasmid pGL3basic-5xGAL4-TK und dem PPARgamma-Expressionsplasmid pcDNA3-GAL4-humanPPARgammaLBD werden für den zellulären PPARgamma-Test noch das Referenzplasmid pRL-CMV (Firma Promega), sowie das Plasmid pBluescript-SK(+) der Firma Stratagene verwendet. Alle vier Plasmide wurden vor der Transfektion in HEK-Zellen mit einem Plasmidpräparationskit der Firma Qiagen präpariert, der eine möglichst endotoxinfreie Plasmidqualität gewährleistet.

### Durchführung des Tests

Die Aktivität von PPARgamma-Agonisten wird in einem 4-Tagestest bestimmt, der nachfolgend beschrieben ist. Vor der Transfektion werden HEK-Zellen in DMEM-Medium (# 41965-039, Invitrogen) kultiviert, das mit folgenden Zusätzen versetzt ist: 10% FKS (#16000-044, Invitrogen), 1 % Penicillin-Streptomycin-Lösung (#15140-122, Invitrogen) und 2 mM L-Glutamin (#25030-024, Invitrogen).

### Tag 1

Zunächst wird Lösung A hergestellt, ein Transfektionsgemisch, das neben DMEM-Medium alle vier bereits beschriebenen Plasmide enthält. Für einen Test werden pro 96-well Mikrotiterplatte folgende Mengen zum Ansetzen von 3 ml Lösung A verwendet: 2622 µl antibiotika- und serumfreies DMEM-Medium (# 41965-039, Invitrogen), 100 µl Referenzplasmid pRL-CMV (1 ng/µl), 100 µl Luziferase-Reporterplasmid pGL3basic-5xGAL4-TK (10 ng/µl), 100 µl PPARgamma-Expressionsplasmid pcDNA3-GAL4-humanPPARgammaLBD (100 ng/µl) und 78 µl Plasmid pBluescript-SK(+) (500 ng/µl). Danach werden pro 96-well Mikrotiterplatte 2 ml Lösung B durch Mischen von 1,9 ml DMEM-Medium (# 41965-039, Invitrogen) mit 100 µl PolyFect-Transfektionsreagens (Firma Qiagen) hergestellt. Anschließend werden 3 ml Lösung A mit 2 ml Lösung B zu 5 ml Lösung C versetzt, durch mehrfaches Pipettieren gründlich gemischt und für 10 min bei Raumtemperatur inkubiert. 80% konfluente HEK-Zellen aus einer 175 cm² großen Zellkulturflasche werden einmal mit 15 ml PBS gewaschen (#14190-094, Invitrogen) und mit 3 ml Trypsinlösung (#25300-054, Invitrogen) für 2 min bei 37°C behandelt. Danach werden die Zellen in 15 ml DMEM-Medium (# 41965-039, Invitrogen) aufgenommen, welches mit 10% FKS (# 16000-044, Invitrogen), 1% Penicillin-Streptomycin-Lösung (#15140-122, Invitrogen) und 2 mM L-Glutamin (#25030-024, Invitrogen) versetzt ist. Nach dem Zählen der Zellsuspension im Zellzählgerät wird die Suspension auf 250.000 Zellen/ml verdünnt. Für 1 Mikrotiterplatte werden 15 ml dieser Zellsuspension mit 5 ml der Lösung C vermengt. Pro well einer 96 well-Mikrotiterplatte mit klarem Plastikboden (#3610, Coming Costar) werden 200 µl der Suspension ausgesät. Die Platten werden für 24 h in einem Zellkulturbrutschrank bei 37°C und 5 % CO₂ inkubiert.

### Tag 2

Zu testende PPAR-Agonisten werden in einer Konzentration von 10 mM in DMSO gelöst. Diese Stocklösung wird in DMEM-Medium (# 41965-039, Invitrogen) verdünnt, welches mit 2 % Ultroser (#12039-012, Biosepra), 1 % Penicillin-Streptomycin-Lösung (#15140-122, Invitrogen) und 2 mM L-Glutamin (#25030-024, Invitrogen) versetzt ist. Testsubstanzen werden in insgesamt 11 verschiedenen Konzentrationen im Bereich von 10 µM bis 100 pM getestet. Potentere Verbindungen werden in Konzentrationsbereichen von 1 µM bis 10 pM geprüft.
Das Medium der an Tag 1 transfizierten und ausgesäten HEK-Zellen wird vollständig abgesaugt und die in Medium verdünnten Testsubstanzen sofort zu den Zellen zugegeben. Die Verdünnung und Zugabe der Substanzen erfolgt mit einem Roboter (Beckman FX). Das Endvolumen der in Medium verdünnten Testsubstanzen beträgt 100 µl pro well einer 96 well-Mikrotiterplatte. Jede Platte wird mit einem Standard PPARgamma-Agonisten belegt, der ebenfalls in 11 verschiedenen Konzentrationen verdünnt wird, um die Funktionsfähigkeit des Tests in jeder Einzelplatte nachzuweisen. Die Testplatten werden für 48 h in einem Brutschrank bei 37 °C und 5 % CO₂ inkubiert.

### Tag 4

Nach dem Absaugen des Mediums werden gemäß den Angaben des Herstellers pro well je 50 µl Dual-Glo^{™} Reagens (Dual-Glo^{™} Luciferase Assay System; Firma Promega) zugegeben, um die Zellen zu lysieren und das Substrat für die in den Zellen gebildete Firefly-Luziferase (Photinus pyralis) zur Verfügung zu stellen. Nach einer 10 minütigen Inkubation im Dunkeln bei Raumtemperatur wird die Firefly-Luziferasevermittelte Chemilumineszenz im Messgerät gemessen (1 sec. Meßzeit/well; Trilux der Firma Wallac). Danach wird pro well je 50 µl des Dual-Glo^{™} Stop & Glo Reagens (Dual-Glo^{™} Luciferase Assay System; Firma Promega) zugegeben, um die Aktivität der Firefly-Luziferase abzustoppen und das Substrat für die vom Referenzplasmid pRL-CMV aus exprimierten Renilla-Luciferase zur Verfügung zu stellen. Nach einer weiteren 10 minütigen Inkubation im Dunkeln bei Raumtemperatur wird erneut für 1 sec/well die durch die Renilla-Luziferase vermittelte Chemilumineszenz im Messgerät gemessen.

### Auswertung

Die Rohdaten des Lumineszenzmeßgerätes werden in ein Microsoft Excel-File transferiert. Für jeden Meßpunkt, der sich von einem well der Mikrotiterplatte ableitet, wird der Quotient Firefly/Renilla-Luciferaseaktivität bestimmt. Aus den Quotienten werden die Dosis-Wirkungskurven und EC50-Werte von PPAR-Agonisten mit dem Program XL.Fit nach Vorgabe des Herstellers (Firma IDBS) berechnet.
Mit den in dieser Anmeldung beschriebenen PPAR-Agonisten wurden PPARgamma-EC5O-Werte im Bereich von 6nM bis >10 µM gemessen.

Die nachfolgend aufgeführten Beispiele dienen zur Erläuterung der Erfindung, ohne diese jedoch einzuschränken.

**Tabelle II:**

| | | | | | | | | | | | |
|---|---|---|---|---|---|---|---|---|---|---|---|
| 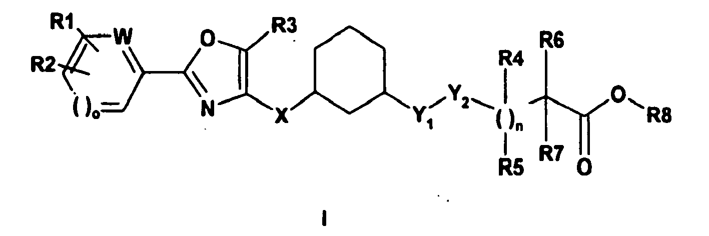 | | | | | | | | | | | |
| Im folgenden sind: | | | | | | | | | | | |
| W = CH, falls o =1, R4= R5 = R8 =H. | | | | | | | | | | | |

| **Bs P** | **R1** | **R2** | **R3** | **X** | **Y1** | **Y2** | **n** | **R6** | **R7** | **R9** | **R10** |
|---|---|---|---|---|---|---|---|---|---|---|---|
| 1 | 4-F | H | Me | CH2O | O | CH2 | 1 | H | Et | - | - |
| 2 | 4-F | H | Me | CH2O | O | CH2 | 1 | H | n-Pr | - | - |
| 3 | 4-F | H | Me | CH2O | O | CH2 | 1 | H | H | - | - |
| 4 | 4-F | H | Me | CH2O | O | CH2 | 1 | H | Me | - | - |
| 5 | 3-Me | H | Me | CH2O | O | CH2 | 0 | Cyclopropyl | | - | - |
| 6 | 3-Me | H | Me | CH2O | O | CH2 | 0 | H | CH2NR9R10 | Me | Bn |
| 7 | 3-Me | H | Me | CH2O | O | CH2 | 0 | H | CH2NR9R10 | Me | PhCH2CH2 |
| 8 | 3-Me | H | Me | CH2O | O | CH2 | 0 | H | MeOCH2 | - | - |
| 9 | 3-Me | H | Me | CH2O | O | CH2 | 0 | H | 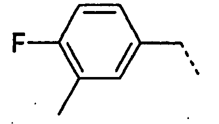 | - | - |
| 10 | 3-Me | H | Me | CH2O | O | CH2 | 0 | H | n-PrO | - | - |
| 11 | 3-Me | H | Me | CH2O | O | CH2 | 0 | H | 4-CF₃-(C6H4)-CH2O | - | - |
| 12 | 3-Me | H | Me | CH2O | O | CH2 | 0 | H | MeO | - | - |
| 13 | 3-Me | H | Me | CH2O | O | CH2 | 0 | H | 4-CF₃-(C6H4)-CH2O | - | - |
| 14 | 3-Me | H | Me | CH2O | O | CH2 | 0 | H | 3-CF₃-(C6H4)-CH2O | - | - |
| 15 | 3-Me | H | Me | CH2O | O | CH2 | 0 | H | 3-MeO-(C6H4)-CH2O | - | - |
| 16 | 3-Me | H | Me | CH2O | O | CH2 | 0 | H | 2-Me-4-Me-(C6H4)-CH2O | - | - |
| 17 | 3-Me | H | Me | CH2O | 0 | CH2 | 0 | H | 4-Me-(C6H4)-CH2O | - | - |
| 18 | 3-Me | H | Me | CH2O | O | CH2 | 0 | H | 4-^{t}Bu--(C6H4)-CH2O | - | - |
| 19 | 3-Me | H | Me | CH2O | 0 | CH2 | 0 | H | 2-CF₃-(C6H4)-CH2O | - | - |
| 20 | 3-Me | H | Me | CH2O | O | CH2 | 0 | H | 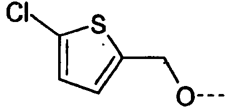 | - | - |
| 21 | 3-Me | H | Me | CH2O | O | CH2 | 0 | H | 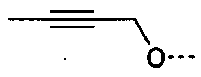 | - | - |
| 22 | 3-Me | H | Me | CH2O | O | CH2 | 0 | H | 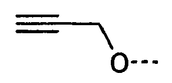 | - | - |
| 23 | 3-Me | H | Me | CH2O | O | CH2 | 0 | H | 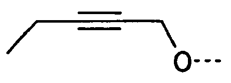 | - | - |
| 24 | 3-Me | H | Me | CH2O | O | CH2 | 0 | H | 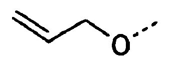 | - | - |
| 25 | 3-Me | H | Me | CH2O | O | CH2 | 0 | H | 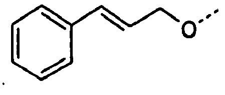 | - | - |
| 26 | 3-Me | H | Me | CH2O | O | CH2 | 0 | H | 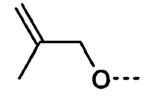 | - | - |
| 27 | 3-Me | H | Me | CH2O | O | CH2 | 0 | H | BnO | - | - |
| 28 | H | H | Me | CH2O | O | CH2 | 0 | H | MeO | - | - |
| 29 | 3-CF3 | H | Me | CH2O | O | CH2 | 0 | H | MeO | - | - |
| 30 | 3-OCF3 | H | Me | CH2O | O | CH2 | 0 | H | MeO | - | - |
| 31 | 4-CF3 | H | Me | CH2O | O | CH2 | 0 | H | MeO | - | - |
| 32 | 4-Me | H | Me | CH2O | O | CH2 | 0 | H | MeO | - | - |
| 33 | 2-Me | 6-Me | Me | CH2O | O | CH2 | 0 | H | MeO | - | - |
| 34 | 2-CF3 | H | Me | CH2O | O | CH2 | 0 | H | MeO | - | - |
| 35 | 2-Naphthyl | | Me | CH2O | O | CH2 | 0 | H | MeO | - | - |
| 36 | 2-Me | 4-Me | Me | CH2O | O | CH2 | 0 | H | MeO | - | - |
| 37 | 4-Ph | H | Me | CH2O | O | CH2 | 0 | H | MeO | - | - |
| 38 | 3-OMe | H | Et | CH2O | O | CH2 | 0 | H | MeO | - | - |
| 39 | 4-Me | H | Et | CH2O | O | CH2 | 0 | H | MeO | - | - |
| 40 | 2-CF3 | H | Et | CH2O | O | CH2 | 0 | H | MeO | - | - |
| 41 | 2-Me | 6-Me | Et | CH2O | O | CH2 | 0 | H | MeO | - | - |
| 42 | H | H | Me | CH2O | O | CH2 | 0 | H | n-PrO | - | - |
| 43 | 3-Me | H | Me | CH2O | O | CH2 | 0 | H | n-PrO | - | - |
| 44 | 3-OCF3 | H | Me | CH2O | 0 | CH2 | 0 | H | n-PrO | - | - |
| 45 | 3-OMe | H | Me | CH2O | O | CH2 | 0 | H | n-PrO | - | - |
| 46 | 4-CF3 | H | Me | CH2O | O | CH2 | 0 | H | n-PrO | - | - |
| 47 | 4-Me | H | Me | CH2O | O | CH2 | 0 | H | n-PrO | - | - |
| 48 | 4-CF3 | H | Me | CH2O | O | CH2 | 0 | H | n-PrO | - | - |
| 49 | 4-i-Pr | H | Me | CH2O | O | CH2 | 0 | H | n-PrO | - | - |
| 50 | 2-CF3 | H | Me | CH2O | O | CH2 | 0 | H | n-PrO | - | - |
| 51 | 2-Naphthyl | | Me | CH2O | O | CH2 | 0 | H | n-PrO | - | - |
| 52 | 3-CF3 | H | Me | CH2O | O | CH2 | 0 | H | n-PrO | - | - |
| 53 | 3-OMe | H | Et | CH2O | O | CH2 | 0 | H | n-PrO | - | - |
| 54 | 4-Me | H | Et | CH2O | O | CH2 | 0 | H | n-PrO | - | - |
| 55 | 2-CF3 | H | Et | CH2O | O | CH2 | 0 | H | n-PrO | - | - |
| 56 | 2-Me | 6-Me | Et | CH2O | O | CH2 | 0 | H | n-PrO | - | - |
| 57 | 3-CF3 | H | Me | CH2O | O | CH2 | 0 | H | EtO | - | - |
| 58 | 3-OCF3 | H | Me | CH2O | O | CH2 | 0 | H | EtO | - | - |
| 59 | 4-CF3 | H | Me | CH2O | O | CH2 | 0 | H | EtO | - | - |
| 60 | 4-OCF3 | H | Me | CH2O | O | CH2 | 0 | H | EtO | - | - |
| 61 | 4-i-Pr | H | Me | CH2O | O | CH2 | 0 | H | EtO | - | - |
| 62 | 2-CF3 | H | Me | CH2O | O | CH2 | 0 | H | EtO | - | - |
| 63 | 2-Me | 4-Me | Me | CH2O | O | CH2 | 0 | H | EtO | - | - |
| 64 | 4-Ph | H | Me | CH2O | 0 | CH2 | 0 | H | EtO | - | - |
| 65 | 4-Me | H | Et | CH2O | O | CH2 | 0 | H | EtO | - | - |
| 66 | 4-i-Pr | H | Et | CH2O | O | CH2 | 0 | H | EtO | - | - |
| 67 | 2-CF3 | H | Et | CH2O | O | CH2 | 0 | H | EtO | - | - |
| 68 | 2-Me | 6-Me | Et | CH2O | O | CH2 | 0 | H | EtO | - | - |
| 69 | 4-i-Pr | H | Et | CH2O | O | CH2 | 0 | H | n-PrO | - | - |
| 70 | H | H | Me | CH2O | O | CH2 | 0 | H | EtO | - | - |
| 71 | 3-Me | H | Me | CH2O | O | CH2 | 0 | H | EtO | - | - |
| 72 | 3-OMe | H | Me | CH2O | O | CH2 | 0 | H | EtO | - | - |
| 73 | 4-Me | H | Me | CH2O | O | CH2 | 0 | H | EtO | - | - |
| 74 | 2-Naphthyl | | Me | CH2O | O | CH2 | 0 | H | EtO | - | - |
| 75 | 3-OMe | H | Et | CH2O | O | CH2 | 0 | H | EtO | - | - |
| 76 | 3-Me | H | Me | CH2O | O | CH2 | 0 | H | 4-CF3-(C6H4)-CH2O | - | - |
| 77 | 3-CF3 | H | Me | CH2O | O | CH2 | 0 | H | 4-CF3-(C6H4)-CH2O | - | - |
| 78 | 3-OCF3 | H | Me | CH2O | O | CH2 | 0 | H | 4-CF3-(C6H4)-CH2O | - | - |
| 79 | 4-i-Pr | H | Me | CH2O | O | CH2 | 0 | H | 4-CF3-(C6H4)-CH2O | - | - |
| 80 | 2-Naphthyl | | Me | CH2O | 0 | CH2 | 0 | H | 4-CF3-(C6H4)-CH2O | - | - |
| 81 | 4-Me | H | Me | CH2O | O | CH2 | 0 | H | 4-CF3-(C6H4)-CH2O | - | - |
| 82 | 4-OCF3 | H | Me | CH2O | O | CH2 | 0 | H | 4-CF3-(C6H4)-CH2O | - | - |
| 83 | 4-CF3 | H | Me | CH2O | 0 | CH2 | 0 | H | 4-CF3-(C6H4)-CH2O | - | - |
| 84 | 3-OMe | H | Me | CH2O | O | CH2 | 0 | H | 4-CF3-(C6H4)-CH2O | - | - |
| 85 | 3-CF3 | H | Me | CH2O | O | CH2 | 0 | H | i-BuO | - | - |
| 86 | 3-OMe | H | Me | CH2O | O | CH2 | 0 | H | i-BuO | - | - |
| 87 | 4-Me | H | Me | CH2O | O | CH2 | 0 | H | i-BuO | - | - |
| 88 | 4-i-Pr | H | Me | CH2O | O | CH2 | 0 | H | i-BuO | - | - |
| 89 | 3-Me | H | Me | CH2OCH 2 | O | CH2 | 0 | H | 4-CF3-(C6H4)-CH2O | - | - |
| 90 | 3-Me | H | Me | CH2OCH 2 | O | CH2 | 0 | H | 4-CF3-(C6H4)-CH2O | - | - |
| 91 | 3-Me | H | Me | CH2O | O | CH2 | 1 | H | m-CF3-(C6H4)-CH2 | - | - |

Die gestrichelte Linie gibt die Verknüpfungsstelle an.

Die erfindungsgemäßen Verbindungen der Formel I können entsprechend den folgenden Reaktionsschemata erhalten werden:

### Verfahren A:

Dieses Verfahren dient zur Synthese des Bausteins A-A, worin R1, R2, W und R3 die oben genannten Bedeutungen haben.

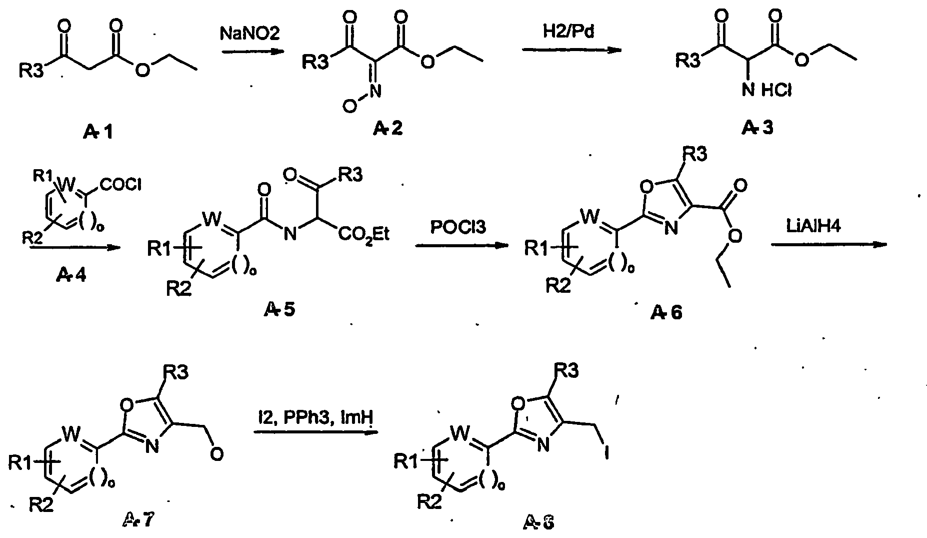

Der Ester A-1, worin R3 die oben genannte Bedeutung hat, wird mit Natriumnitrit und Salzsäure zum Oxim A-2 umgesetzt, welches durch Hydrierung mit Wasserstoff an Palladium/Kohle zum Amin A-3 reduziert wird.
Die Verbindung A-3 wird mit Säurechloriden der allgemeinen Formel A-4, worin R1, W und R2 die oben genannten Bedeutungen haben, und Base (beispielsweise Triethylamin) zur Verbindung A-5 umgesetzt.
Die Verbindung A-5 wird durch Erhitzen in Phosphorylchlorid zur Verbindung A-6 umgesetzt.
Der Ester A-6 wird mit Lithiumaluminiumhydrid in Diethylether zum Alkohol A-7 reduziert. Dieser wird mit lod, Imidazol (ImH) und Triphenylphosphin in das lodid A-8 überführt.

### Verfahren B:

Dieses Verfahren dient zur Synthese des Bausteins A-8, worin R1, R2, W und R3 die oben genannten Bedeutungen haben.

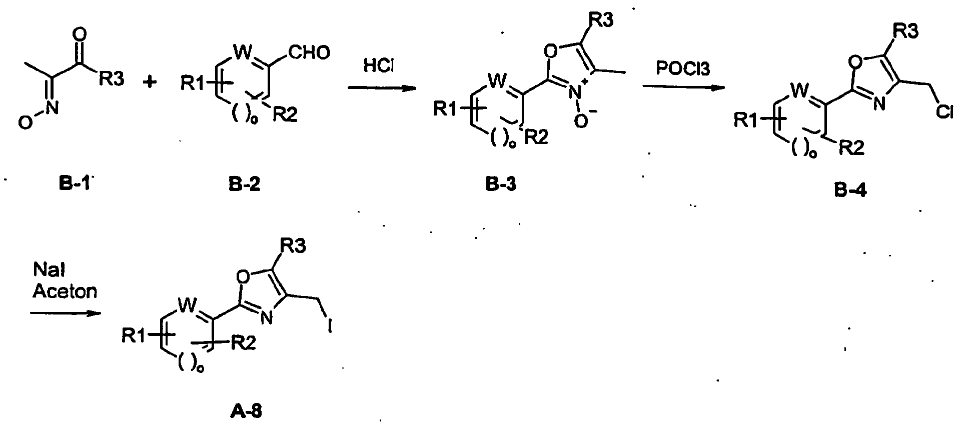

Die Verbindung B-1 wird mit dem Aldehyd B-2, worin R1, R2, W und R3 die oben beschriebenen Bedeutungen haben, in Ethanol mit Chlorwasserstoff zur Verbindung B-3 umgesetzt.
Die Verbindung B-3 wird in Phosphorylchlorid zum Sieden erhitzt, wobei die Verbindung B-4, erhalten wird. Diese wird mit Natriumiodid in Aceton zum Sieden erhitzt. Dabei erhält man die Verbindung A-8.

### Verfahren C:

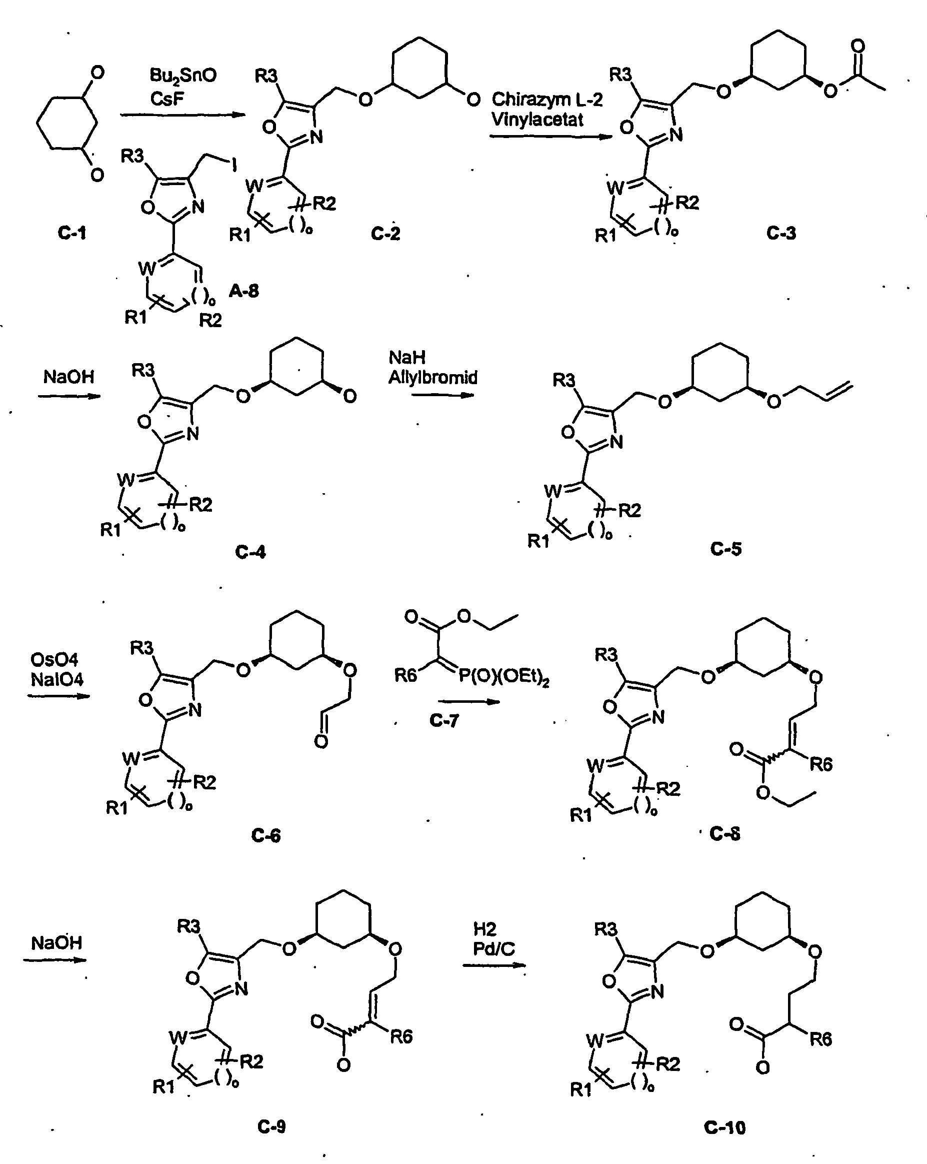

Die Verbindung C-1 wird mit Dibutylzinnoxid in Toluol unter Rückfluss am Wasserabscheider gekocht. Nach Zugabe von Dimethylformamid, Cäsiumfluorid und der Verbindung A-8 (siehe Verfahren A) wird die Suspension bei Raumtemperatur gerührt. Dabei wird die Verbindung C-2 erhalten. Diese wird mit Chirazym L-2 in Vinylacetat in das enantiomerenangereicherte Acetat
C-3 überführt. Das Acetat C-3 wird mit Natriumhydroxid in Methanol zum Alkohol C-4 umgesetzt.
Die Verbindung C-4 wird mit Natriumhydrid und Allylbromid in Dimethylformamid bei Raumtemperatur zur Verbindung C-5 umgesetzt. Die Verbindung C-5 wird mit Osmiumtetroxid und Natriumperiodat in Diethylether zur Verbindung C-6 umgesetzt. Diese wird in einer Homer-Emmons-Wadsworth-Reaktion mit Natriumhydrid und der Verbindung C-7 zur Verbindung C-8 umgesetzt.
Die Verbindung C-8 wird zur freien Säure verseift, indem sie mehrere Stunden bei Raumtemperatur mit Natriumhydroxid in Methanol gerührt wird. Die dabei erhaltenen Verbindung C-9 wird mit Wasserstoff an Palladium/Kohle zur Verbindung C-10 hydriert. Nach diesem Verfahren können die Beispiele 1 bis 4 synthetisiert werden.

### Verfahren D:

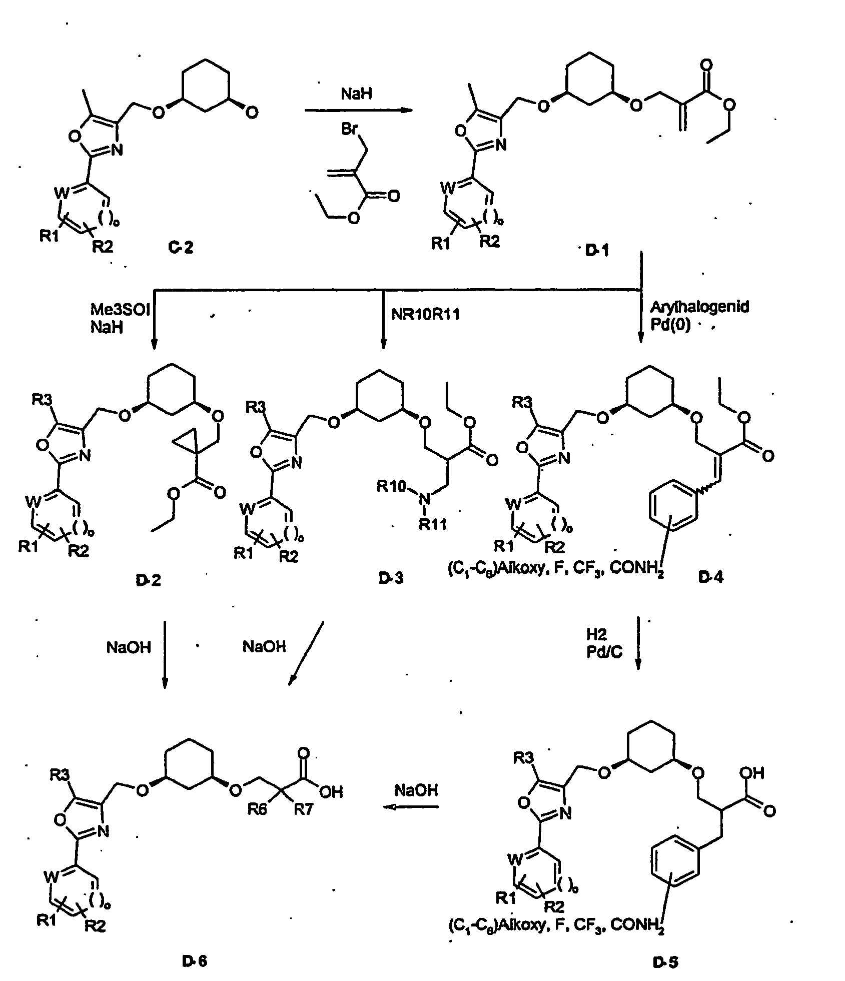

Die Verbindung C-2 wird mit Natriumhydrid und 2-Brommethylacrylsäureethylester in Dimethylformamid bei 0°C zur Verbindung D-1 umgesetzt.
Die Verbindung D-1 wird nun entweder mit Trimethylsulfoniumiodid und Natriumhydrid in Dimethylsulfoxid zur Verbindung D-2, oder mit einem sekundären Amin NR10R11, worin R10 und R11 die oben beschriebenen Bedeutungen haben, zur Verbindung D-3 oder mit einem Arylhalogenid und einem Palladium(0)-Katalysator in einer Heck-Reaktion zur Verbindung D-4 umgesetzt. Die Verbindung D-4 wird dann mit Wasserstoff an Palladium auf Kohle zur Verbindung D-5 hydriert.

Die Verbindungen D-2, D-3 und D-5 werden mit Natriumhydroxid zu Verbindungen der allgemeinen Formel D-6 umgesetzt, wobei R6 und R7 die oben beschriebenen Bedeutungen haben.
Nach diesem Verfahren können die Beispiele 5 bis 9 synthetisiert werden.

### Verfahren E:

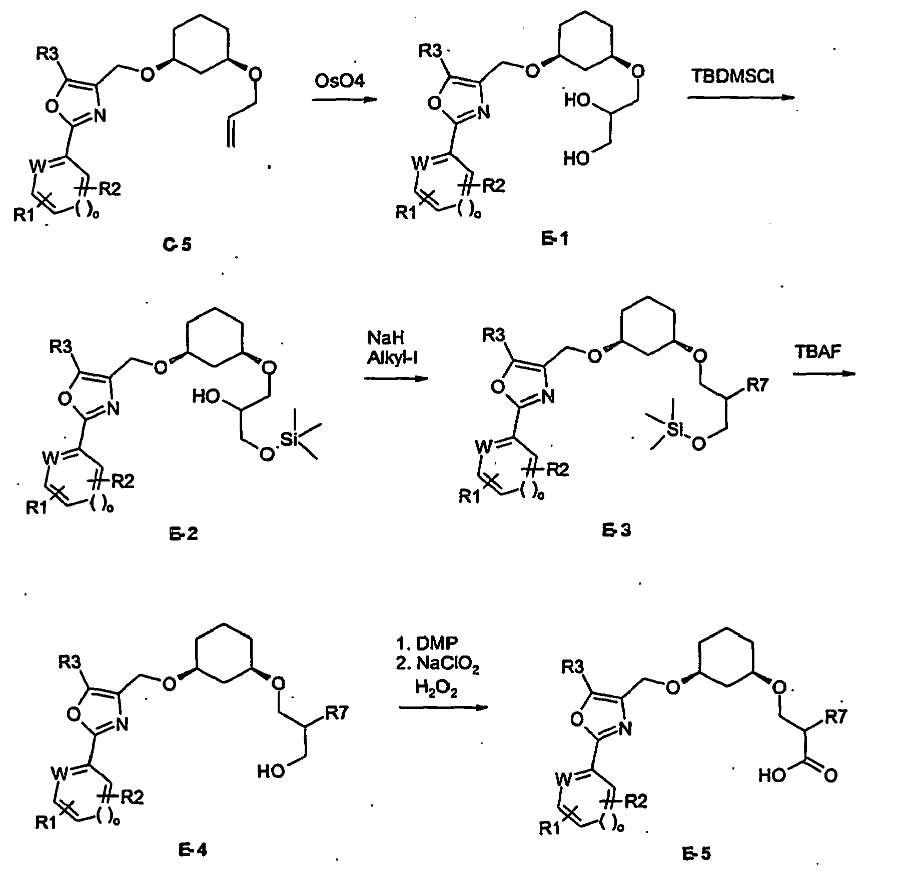

Die Verbindung C-5 wird mit Osmiumtetroxid, 1,5-Diazabicyclo[2.2.2]octan (DABCO) und N-Methylmorpholin-N-oxid zur Verbindung E-1 dihydroxyliert. Anschließend wird die primäre Hydroxylgruppe als Trialkylsilylether E-2 geschützt, indem die Verbindung E-1 mit einem Triatkylchlorsilan (z. B. tert-Butyldimethylsilylchlorid) und Imidazol als Base in Dimethylformamid bei Raumtemperatur gerührt wird. Anschließend wird die Verbindung E-2 mit einer starken Base (z. B. Natriumhydrid oder Kalium-tert-butylat) und einem Alkylhalogenid zur Verbindung E-3, worin R7 die oben beschriebene Bedeutung hat, umgesetzt. Die Silylschutzgruppe wird mit Tetrabutylammoniumfluorid in Tetrahydrofuran abgespalten, wobei die Verbindung E-4 erhalten wird.
Die Verbindung E-4 wird mit Dess-Martin-Periodinan (DMP) in Dichlormethan mehrere Stunden bei Raumtemperatur gerührt, aufgearbeitet und anschließend mit Natriumchlorit und Wasserstoffperoxid in Acetonitril zur Verbindung E-5 umgesetzt. Alternativ kann E-5 durch direkte Oxidation von E-4 mit einer Chrom-(VI)-Verbindung (z. B. CrO3 in Schwefelsäure) aus E-4 synthetisiert werden.
Nach diesem Verfahren können die Beispiele 10 bis 27 synthetisiert werden.

### Verfahren F:

Dieses Verfahren dient zur alternativen Herstellung der Zwischenstufen E-3 (s. Verfahren E)

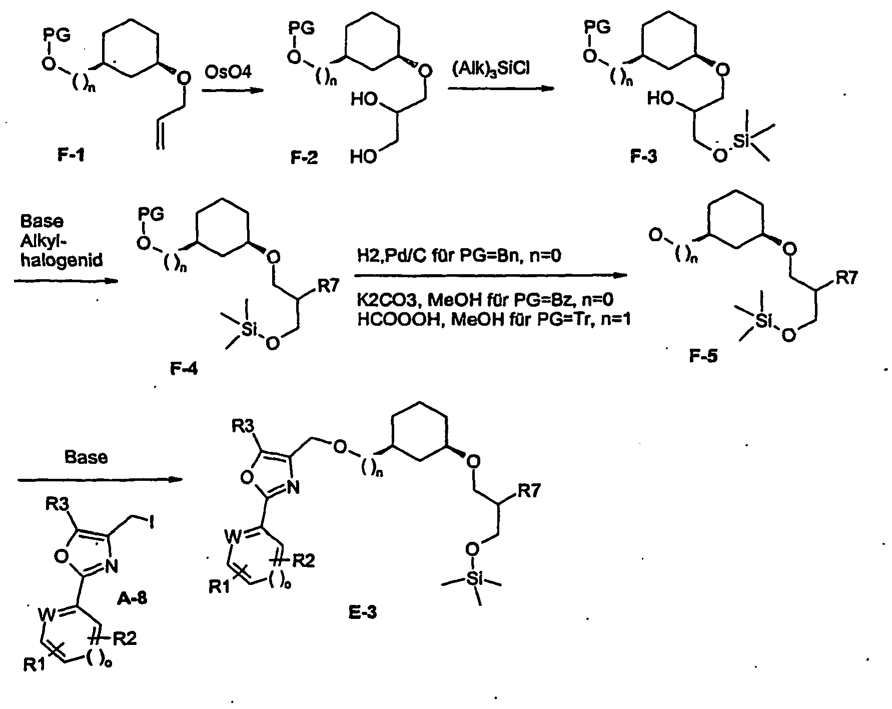

Die racemische oder enantiomerenreine Verbindung F-1, worin PG die im Schema angegebene Bedeutung hat, wird mit Osmiumtetroxid, 1,5-Diazabicyclo[2.2.2]octan (DABCO) und N-Methylmorpholin-N-oxid zur Verbindung F-2 dihydroxyliert. Anschließend wird die primäre Hydroxylgruppe als Trialkylsilylether F-3 geschützt, indem die Verbindung F-2 mit einem Trialkylchlorsilan (z. B. tert-Butyldimethylsilylchlorid) und Imidazol als Base in Dimethylformamid bei Raumtemperatur gerührt wird. Anschließend wird die Verbindung F-3 mit einer starken Base (z. B. Natriumhydrid oder Kalium-tert-butylat) und einem Alkylhalogenid zur Verbindung F-4, worin R7 die oben beschriebene Bedeutung hat, umgesetzt. Die Benzylschutzgruppe wird mit Wasserstoff an Pd (10% auf Kohle) hydrogenolytisch abgespalten, die Benzoylschutzgruppe wird mit Kaliumcarbonat in Methanol und die Tritylgruppe mit Ameisensäure in Methanol abgespalten, wobei die Verbindung F-5 erhalten wird.
Die Verbindung F-5 wird mit dem Alkyliodid A-8 in Gegenwart einer starken Base (beispielsweise Natriumhydrid oder Kalium-tert-butylat) in einem inerten Lösungsmittel (z. B. MTBE, Chlorbenzol) zur Verbindung E-3, worin R1, R2, R3, R7 und W die oben angegebene Bedeutung haben, umgesetzt.
Die anschließenden Umsetzungen von E-3 nach E-5 erfolgen wie unter Verfahren E beschrieben.
Nach diesem Verfahren können die Beispiele 28 bis 90 synthetisiert werden.

### Verfahren G:

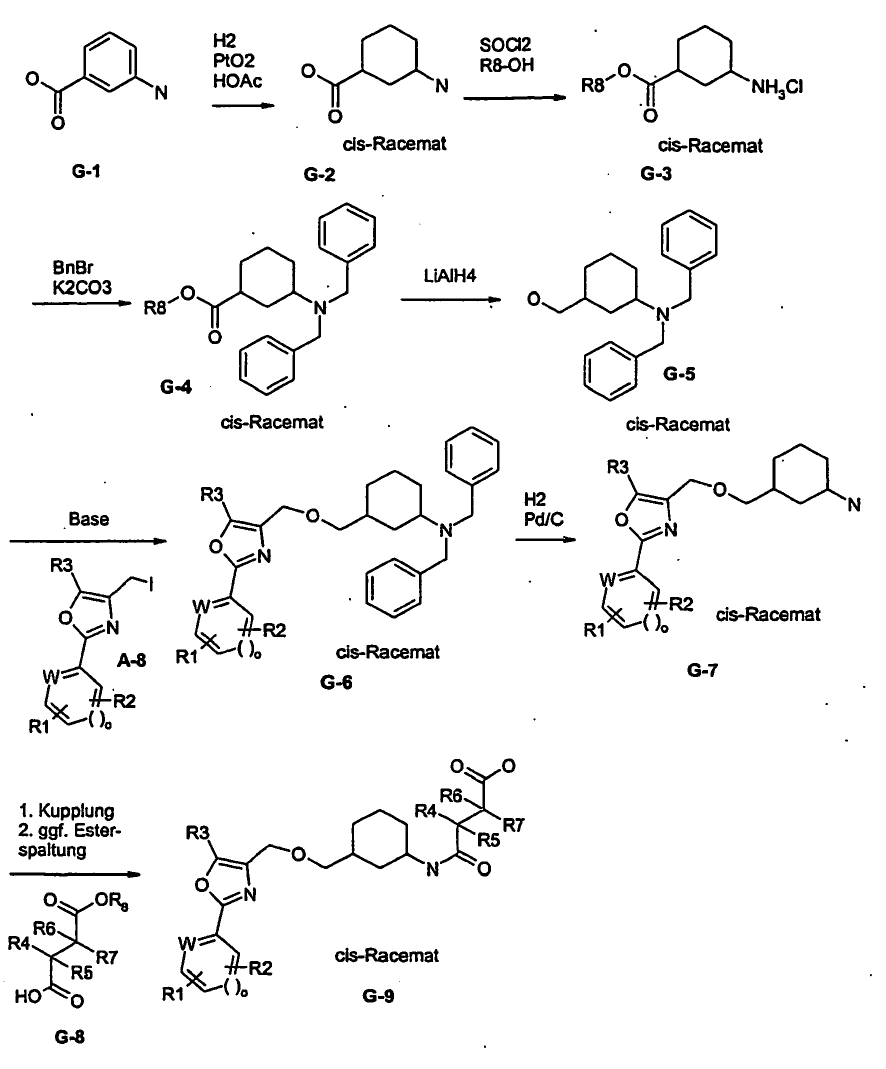

Die Verbindung G-1 (3-Aminobenzoesäure) wird in Essigsäure mit Wasserstoff an Platindioxid bei erhöhtem Druck hydriert, wobei die Verbindung G-2 erhalten wird. Diese wird mit Thionylchlorid in einem Alkohol R80H, worin R8 die oben angegebene Bedeutung (außer R8 = H) hat, in einen Ester überführt, wobei die Verbindung G-3 erhalten wird. Anschließend wird die Verbidnung G-3 mit Benzylbromid und Kaliumcarbonat zum Dibenzylamin G-4 umgesetzt. Reduktion von G-4 mit LiAIH4 liefert den Alkohol G-5.
Die Verbindung G-5 wird mit einem Alkyliodid A-8 zur Verbindung G-6, worin R1, R2, W und R3 die oben angegebene Bedeutung haben, umgesetzt. Die Verbindung G-6 wird mit Wasserstoff an Palladium zur Verbindung G-7, worin R1, R2, W und R3 die oben angegebene Bedeutung haben, hydriert.
Die Verbindung G-7 wird mit Carbonsäurederivaten G-8, worin R4, R5, R6 und R7 die oben angegebene Bedeutung haben, gekuppelt. Werden Dicarbonsäuremonoester G-8 eingesetzt, so schließt sich an die Kupplung eine Esterspaltung an (z. B. mit LiOH in THF/Methanol/Wasser für R8 = Methyl oder Ethyl). Dabei wird die Verbindung G-9, worin R1, R2, W, R3, R4, R5, R6 und R 7 die oben angegebene Bedeutung haben, erhalten.
Nach diesem Verfahren können die Beispiele 28 bis 90 synthetisiert werden.

Die verwendeten Abkürzungen stehen für:
- Ac: Acetyl
- Bn: Benzyl
- ⁱBu: Isobutyl
- ^{t}Bu: tert-Butyl
- BuLi: n-Butyllithium
- Bz: Benzoyl
- Cy: Cyclohexyl
- DC: Dünnschichtchromatographie
- DCI: direkte chemische lonisation (bei MS)
- DCM: Dichlormethan
- DMAP: 4-N,N-Dimethylaminopyridin
- DMF: N,N-Dimethylformamid
- DMSO: Dimethylsulfoxid
- EE: Essigsäureethylester
- EDC: N'-(3-Dimethylaminopropyl)-N-ethylcarbodiimid x HCl
- EI: Elektronenstoß-Ionisation (bei MS)
- eq: Äquivalent
- ESI: Elektronenspray-lonisation (bei MS)
- Et: Ethyl
- ges.: gesättigt
- h: Stunde
- HATU: O-(7-Azabenzotriazol-1-yl)-N,N,N',N'tetramethyluronium-Hexafluorophosphat
- HOBt: 1-Hydroxy-1H-benzotriazol x H₂O
- HPLC: Hochdruck-, Hochleistungsflüssigchromatographie
- LC-MS: Flüssigchromatographie-gekoppelte Massenspektroskopie
- Me: Methyl
- MS: Massenspektroskopie
- MsCl: Methansulfonylchlorid
- NMR: Kernresonanzspektroskopie
- Pd/C: Palladium auf Kohle
- ⁱPr: Isopropyl
- ⁿPr: n-Propyl
- R_{f}: Retentionszeit (bei DC)
- RT: Raumtemperatur
- TBAF: Tetrabutylammoniumfluorid
- TBAI: Tetrabutylammoniumiodid
- TBDPSCI: tert.Butyldiphenylsilylchlorid
- TBDMSCI: tert.Butyldimethylsilylchlorid
- THF: Tetrahydrofuran
- Tr: Trityl

Andere Verbindungen können entsprechend den oben genannten Verfahren hergestellt werden.

### Bausteinsynthese nach Verfahren A:

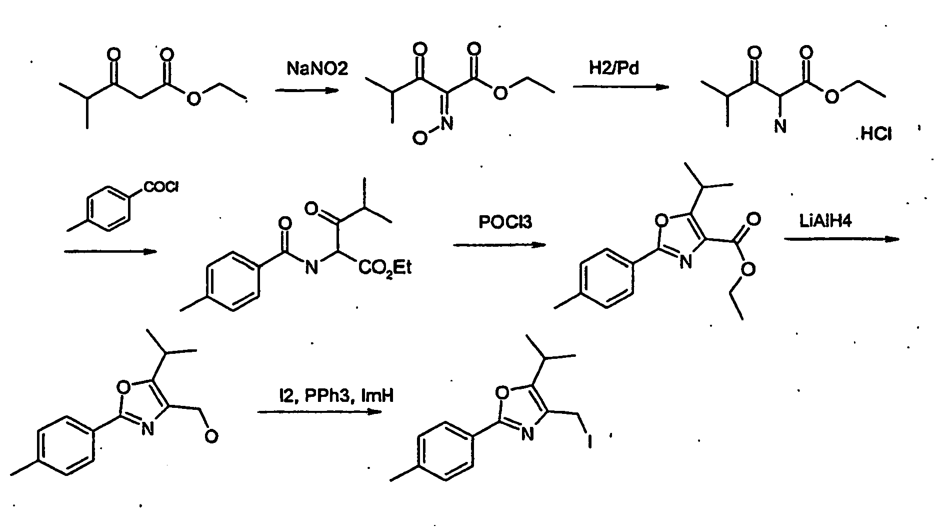

### 2-Hydroxyimino-4-methyl-3-oxo-pentansäureethylester

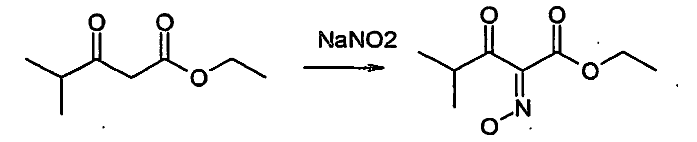

42.4 g 4-Methyl-3-oxo-pentansäureethylester werden in 100 ml Eisessig gelöst und bei 5°C mit 21 g Natriumnitrit, gelöst in 100 ml Wasser, versetzt. Man lässt innerhalb einer Stunde auf Raumtemperatur erwärmen, fügt sodann 100 ml Wasser hinzu und rührt eine weiter Stunde bei Raumtemperatur nach. Man extrahiert dreimal mit je 150 ml Methyl-tert-butylether, die vereinigten organischen Phasen werden mit 200 ml Wasser versetzt und durch Zugabe von festem NaHCO3 neutralisiert. Die organische Phase wird abgetrennt, mit gesättigter NaCl-Lösung gewaschen, über MgSO4 getrocknet und anschließend das Lösungsmittel im Vakuum entfernt. Man erhält 46 g 2-Hydroxyimino-4-methyl-3-oxo-pentansäure-ethylester als Öl. C8H13NO4 (187.20), MS(ESI) = 188 (M+H⁺).

### 2-Amino-4-methyl-3-oxo-pentansäureethylesterhydrochlorid

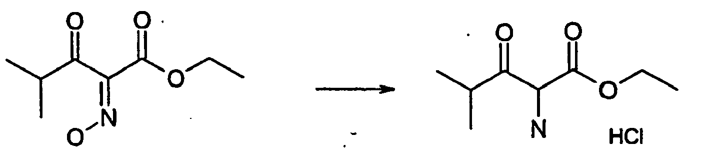

In 200 ml Ethanol werden 10 g HCl eingeleitet. 46 g 2-Hydroxyimino-4-methyl-3-oxo-pentansäureethylester werden darin gelöst und mit 5 g Pd(10% auf Kohle) versetzt und 8 Stunden unter einer Wasserstoffatmosphäre (5 bar) gerührt. Das Reaktionsgemisch wird über Celite filtriert und das Lösungsmittel im Vakuum entfernt. Man erhält 45 g 2-Amino-4-methyl-3-oxo-pentansäureethylester hydrochlorid als weißen Feststoff. C8H15N03*HCl (209.5), MS(ESI) = 188 (M+H⁺).

### 4-Methyl-2-(4-methyl-benzoylamino)-3-oxo-pentansäureethylester

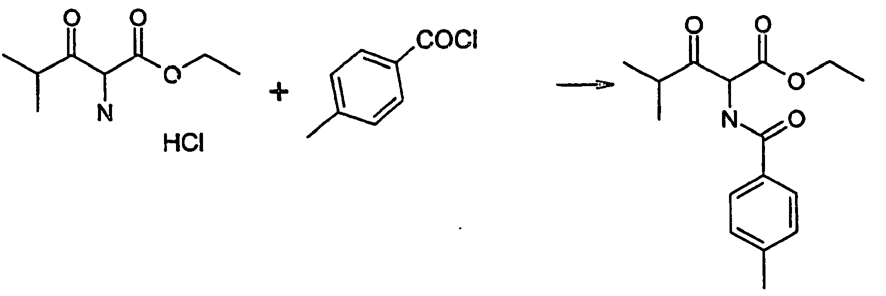

10 g 2-Amino-4-methyl-3-oxo-pentansäureethylesterhydrochlorid und 7.4 g 4-Methyl-benzoylchlorid werden in 250 ml Dichlormethan gelöst und bei 0°C langsam und tropfenweise mit 13.3 ml Triethylamin versetzt. Man rührt eine Stunde bei Raumtemperatur nach, dann wird mit Wasser gewaschen, die organische Phase abgetrennt, über MgSO4 getrocknet und anschließend das Lösungsmittel im Vakuum entfernt. Man erhält 13 g 4-Methyl-2-(4-methyl-benzoylamino)-3-oxo-pentansäureethylester als Öl.
C16H21N04 (291.35), MS(ESI) = 292 (M+H⁺).

### 5-Isopropyl-2-p-tolyhxazol-4-carbonsäureethylester

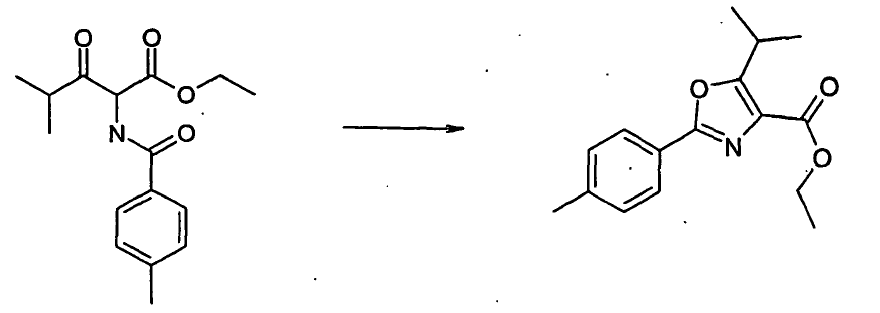

13 g 4-Methyl-2-(4-methyl-benzoylamino)-3-oxo-pentansäureethylester werden in 80 ml Phosphoroxychlorid 2h unter Rückfluss zum Sieden erhitzt. Das Phosphoroxychlorid wird im Vakuum entfernt und der resultierende Rückstand in 200 ml Dichlormethan gelöst , dreimal mit gesättigter NaHCO₃-Lösung gewaschen, über MgSO4 getrocknet und anschließend das Lösungsmittel im Vakuum entfernt. Man erhält 11 g 5-Isopropyl-2-p-tolyl-oxazol-4-carbonsäureethylester als bräunlichen Feststoff.C16H19NO3 (273.33), MS(ESI) = 292 (M+H⁺), Rf(n-Heptan:Ethylacetat) = 2:1) = 0.43.

### (5-Isopropyl-2-p-tolyl-oxazol-4-yl)-methanol

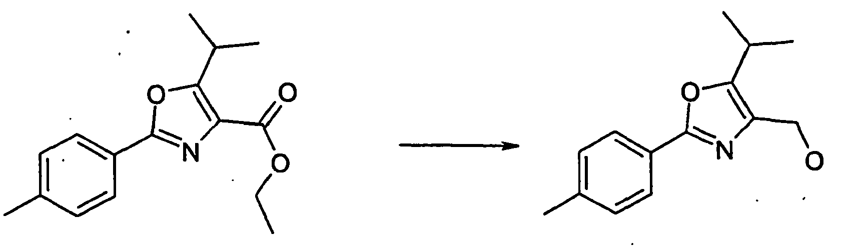

11 g 5-Isopropyl-2-p-tolyl-oxazol-4-carbonsäureethylester werden in 100 ml Tetrahydrofuran gelöst und bei 0°C mit 40 ml einer 1 molaren Lösung von Lithiumaluminiumhydrid in Tetrahydrofuran versetzt. Nach 30 min wird das Reaktionsgemisch mit 50 ml 1N HCl versetzt und fünfmal mit Ethylacetat extrahiert. Die vereinigten organischen Phasen werden über MgSO4 getrocknet und anschließend das Lösungsmittel im Vakuum entfernt. Der Rückstand wird an Kieselgel mit dem Laufmittel n-Heptan:Ethylacetat = 6:1 => 1:1 gereinigt. Man erhält 4.3 g (5-Isopropyl-2-p-tolyl-oxazol-4-yl)-methanol als hellgelben Feststoff. C14H17NO2 (231.30), MS(ESI) = 232 (M+H⁺), Rf(n-Heptan:Ethylacetat) = 1:1) = 0.17.

### 4-lodmethyl-5-isopropyl-2-p-tolyloxazol

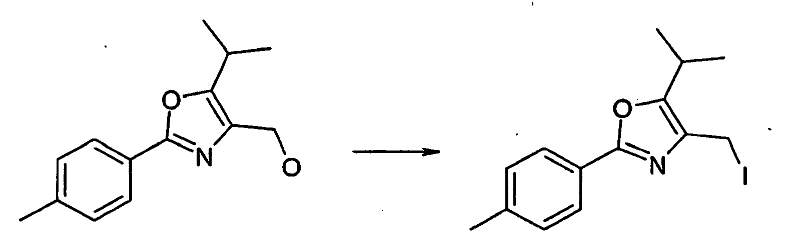

500 mg (5-Isopropyl-2-p-tolyl-oxazol-4-yl)-methanol werden zusammen mit 690 mg Triphenylphosphin und 600 mg Imidazol in 20 ml Toluol gelöst. Man gibt 715 mg lod hinzu und rührt 1 Stunde bei Raumtemperatur nach. Dann wird 10 ml gesättigte Natriumcarbonat-Lösung und 500 mg lod nachgegeben. Nach 10 Minuten wird die organische Phase abgetrennt und zweimal mit gesättigter Na2S2O3-Lösung gewaschen, über MgSO4 getrocknet und anschließend die lösungsmittel im Vakuum entfernt. Der Rückstand wird an Kieselgel mit dem Laufmittel n-Heptan:Ethylacetat = 10:1 gereinigt. Man erhält 400 mg 4-lodmethyl-5-isopropyl-2-p-tolyl-oxazol als weißen Feststoff. C14H161NO (341.19), MS(ESI): 342 (M+H⁺), Rf(n-Heptan:Ethylacetat = 1:1) = 0.75.

Analog zur Bausteinsynthese nach Verfahren K wurde aus 2-Amino-4-methyl-3-oxo-pentansäureethylester hydrochlorid und 3-Methoxy-benzoylchlorid 4-lodmethyl-2-(3-methoxy-phenyl)-5-isopropyl-oxazol erhalten. 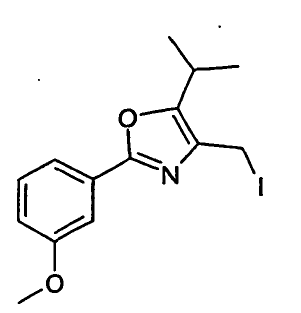 C14H16INO2 (357.19), MS(ESI): 358 (M+H⁺), Rf(n-Heptan:Ethylacetat = 1:1) = 0.60.

Analog zur Bausteinsynthese von 4-lodmethyl-5-isopropyl-2-p-tolyl-oxazol wurde aus 4,4,4-Trifluoro-3-oxo-buttersäureethylester und 3-Methoxy-benzoylchlorid 4-lodmethyl-2-(3-methoxy-phenyl)-5-trifluormethyl-oxazol erhalten. 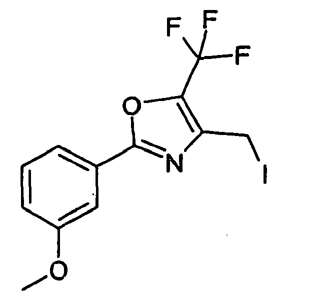 C12H9F3INO2 (383.11), MS(ESI): 384 (M+H⁺).

Analog zur Bausteinsynthese von 4-lodmethyl-5-isopropyl-2-p-tolyl-oxazol wurde aus 4,4,4-Trifluoro-3-oxo-buttersäureethylester und 3-Trifluormethylbenzoylchlorid 4-lodmethyl-2-(3-trifluormethyl -phenyl)-5-trifluormethyl-oxazol erhalten. 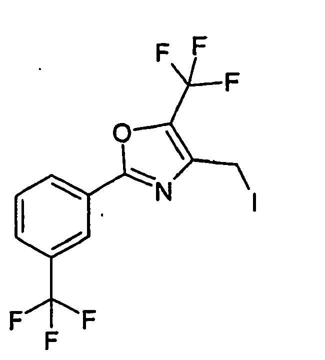 C12H6F6INO (421.08), MS(ESI): 422 (M+H⁺).

Analog zur Bausteinsynthese von 4-lodmethyl-5-isopropyl-2-p-tolyl-oxazol wurde aus 4,4,4-Trifluoro-3-oxo-buttersäureethylester und 4-Methyl-benzoylchlorid 4-Iodrnethyl-5-trifluormethyl-2-p-tolyl-oxazol erhalten. 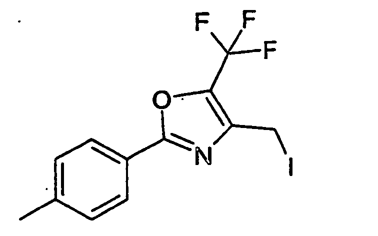 C12H9F3INO (367.11), MS(ESI): 368 (M+H⁺).

### Bausteinsynthese nach Verfahren B:

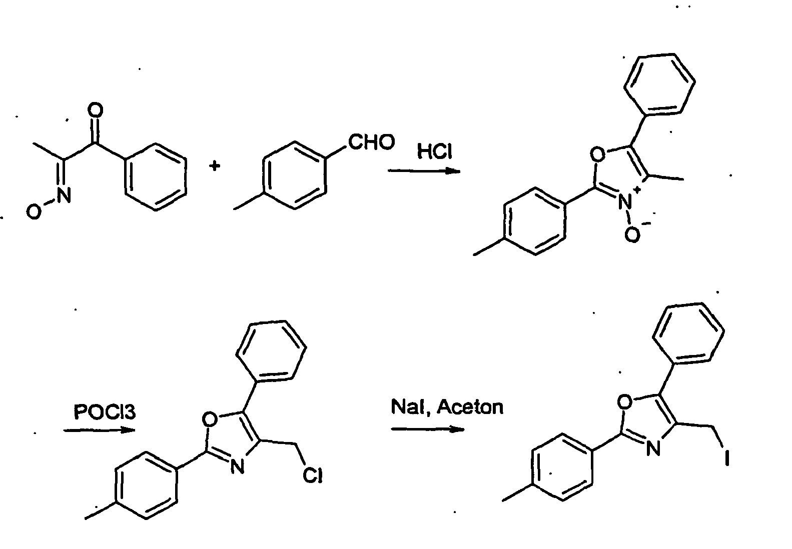

### 4-Methyl-5-phenyl-2-p-tolyl-oxazol 3-oxid

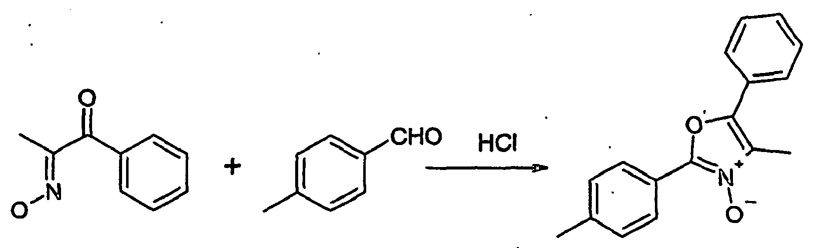

12.5 g 1-Phenyl-1,2-propandion-2-oxim und 10ml p-Toluolaldehyd werden in 50 ml Eisessig gegeben und 30 Minuten unter Eiskühlung HCl Gas durchgeleitet. Durch Zugabe von Methyl-tert-butylether wird das Produkt als Hydrochlorid ausgefällt abgesaugt und der Niederschlag mit Methyl-tert-butylether gewaschen. Man suspendiert den Niederschlag in Wasser und stellt mit Ammoniak einen basischen pH-Wert ein. Es wird dreimal mit je 200 ml Dichlormethan extrahiert, die vereinigten organischen Phasen werden über MgS04 getrocknet und anschließend das Lösungsmittel im Vakuum entfernt. Man erhält 6.4 g 4-Methyl-5-phenyl-2-p-tolyl-oxazol 3-oxid als weißen Feststoff. C17H15NO2 (265.31), MS(ESI) = 266 (M+H⁺).

### 4-Chlormethyl-5-phenyl-2-p-tolyl-oxazol

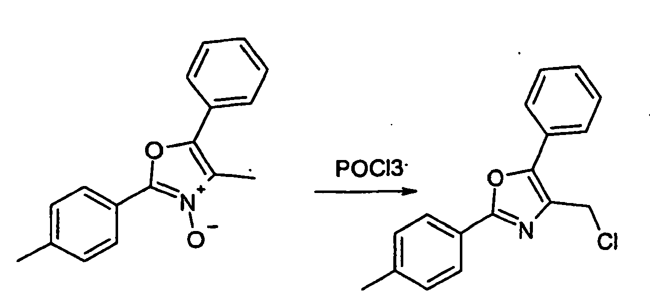

6.4 g 4-Methyl-5-phenyl-2-p-tolyl-oxazol 3-oxid werden in 50 ml Chloroform gelöst, mit 2.4 ml Phosphoroxychlorid versetzt und 30 Minuten unter Rückfluss zum Sieden erhitzt. Das Reaktionsgemisch wird auf 0°C abgekühlt, mit Ammoniak ein schwach alkalischer pH-Wert eingestellt und dreimal mit je 100 ml Ethylacetat extrahiert. Die vereinigten organischen Phasen werden mit Wasser gewaschen, über MgSO4 getrocknet und anschließend das Lösungsmittel im Vakuum entfernt. Man erhält 5.4 g 4-Chlormethyl-5-phenyl-2-p-tolyl-oxazol als gelben Feststoff. C17H14CINO (283.76), MS(ESI) = 284 (M+H⁺), Rf(n-Heptan:Ethylacetat) = 7:1) = 0.41.

### 4-Iodmethyl-5-phenyl-2-p-tolyl-oxazol

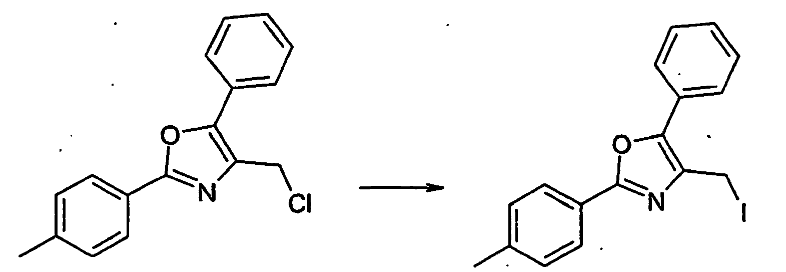

1.8 g 4-Chlormethyl-5-phenyl-2-p-tolyl-oxazol werden zusammen mit 3 g Natriumiodid in 150 ml Aceton 2 Stunden unter Rückfluss zum Sieden erhitzt. Nach dem Abkühlen des Reaktionsgemischs wird 300 ml Methyl-tert-butylether zugefügt, das Gemisch dreimal mit gesättigter Na2S2O3-Lösung gewaschen, über MgSO4 getrocknet und anschließend die Lösungsmittel im Vakuum entfernt. Man erhält 2.7 g 4-lodmethyl-5-phenyl-2-p-tolyl-oxazol als hellgelben Feststoff. C17H141NO (375.21), MS(ESI): 376 (M+H⁺).

Analog zur Bausteinsynthese von 4-Iodmethyl-5-phenyl-2-p-tolyl-oxazol wurde aus 1-Phenyl-1,2-propandion-2-oxim und m-Anisaldehyd 4-lodmethyl-2-(3-methoxy-phenyl)-5-phenyl-oxazol erhalten. 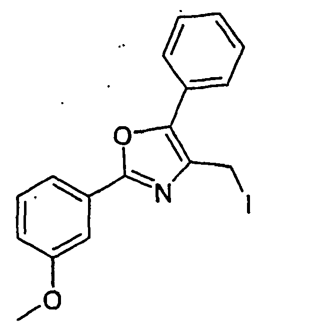 C17H14INO2 (391.21), MS(ESI): 392 (M+H⁺).

Analog zur Bausteinsynthese von 4-Iodmethyl-5-phenyl-2-p-tolyl-oxazol wurde aus 1-. Ethyl-1,2-propandion-2-oxim und m-Anisaldehyd 4-Iodmethyl-5-ethyl-2-(3-methoxyphenyl)-oxazol erhalten. 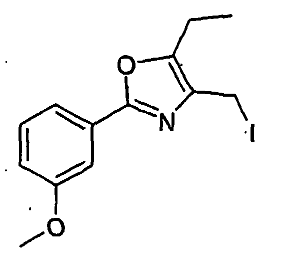 C13H14INO2 (343.17), MS(ESI): 344 (M+H⁺).

Analog zur Bausteinsynthese von 4-lodmethyl-5-phenyl-2-p-tolyl-oxazol wurde aus 1-Ethyl-1,2-propandion-2-oxim und p-Toluolaldehyd 4-Iodmethyl-5-ethyl-2-p-tolyl-oxazol erhalten. 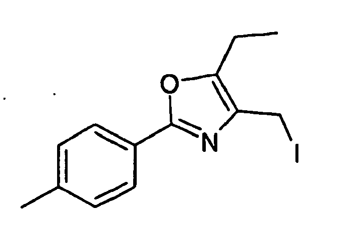 C13H141NO (327.17), MS(ESI): 328 (M+H⁺).

Analog zur Bausteinsynthese von 4-Iodmethyl-5-phenyl-2-p-tolyl-oxazol wurde aus 1-Ethyl-1,2-propandion-2-oxim und 2,6-Dimethylbenzaldehyd 4-Iodmethyl-5-ethyl-2-(2,6-dimethylphenyl)-oxazol erhalten. 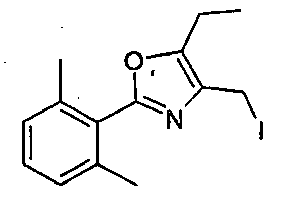 C14H16INO (341,19), MS(ESI): 342 (M+H⁺).

Analog zur Bausteinsynthese von 4-Iodmethyl-5-phenyl-2-p-tolyl-oxazol wurde aus 1-Ethyl-1,2-propandion-2-oxim und 2-Trifluormethylbenzaldehyd 4-lodmethyl-5-ethyl-2-(2-trifluormethylphenyl)-oxazol erhalten. 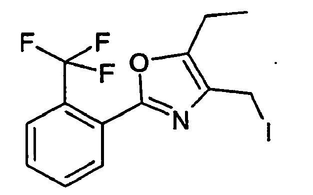 C13H11F3INO (381,14), MS(ESI): 382 (M+H⁺).

Analog zur Bausteinsynthese von 4-Iodmethyl-5-phenyl-2-p-tolyl-oxazol wurde aus 1-Cyclohexyl-1,2-propandion-2-oxim und m-Anisaldehyd 4-Iodmethyl-5-cyclohexyl-2-(3-methoxy-phenyl)-oxazol erhalten. 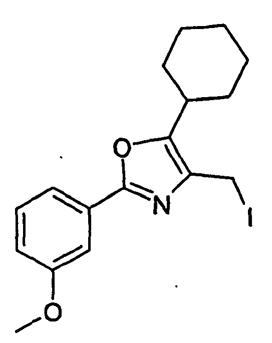 C17H20INO2 (397.26), MS(ESI): 398 (M+H⁺).

Analog zur Bausteinsynthese von 4-Iodmethyl-5-phenyl-2-p-tolyl-oxazol wurde aus 1-Cyclohexyl-1,2-propandion-2-oxim und p-Toluolaldehyd 4-lodmethyl-5-cyclohexyl-2-p-tolyl-oxazol erhalten. 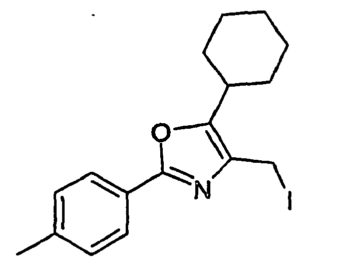 C17H20INO (381.26), MS(ESI): 382 (M+H⁺).

Analog zur Bausteinsynthese von 4-Iodmethyl-5-phenyl-2-p-tolyl-oxazol wurde aus Diacetylmonoxim und p-Toluolaldehyd 4-Iodmethyl-5-methyl-2-p-tolyl-oxazol erhalten. 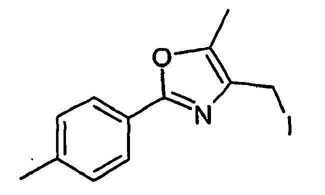 C12H12INO (313.14), MS(ESI): 314 (M+H⁺).

Analog zur Bausteinsynthese von 4-Chlormethyl-5-phenyl-2-p-tolyl-oxazol wurde aus Diacetylmonoxim und Benzaldehyd 4-Chlormethyl-5-methyl-2-phenyl-oxazol erhalten. 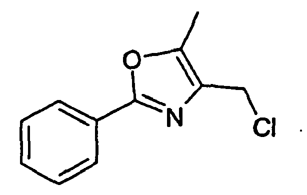 C11H10INO (207,66), MS(ESI): 208 (M+H⁺).

Analog zur Bausteinsynthese von 4-Iodmethyl-5-phenyl-2-p-biphenyl-oxazol wurde aus Diacetylmonoxim und p-Biphenylcarbaldehyd 4-lodmethyl-5-methyl-2-p-biphenyl-oxazol erhalten. 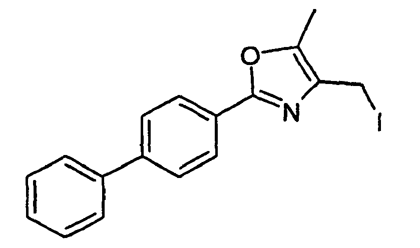 C12H12INO (375,21), MS(ESI): 376 (M+H⁺).

Analog zur Bausteinsynthese von 4-Iodmethyl-5-phenyl-2-p-tolyl-oxazol wurde aus Diacetylmonoxim und 2-Naphthalincarbaldehyd 4-Iodmethyl-5-methyl-2-naphthyl-oxazol erhalten. 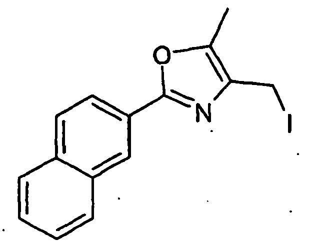 C12H12INO (349,17), MS(ESI): 350 (M+H⁺).

Analog zur Bausteinsynthese von 4-Iodmethyl-5-phenyl-2-p-tolyl-oxazol wurde aus Diacetylmonoxim und 2,4-Dimethylbenzaldehyd 4-lodmethyl-5-methyl-2-(2,4-dimethylphenyl)-oxazol erhalten. 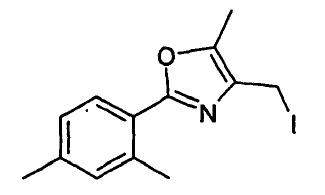 C13H14INO (327,17), MS(ESI): 328 (M+H⁺).

Analog zur Bausteinsynthese von 4-Iodmethyl-5-phenyl-2-p-tolyl-oxazol wurde aus Diacetylmonoxim und 2,6-Dimethylbenzaldehyd 4-lodmethyl-5-methyl-2-(2,6-dimethylphenyl)-oxazol erhalten. 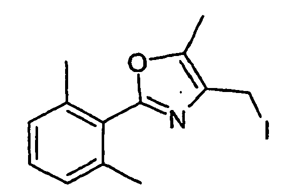 C13H14INO (327,17), MS(ESI): 328 (M+H⁺).

Analog zur Bausteinsynthese von 4-Iodmethyl-5-phenyl-2-p-tolyl-oxazol wurde aus Diacetylmonoxim und m-Anisaldehyd 4-Iodmethyl-2-(3-rnethoxy-phenyl)-5-methyl-oxazol erhalten. 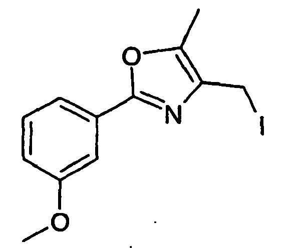 C12H12INO2 (329:14), MS(ESI): 330 (M+H⁺).

Analog zur Bausteinsynthese von 4-Iodmethyl-5-phenyl-2-p-tolyl-oxazol wurde aus Diacetylmonoxim und 3-Trifluormethylbenzaldehyd 4-lodmethyl-5-methyl-2-(3-trifluoromethyl-phenyl)-oxazol erhalten. 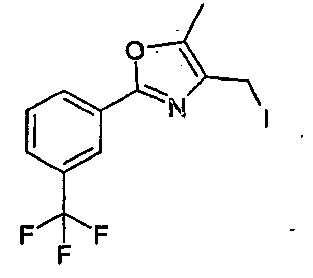 C12H9F3INO (367.11), MS(ESI): 368 (M+H⁺).

Analog zur Bausteinsynthese von 4-Iodmethyl-5-phenyl-2-p-tolyl-oxazol wurde aus Diacetylmonoxim und 4-Fluorbenzaldehyd 2-(4-Fluoro-phenyl)-4-iodmethyl-5-methyl-oxazol erhalten. 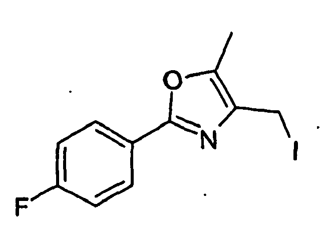 C11H9FINO (317.10), MS(ESI):318 (M+H+).

Analog zur Bausteinsynthese von 4-Iodmethyl-5-phenyl-2-p-tolyl-oxazol wurde aus Diacetylmonoxim und 4-Methoxybenzaldehyd 4-lodmethyl-2-(4-methoxy-phenyl)-5-methyl-oxazol erhalten. 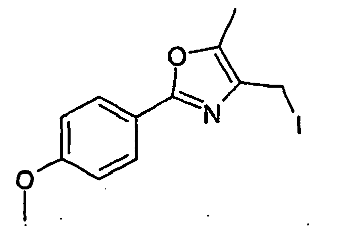 C12H12INO2 (329.14), MS(ESI):330 (M+H+).

Analog zur Bausteinsynthese von 4-Iodmethyl-5-phenyl-2-p-tolyl-oxazol wurde aus Diacetylmonoxim und 4-Trifluormethylbenzaldehyd 4-lodmethyl-5-methyl-2-(4-trifluoromethyl-phenyl)-oxazol erhalten. 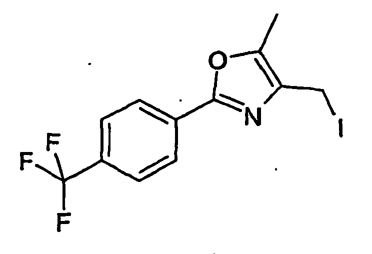 C12H9F3INO (367.11), MS(ESI):368 (M+H+).

Analog zur Bausteinsynthese von 4-Iodmethyl-5-phenyl-2-p-tolyl-oxazol wurde aus Diacetylmonoxim und 2-Trifluonnethylbenzaldehyd 4-Iodmethyl-5-methyl-2-(2-trifluoromethyl-phenyl)-oxazol erhalten. 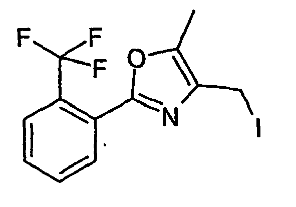 C12H9F3INO (367.11), MS(ESI):368 (M+H+).

Analog zur Bausteinsynthese von 4-Iodmethyl-5-phenyl-2-p-tolyl-oxazol wurde aus Diacetylmonoxim und m-Toluolaldehyd 4-Iodmethyl-5-methyl-2-m-tolyl-oxazol erhalten. 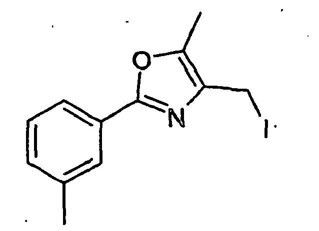 C12H12INO (313.14), MS(ESI):314 (M+H+).

Analog zur Bausteinsynthese von 4-Iodmethyl-5-phenyl-2-p-tolyl-oxazol wurde aus Diacetylmonoxim und 3-Trifluormethoxybenzaldehyd 4-Iodmethyl-5-methyl-2-(3-trifluoromethoxy-phenyl)-oxazol erhalten. 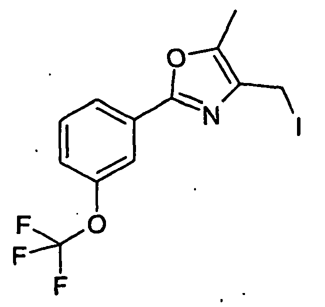 C12H9F3INO2 (383.11), MS(ESI):384 (M+H+).

Analog zur Bausteinsynthese von 4-Iodmethyl-5-phenyl-2-p-tolyi-oxazol wurde aus Diacetylmonoxim und -5-Methylfuran-2-carbaldehyd 4-Iodmethyl-5-methyl-2-(5-methylfuran-2-yl)-oxazol erhalten. 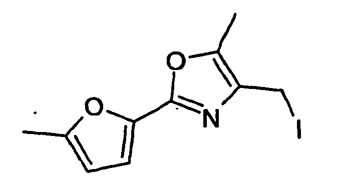 C10H10INO2 (303.11), MS(ESI):304 (M+H+).

Analog zur Bausteinsynthese von 4-Iodmethyl-5-phenyl-2-p-tolyl-oxazol wurde aus Diacetylmonoxim und Thiophen-2-carbaldehyd 4-lodmethyl-5-methyl-2-thiophen-2-yl-oxazol erhalten. 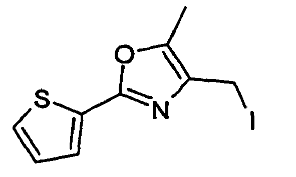 C9H8INOS (305.14), MS(ESI):306 (M+H+).

Analog zur Bausteinsynthese von 4-Iodmethyl-5-phenyl-2-p-tolyl-oxazol wurde aus Diacetylmonoxim und 4-Isopropylbenzaldehyd 4-lodmethyl-2-(4-isopropyl-phenyl)-5-methyl-oxazol erhalten. 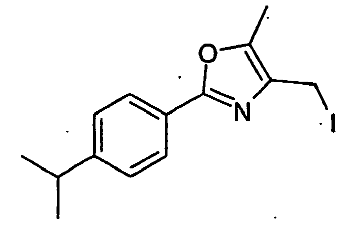 C14H16INO (341.19), MS(ESI):342 (M+H+).

### Beispiel 1:

### 2-Ethyl-4-{(1R,3S)-3-[2-(4-fluoro-phenyl)-5-methyl-oxazol-4-ylmethoxy]-cyclohexyloxy}-butansäure

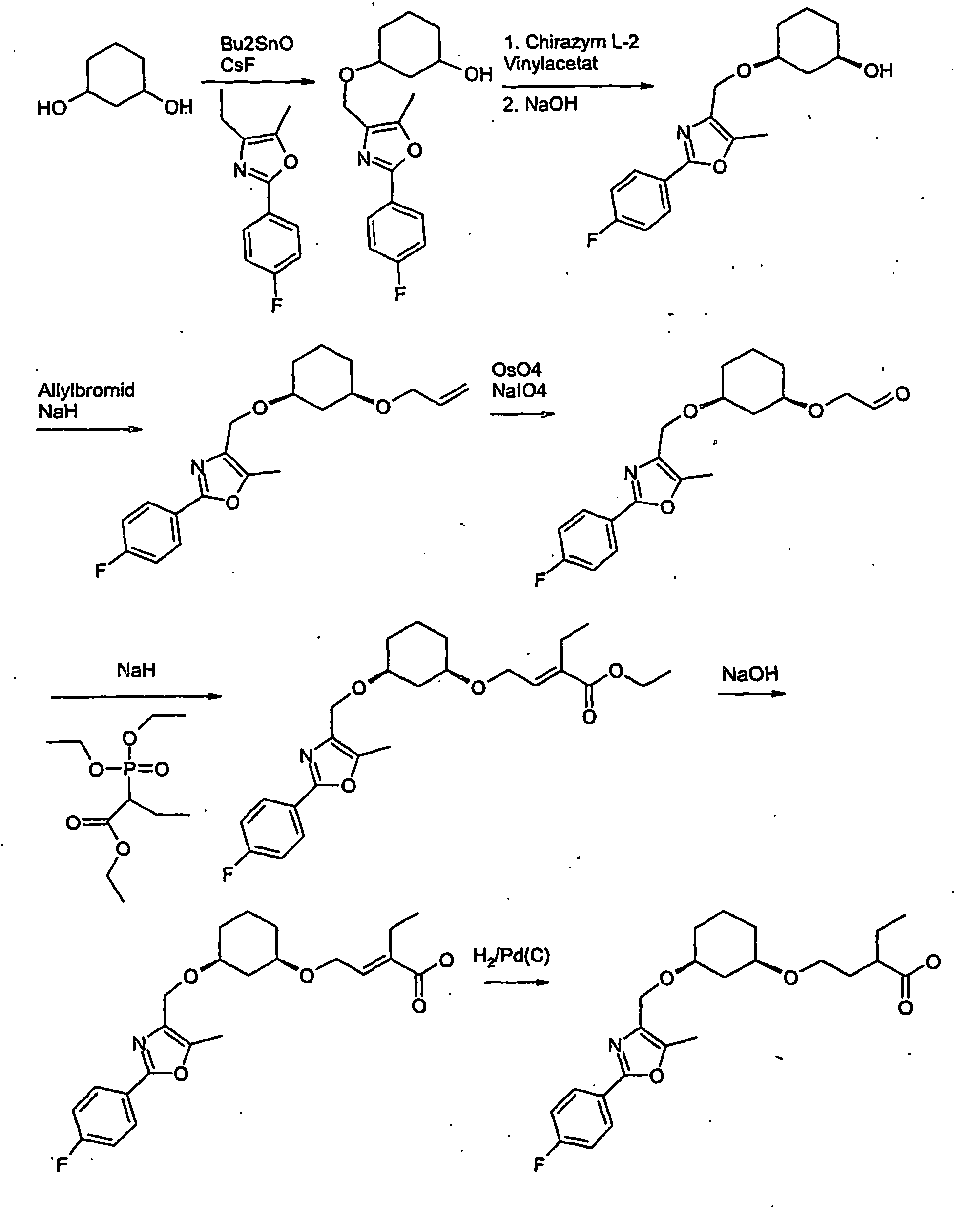

### rac-3-(cis-5-Methyl-2-m-tolyl-oxazol-4-ylmethoxy)-cyclohexanol

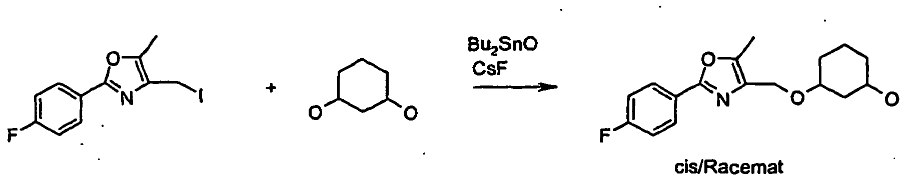

21.7 g 1,3-Cyclohexandiol werden mit 30.3 g Dibutylzinnoxid in 450 ml Toluol gelöst und unter Rückfluss am Wasserabscheider zum Sieden erhitzt. Das Reaktionsvolumen wird während der Reaktionsdauer auf die Hälfte reduziert. Nach 3 Stunden wird das Reaktionsgemisch auf Raumtemperatur gekühlt und mit 300 ml Dimethylformamid, 29 g 2-(4-Fluoro-phenyl)-4-iodmethyl-5-methyl-oxazol und 23.5 g Cäsiumfluorid versetzt. Man rührt 18 Stunden bei Raumtemperatur nach. Das Reaktionsgemisch wird durch Zugabe von Ethylacetat verdünnt und mit gesättigter Natriumchlorid -Lösung gewaschen. Die organische Phase wird über Magnesiumsulfat getrocknet, das Lösungsmittel im Vakuum entfernt und der Rückstand durch Flash-Chromatographie an Kieselgel (n-Heptan/Ethylacetat = 10:1 -> 1:4) gereinigt. Man erhält 58 g rac-3-[2-(4-Fluoro-phenyl)-5-methyl-oxazol-4-ylmethoxy]-cyclohexanol als gelblichen Feststoff, der aus n-Heptan/Ethylacetat umkristallisiert wird. C17H20FNO3 (305.35), MS (ESI): 306 (M + H+).

### 3-[2-(4-Fluoro-phenyl)-5-methyl-oxazol-4-ylmethoxy]-cyclohexanol

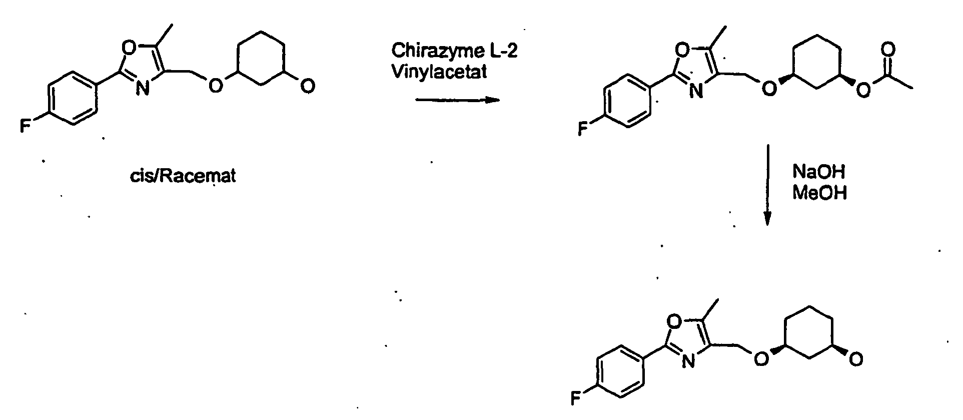

25 g rac-3-[2-(4-Fluoro-phenyl)-5-methyl-oxazol-4-ylmethoxy]-cyclohexanol werden in 320 ml Vinylacetat gelöst und mit 1,3 g Chirazyme L-2 Lyo (Boehringer Mannheim) versetzt. Nach ca. dreistündigem Rühren bei Raumtemperatur (LC-MS Kontrolle auf 40-45% Umsatz) wird das Enzym abfiltriert, mit Ethylacetat nachgewaschen und das Lösungsmittel im Vakuum entfernt. Der Rückstand wird durch Flash-Chromatographie an Kieselgel (n-Heptan/Ethylacetat = 3:1) gereinigt. Man erhält 8 g des Acetats als farbloses Öl. C19H22FNO4 (347.39), MS, (ESI): 348 (M + H+). Man nimmt das Acetat in 170 ml Methanol auf und rührt nach Zugabe von 27 ml 2N NaOH für eine Stunde bei Raumtemperatur. Der größte Teil des Lösungsmittels wird im Vakuum entfernt. Nach Zugabe von je 150 ml Wasser und Ethylacetat, wird die organische Phase mit Natriumchlorid -Lösung gewaschen. Die organische Phase wird über Magnesiumsulfat getrocknet, das Lösungsmittel im Vakuum entfernt. Man erhält 6,7 g 3-((1R,3S)-cis-3-[2-(4-Fluoro-phenyl)-5-methyl-oxazol-4-ylmethoxy]-cyclohexanol als gelblichen Feststoff. C17H20FNO3 (305.35), MS (ESI): 306 (M + H+).

### 4-(3-Allyloxy-cyclohexyloxymethyl)-2-(4-fluoro-phenyl)-5-methyl-oxazol

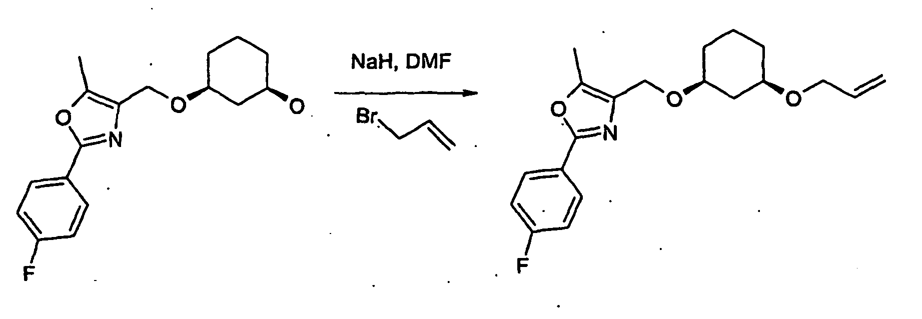

2 g des 3-[2-(4-Fluoro-phenyl)-5-methyl-oxazol-4-ylmethoxy]-cyclohexanols werden in 15 ml Dimethylformamid gelöst und mit 0.3 g Natriumhydrid versetzt. Nach 30 Minuten werden 2.4 g Allylbromid zugetropft. Man rührt 5 Stunden bei Raumtemperatur nach. Dann wird 15 ml 1 N HCl zum Reaktionsgemisch gegeben und dreimal mit 15 ml Ethylacetat gewaschen. Die organische Phase wird über Magnesiumsulfat getrocknet und anschließend das Lösungsmittel im Vakuum entfernt. Der Rückstand wird mittels RP-HPLC gereinigt. Man erhält 2.4 g 4-(3-Allyloxy-cyclohexyloxymethyl)-2-(4-fluorophenyl)-5-methyl-oxazol als gelbliches Öl. C20H24FNO3 (345,42) MS(ESI): 346 (M+H+)

### [3-[2-(4-Fluoro-phenyl)-5-methyl-oxazol-4-ylmethoxy]-cyclohexyl]-acetaldehyd

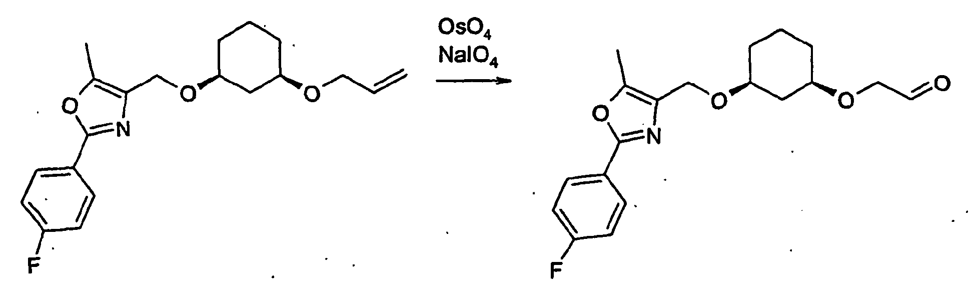

2.0 g 4-(3-Allyloxy-cyclohexyloxymethyl)-2-(4-fluoro-phenyl)-5-methyl-oxazol werden in 50 ml Diethylether gelöst und mit 3.8 g Natriumperiodat, gelöst in 50 ml Wasser versetzt. Man gibt bei 0 °C 1 ml einer Osmiumtetroxid-Lösung (2.5 Gewichts% in tert-Butanol) hinzu und rührt kräftig bei Raumtemperatur nach. Nach 8 h wird 100 ml Methyl-tert-butylether zugegeben und mit einer gesättigten Natriumthiosulfat-Lösung gewaschen. Die organische Phase wird über Magnesiumsulfat getrocknet und das Lösungsmittel im Vakuum entfernt. Der Rückstand wird an Kieselgel (n-Heptan:Ethylacetat = 1:1 → 1:5) gereinigt. Man erhält 1.4 g des [3-[2-(4-Fluorophenyl)-5-methyl-oxazol-4-ylmethoxy]-cyclohexyl]-acetaldehyds als gelbbraunes Öl. C20H25NO4 (343.42), MS(ESI): 344 (M+H+), Rf(n-Heptan:Ethylacetat = 1:1) = 0.25.

### 2-Ethyl-4-{(1R,3S)-3-[2-(4-fluoro-phenyl)-5-methyl-oxazol-4-ylmethoxy]-cyclohexyloxy}-but-2-ensäureethylester

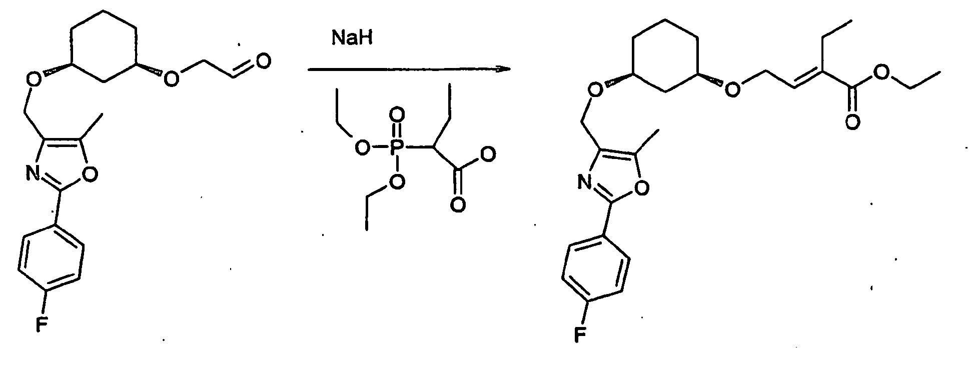

0,58 g des 2-(Diethoxy-phosphoryl)-butansäureethylesters werden in Tetrahydrofuran (20 ml) gelöst und bei 0 °C mit 0,06 g Natriumhydrid versetzt. Die Suspension wird 30 min. bei 0 °C und 30 min bei Raumtemperatur gerührt und anschließend auf - 70 °C gekühlt. Nach Zugabe von 0,4 g 2-((1R;3S)-[3-(5-Methyl-2-m-tolyl-oxazol-4-ylmethoxy)-cyclohexyloxy]-acetaldehyd (gelöst in 5 ml Tetrahydrofuran) wird 60 min bei - 70 °C und anschließend 12 h bei Raumtemperatur gerührt. Es wird mit 10 ml Wasser versetzt, mit Ethylacetat (3 x 10 ml) extrahiert und die vereinigten organischen Phasen mit gesättigter Natriumchlorid-Lösung (10 ml) gewaschen. Das Lösungsmittel wird im Vakuum entfernt und der Rückstand durch HPLC gereinigt. Man erhält 0,32 g des 2-Ethyl-4-{(1R,3S)-3-[2-(4-fluoro-phenyl)-5-methyl-oxazol-4-ylmethoxy]-cyclohexyloxy}-but-2-ensäureethylesters C25H32FNO5 (445,54) MS(ESI): 446 (M + H+)

### 2-Ethyl-4-{(1R,3S)-3-[2-(4-fluoro-phenyl)-5-methyl-oxazol-4-ylmethoxy]-cyclohexyloxy}-but-2-ensäure

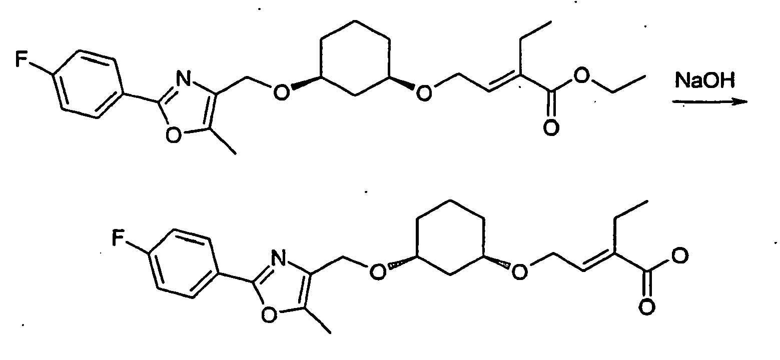

0,5 g des 2-Ethyl-4-{(1R,3S)-3-[2-(4-fluoro-phenyl)-5-methyl-oxazol-4-ylmethoxy]-cyclohexyloxy}-but-2-ensäureethylesters werden in 5 ml Methanol gelöst und mit 2.5 ml 1N Natronlauge versetzt. Nach 12 h Rühren bei Raumtemperatur wird mit 3 ml 1N Salzsäure angesäuert und der entstandene Niederschlag in Ethylacetat aufgenommen. Das Lösungsmittel wird im Vakuum entfernt und der Rückstand der Esterverseifung 2-Ethyl-4-{(1R,3S)-3-[2-(4-fluoro-phenyl)-5-methyl-oxazol-4-ylmethoxy]-cyclohexyloxy}-but-2-ensäure als 0,45 g weißer Feststoff erhalten. C₂₃H₂₈FNO₅ (417,48) MS(ESI): 418 (M + H⁺)

### 2-Ethyl-4-{(1R,3S)-3-[2-(4-fluoro-phenyl)-5-methyl-oxazol-4-ylmethoxy]-cyclohexyloxy}-butansäure

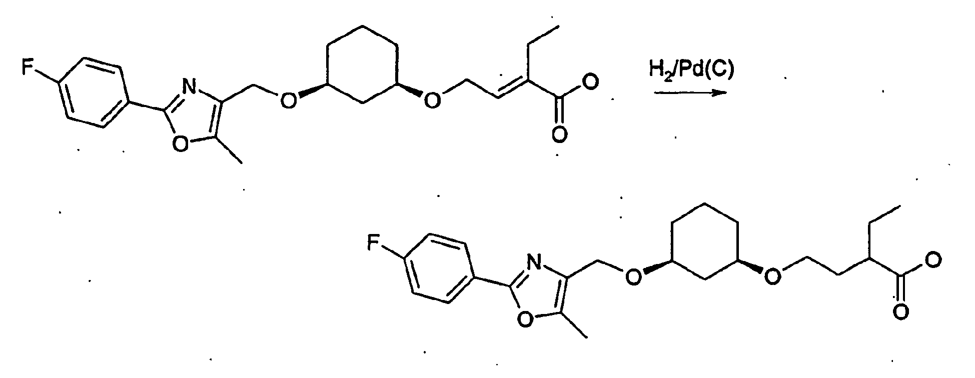

0,3 g der 2-Ethyl-4-{(1R,3S)-3-[2-(4-fluoro-phenyl)-5-methyl-oxazol-4-ylmethoxy]-cyclohexyloxy}-but-2-ensäure wird in einem Lösungsmittelgemisch aus 2 ml Ethylacetat und 1 ml Methanol gelöst und mit 0,05 g Palladium (10% auf Kohle) versetzt. Anschließend wird 3 h bei 1 bar Wasserstoffdruck hydriert. Nach Filtration vom Palladium wird das Lösungsmittelgemisch im Vakuum entfernt und der Rückstand aus Acetonitril umkristallisiert. Man erhält 0,25 g der 2-Ethyl-4-{(1R,3S)-3-[2-(4-fluoro-phenyl)-5-methyl-oxazol-4-ylmethoxy]-cyclohexyloxy}-butansäure als weißen Feststoff. C₂₃H₃₀FNO₅ (419.49), MS(ESI): 420 (M + H⁺)

### Beispiel 2:

Angepaßt an die Reaktionsbedingungen des Beispiels 1 erhält man aus 2-((1R,3S)-[3-(2-(4-Fluoro-phenyl)-5-methyl-oxazol-4-ylmethoxy)-cyclohexyloxy]-acetaldehyd und 2-(Diethoxy-phosphoryl)-pentansäureethylester 2-Propyl-4-[3-(2-(4-Fluoro-phenyl)-5-methyl-oxazol-4-ylmethoxy)-cyclohexyloxy]-butansäure. 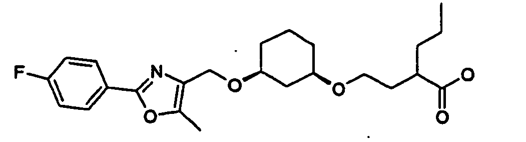 C₂₄H₃₂FNO₅ (433,52) MS(ESI): 434 (M+H⁺)

### Beispiel 3:

Angepaßt an die Reaktionsbedingungen des Beispiels 1 erhält man aus 2-((1R,3S)-[3-(2-(4-Fluoro-phenyl)-5-methyl-oxazol-4-ylmethoxy)-cyclohexyloxy]-acetaldehyd und 2-(Diethoxyphosphoryl)-essigsäureethylester 4-{3-[2-(4-Fluoro-phenyl)-5-methyl-oxazol-4-ylmethoxy]-cyclohexyloxy}-butansäure 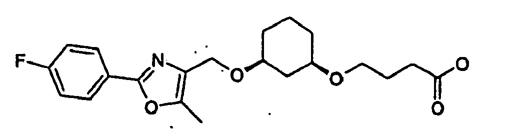 C₂₁H₂₆FNO₅ (391,44) MS(ESI): 392 (M + H⁺)

### Beispiel 4:

Angepaßt an die Reaktionsbedingungen des Beispiels 1 erhält man aus 2-((1R,3S)-[3-(2-(4-Fluoro-phenyl)-5-methyl-oxazol-4-ylmethoxy)-cydohexyloxy]-acetaldehyd und 2-(Diethoxyphosphoryl)-propionsäureethylester 4-{3-[2-(4-Fluoro-phenyl)-5-methyl-oxazol-4-ylmethox-y]-cyclohexyloxy}-2-methyl-butansäure 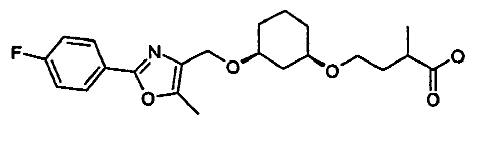 C22H28FNO5 (405,47) MS(ESI): 406 (M + H+)

### Beispiel 5:

### ((1R,3S)-2-[3-(5-Methyl-2-m-tolyl-oxazol-4-ylmethoxy)-cyclohexyloxymethyl]-acrylsäureethylester

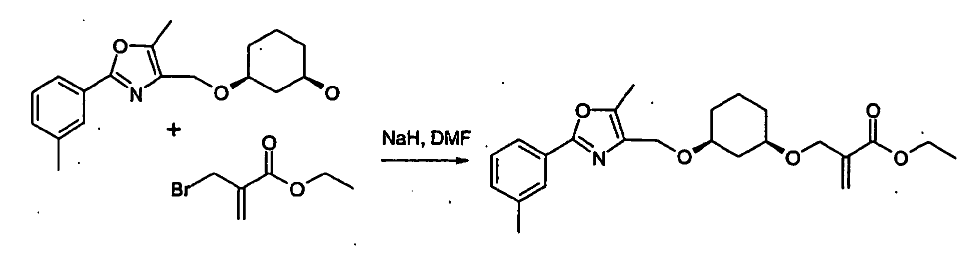

Zu einer Lösung von 754 mg 3-((1R,3S)-cis-5-Methyl-2-m-tolyl-oxazol-4-ylmethoxy)-cyclohexanol in 10 ml Dimethylformamid/5 ml Tetrahydrofuran werden bei Raumtemp. 200 mg 60-proz. Natriumhydrid-Suspension gegeben und 20 min bei Raumtemperatur gerührt. Anschließend werden bei 0°C 1 g 2-Bromomethyl-acrylsäureethylester addiert und rührt 2 h bei dieser Temperatur. Es werden 100 ml Ethylacetat und 150 ml ges. NaCl-Lösung zugegeben. Die organische Phase wird über Natriumsulfat getrocknet, das Lösungsmittel im Vakuum entfernt und der Rückstand durch Flash-Chromatographie an Kieselgel (n-Heptan/Ethylacetat = 2:1) gereinigt. Man erhält 1,18 g ((1R,3S)-2-[3-(5-Methyl-2-m-tolyl-oxazol-4-ylmethoxy)-cyclohexyloxymethyl]-acrylsäureethylester als farbloses Öl. C₂₄H₃₁NO₅ (413.52), MS (ESI): 414 (M + H⁺).

### ((1R,3S)-1-[3-(5-Methyl-2-m-tolyl-oxazol-4-ylmethoxy)-cyclohexyloxymethyl]-cyclopropancarbonsäureethylester

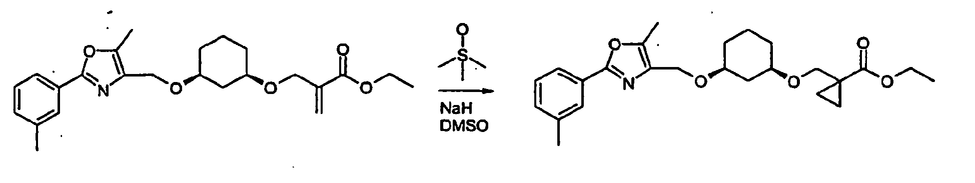

Zu einer Suspension von 55 mg Trimethylsulfoniumiodid in 2 ml DMSO werden bei Raumtemp. 12 mg 60-proz. Natriumhydrid-Suspension gegeben und 20 min bei Raumtemperatur gerührt. Anschließend werden bei 10°C 100 mg ((1R,3S)-2-[3-(5-Methyl-2-m-tolyl-oxazol-4-ylmethoxy)-cyclohexyloxymethyl]-acrylsäureethylester gelöst in 2 ml DMSO addiert und 90 min bei Raumtemp. gerührt. Man gießt auf Eiswasser und extrahiert mit Methyl-tert.-butylether. Die organische Phase wird über Natriumsulfat getrocknet, das Lösungsmittel im Vakuum entfernt und der Rückstand durch Flash-Chromatographie an Kieselgel (n-Heptan/Ethylacetat = 2:1) gereinigt. Man erhält ((1R,3S)-1-[3-(5-Methyl-2-m-tolyl-oxazol-4-ylmethoxy)-cyclohexyloxy-methyl]-cyclopropancarbonsäureethylester als farbloses Öl. C₂₅H₃₃NO₅ (427.55), MS (ESI): 428 (M + H⁺).

### ((1R,3S)-1-[3-(5-Methyl-2-m-tolyl-oxazol-4-ylmethoxy)-cyclohexyloxymethyl]-cyclopropancarbonsäure

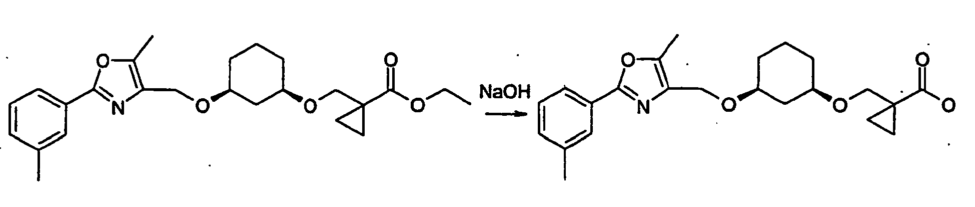

56 mg ((1R,3S)-1-[3-(5-Methyl-2-m-tolyl-oxazol-4-ylmethoxy)-cyclohexyloxymethyl]-cyclopropancarbonsäureethylester werden in 3 ml Methanol gelöst und mit 0,5 ml 5 N NaOH versetzt und 18 h bei Raumtemp. gerührt. Man entfernt das Lösungsmittel im Vakuum, säuert mit Trifluoressigsäure an und reinigt den Rückstand durch RP-HPLC. Man erhält ((1R,35)-1-[3-(5-Methyl-2-m-tolyl-oxazol-4-ylmethoxy)-cyclohexyl-oxymethyl]-cyclopropancarbonsäure als farbloses Öl. C₂₃H₂₉NO₅ (399.49), MS (ESI): 400 (M + H⁺).

### Beispiel 6:

### 2R/S-((1R',3S')-2-[(Methyl-phenethyl-amino)-methyl]-3-[3-(5-methyl-2-m-tolyl-oxazol-4-ylmethoxy)-cyclohexyloxy]-propionsäureethylester

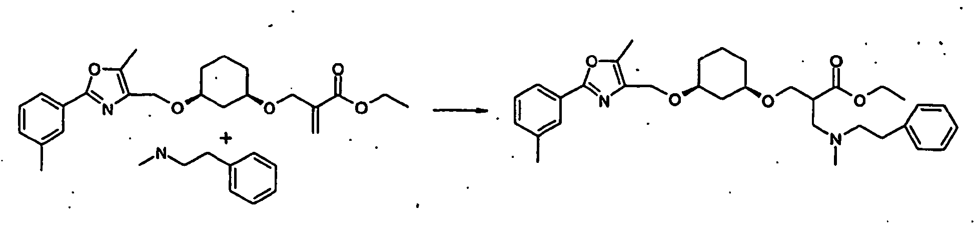

50 mg ((1R,3S)-2-[3-(5-Methyl-2-m-tolyl-oxazol-4-ylmethoxy)-cyc.lohexyloxymethyl]-acrylsäureethylester werden in 5 ml Ethanol gelöst, mit 95 mg N-Methylhomobenzylamin versetzt und 18 h bei Raumtemp. gerührt. Man entfernt das Lösungsmittel im Vakuum und reinigt den Rückstand durch Flash-Chromatographie an Kieselgel (n-Heptan/Ethylacetat = 1:1 + 3% NEt₃) gereinigt. Man erhält 2R/S-((1R',3S')2-[(Methyl-phenethyl-amino)-methyl]-3-[3-(5-methyl-2-m-tolyl-oxazol-4-yl-methoxy)-cyclohexyloxy]-propionsäureethylester als farbloses Öl. C₃₃H₄₄N₂O₅ (548.73), MS (ESI): 549 (M + H⁺).

### ((1R',3S')-2 R/S-[(Methyl-phenethyl-amino)-methyl]-3-[3-(5-methyl-2-m-tolyl-oxazol-4-ylmethoxy)-cyclohexyloxy]-propionsäure

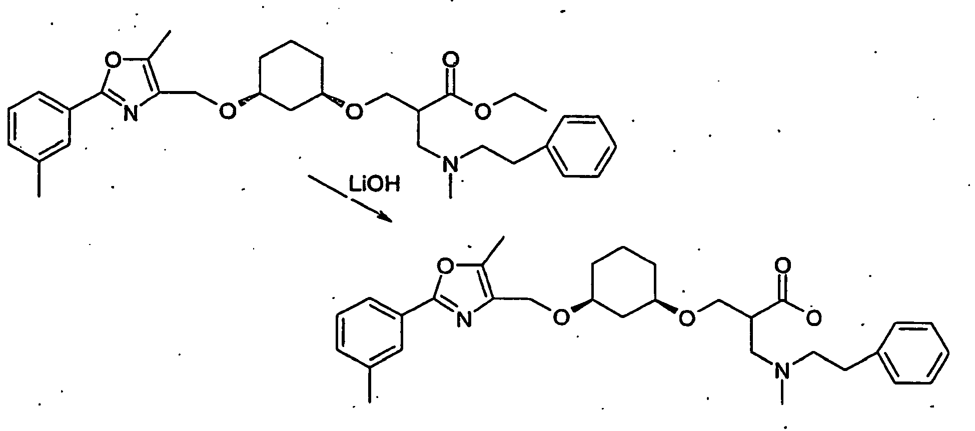

65 mg 2R/S-((1R',3S')-2-[(Methyl-phenethyl-amino)-methyl]-3-[3-(5-methyl-2-m-tolyl-oxazol-4-yl-methoxy)-cyclohexyloxy]-propionsäureethylester werden in 3 ml Tetrahydrofuran/Methanol 3:1 gelöst und mit 0,6 ml 1 N LiOH versetzt und 6 h bei Raumtemp gerührt. Man entfernt das Lösungsmittel im Vakuum säuert mit Trifluoressigsäure an und reinigt den Rückstand durch RP-HPLC. Man erhält 2R/S-((1R',3S')-[(Methyl-phenethyl-amino)-methyl]-3-[3-(5-methyl-2-m-tolyl-oxazol-4-ylmethoxy)-cyclohexyl-oxy]-propionsäure als farbloses Öl. C₃₁H₄₀N₂O₅ (520.67), MS (ESI): 521 (M + H⁺).

### Beispiel 7:

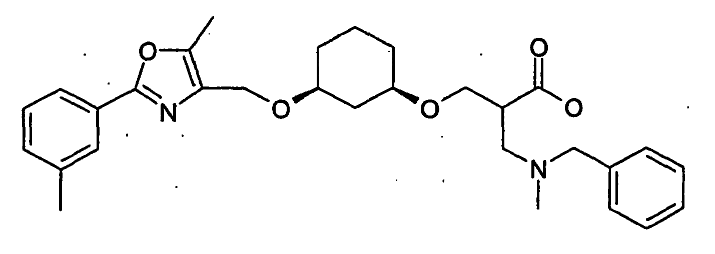

### (1R'.3S')-2R/S-[(Benzyl-methyl-amino)-methyl]-3-[3-(5-methyl-2-m-tolyl-oxazol-4-ylmethoxy)-cyclohexyloxy]-propionsäure

Aus ((1R,3S)-2-[3-(5-Methyl-2-m-tolyl-oxazol-4-ylmethony)-cyclohexyloxymethyl]-acrylsäureethylester und N-Methylbenzylamin erhält man angepaßt an die Reaktionsbedingungen des Beispiels 100 (1R',3S')-2R/S-[(Benzyl-methyl-amino)-methyl]-3-[3-(5-methyl-2-m-tolyl-oxazol-4-ylmethoxy)-cyclohexyloxy]-propionsäure mit dem Molekulargewicht 506.65 (C₃₀H₃₈N₂O₅), MS(ESI): 507.20 (M + H⁺).

### Beispiel 8:

### ((1R',3S')-2R/S-Methoxymethyl-3-[3-(5-methyl-2-m-tolyl-oxazol-4-ylmethoxy)-cyclohexyloxy]-propionsäure

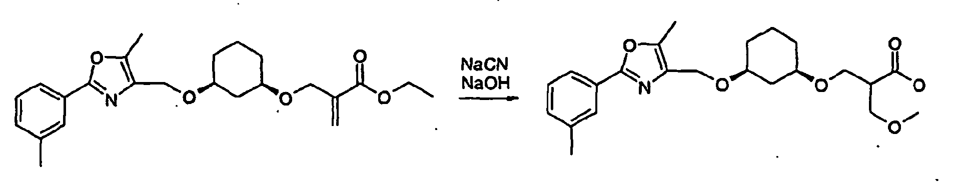

56 mg ((1R,3S)-2-[3-(5-Methyl-2-m-tolyl-oxazol-4-ylmethoxy)-cyclohexyloxymethyl]-acrylsäureethylester werden in 3 ml Methanol gelöst und mit 49 mg Natriumcyanid sowie 0,25 ml 2N NaOH versetzt und 18 h bei Raumtemp. gerührt. Man entfernt das Lösungsmittel im Vakuum, säuert mit Trifluoressigsäure an und reinigt den Rückstand durch RP-HPLC. Man erhält ((1R',3S')-2R/S-Methoxymethyl-3-[3-(5-methyl-2-m-tolyl-oxazol-4-ylmethoxy)-cyclohexyloxy]-propionsäure als farbloses Öl. C₂₃H₃₁NO₆ (417.51), MS (ESI): 418.15 (M + H⁺).

### Beispiel 9:

### Z-((1R',3S')-3-(4-Fluoro-3-methyl-phenyl)-2-[3-(5-methyl-2-m-tolyl-oxazol-4-ylmethoxy)-cyclohexyloxymethyl]-acrylsäureethylester

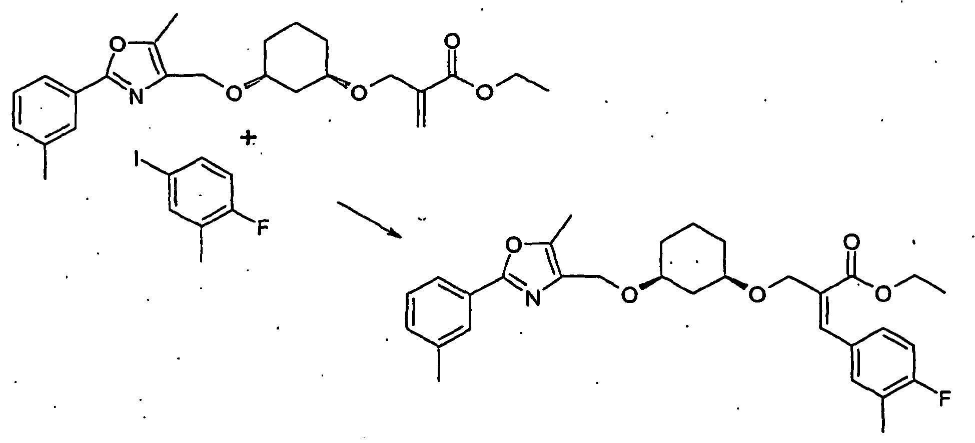

220 mg Tetrabutylammoniumchlorid und 332 mg Katiumcarbonat werden in 4 ml Dimethylformamid suspendiert und 20 min innig gerührt. Man gibt 400 mg des ((1R,3S)-2-[3-(5-Methyl-2-m-tolyl-oxazol-4-ylmethoxy)-cyclohexyloxymethyl]-acrylsäureethylester, 25 mg Triphenylphosphan und 212 mg 4-Fluor-3methyliodbenzol zu, entgast, belüftet mit Argon und addiert 10 mg Palladiumacetat und 0,2 mol Wasser. Man erhitzt 4 h auf 60°C. Nach dem Erkalten werden 20 ml Ethylacetat und 50 ml ges. NaCI-Lösung zugegeben. Die organische Phase wird über Natriumsulfat getrocknet, das Lösungsmittel im Vakuum entfernt und der Rückstand durch Flash-Chromatographie an Kieselgel (n-Heptan/Ethylacetat = 4:1) gereinigt. Man erhält Z-((1R',3S`)-3-(4-Fluoro-3-methyl-phenyl)-2-[3-(5-methyl-2-m-tolyl-oxazol-4-ylmethoxy)-cyclohexyloxy-methyl]-acrylsäureethylester als farbloses Öl. C₃₁H₃₈FNO₅ (523.65), MS (ESI): 524 (M + H⁺).

### 2R/S-(4-Fluoro-3-methyl-benzyl)-(1R',3S')-3-[3-(5-methyl-2-m-tolyl-oxazol-4-ylmethoxy)-cyclohexyloxy]-propionsäureethylester

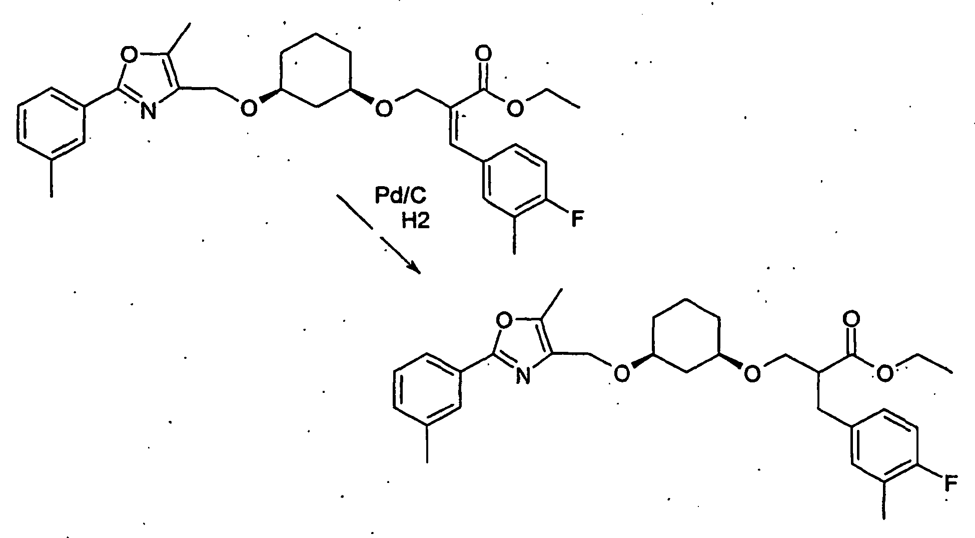

80 mg Z-((1R',3S')-3-(4-Fluoro-3-methyl-phenyl)-2-[3-(5-methyl-2-m-tolyl-oxazol-4-ylmethoxy)-cyclohexyloxymethyl]-acrylsäureethylester werden in 15 ml Ethylacetat gelöst und nach Zugabe von 30 mg Pd/C 10% unter 1 bar H₂ 24 h gerührt. Man filtriert vom Katalysator und evaporiert das Lösungsmittel. Man erhält 2R/S-(4-Fluoro-3-methyl-benzyl)-(1R',3S')-3-[3-(5-methyl-2-m-tolyl-oxazol-4-ylmethoxy)-cyclohexyl-oxy]-propionsäureethylester als farbloses Öl. C₃₁H₃₈FNO₅ (521.63), MS (ESI): 522 (M + H⁺).

### (2R/S)-(4-Fluoro-3-methyl-benzyl)-(1R',3S')-3-[3-(5-methyl-2-m-tolyl-oxazol-4-ylmethoxy)-cyclohexyloxy]-propionsäure

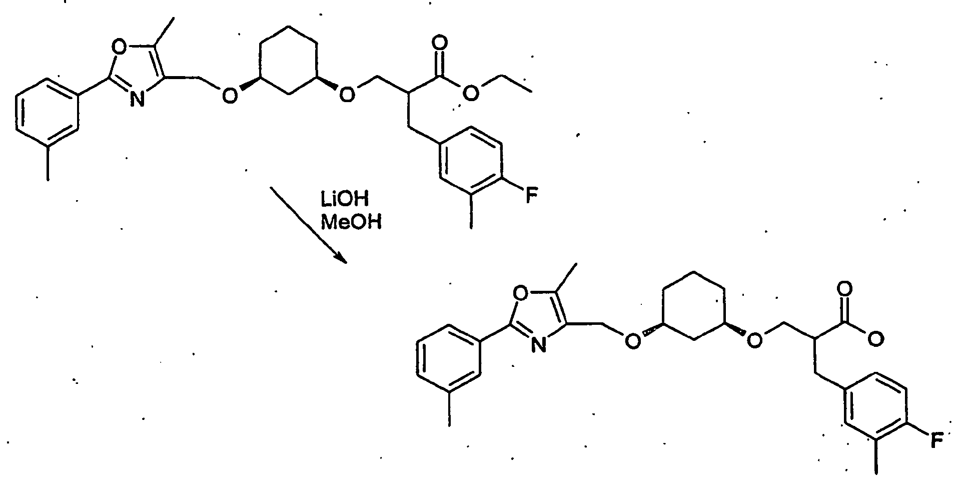

70 mg 2R/S-(4-Fluoro-3-methyl-benzyl)-(1R',3S')-3-[3-(5-methyl-2-m-tolyl-oxazol-4-ylmethoxy)-cyclohexyloxy]-propionsäureethylester werden in 3 ml Tetrahydrofuran/Methanol 3:1 gelöst und mit 0,1 ml 1 N LiOH versetzt und 18 h bei Raumtemp gerührt. Man entfernt das Lösungsmittel im Vakuum säuert mit Trifluoressigsäure an und reinigt den Rückstand durch RP-HPLC. Man erhält 2R/S-(4-Fluoro-3-methyl-benzyl)-(1R',3S')-3-[3-(5-methyl-2-m-tolyl-oxazol-4-ylmethoxy)-cyclohexyloxy]-propionsäure als farbloses Öl. C₂₉H₃₄FNO₅ (495.60), MS (ESI): 496.20 (M + H⁺).

### Beispiel 10:

### rac-3-(cis-5-Methyl-2-m-tolyl-oxazol-4-ylmethoxy)-cyclohexanol

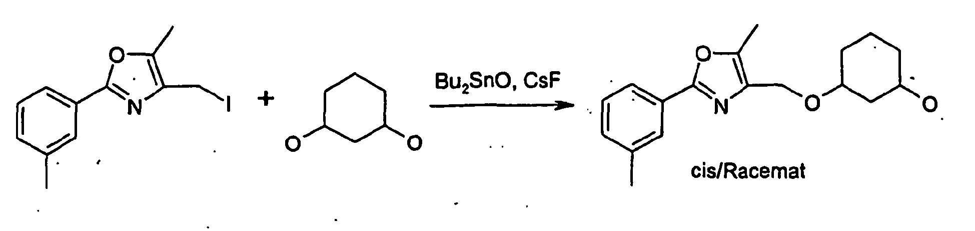

21.7 g 1,3-Cyclohexandiol werden mit 30.3 g Dibutylzinnoxid in 450 ml Toluol gelöst und unter Rückfluss am Wasserabscheider zum Sieden erhitzt. Das Reaktionsvolumen wird während der Reaktionsdauer auf die Hälfte reduziert. Nach 3 Std. wird das Reaktionsgemisch auf Raumtemp. gekühlt und mit 300 ml Dimethylformamid, 29 g 4-iodmethyl-5-methyl-2-m-tolyl-oxazol 1 und 23.5 g Cäsiumfluorid versetzt. Man rührt 18 Std. bei Raumtemp. nach. Das Reaktionsgemisch wird durch Zugabe von Ethylacetat verdünnt und mit gesättigter NaCI-Lösung gewaschen. Die organische Phase wird über Magnesiumsulfat getrocknet, das Lösungsmittel im Vakuum entfernt und der Rückstand durch Flash-Chromatographie an Kieselgel (n-Heptan/Ethylacetat = 10:1 -> 1:4) gereinigt. Man erhält 58 g rac-3-(cis-5-Methyl-2-m-tolyl-oxazol-4-ylmethoxy)-cyclohexanol als gelblichen Feststoff, der aus n-HeptanlEthylacetat umkristallisiert wird. C18H23NO3 (301.39), MS (ESI): 302 (M + H+).

### 3-((1R,3S)-cis-5-Methyl-2-m-tolyl-oxazol-4-ylmethoxy)-cyclohexanol 4

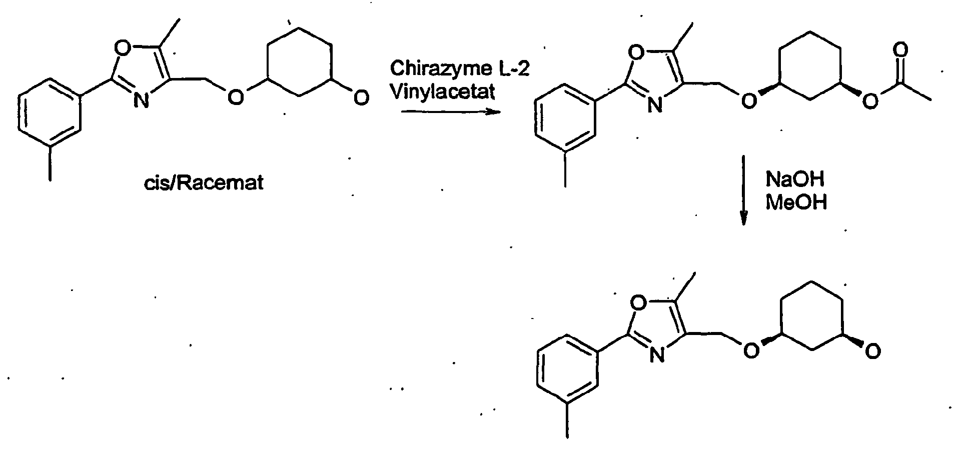

25 g rac-3-(cis-5-Methyl-2-m-tolyl-oxazol-4-ylmethoxy)-cyclohexanol werden in 320 ml Vinylacetat gelöst und mit 1,3 g Chirazyme L-2 Lyo (Boehringer Mannheim) versetzt. Nach ca dreistündigem Rühren bei Raumtemp. (LC-MS Kontrolle auf 40-45% Umsatz) wird das Enzym abfiltriert, mit Ethylacetat nachgewaschen und das Lösungsmittel im Vakuum entfernt. Der Rückstand wird durch Flash-Chromatographie an Kieselgel (n-Heptan/Ethylacetat = 3:1) gereinigt. Man erhält 8 g des Acetats 3 als farbloses Öl. C20H25NO4 (343.43), MS (ESI): 344 (M + H+), Man nimmt das Acetat in 170 ml Methanol auf und rührt nach Zugabe von 27 ml 2N NaOH für eine Stunde bei Raumtemp.. Der größte Teil des Lösungsmittels wird im Vakuum entfernt. Nach Zugabe von je 150 ml Wasser und Ethylacetat, wird die org. Phase mit Nach-Lösung gewaschen. Die organische Phase wird über Magnesiumsulfat getrocknet, das Lösungsmittel im Vakuum entfernt. Man erhält 6,7 g 3-((1R,3S)-cis-5-Methyl-2-m-tolyl-oxazol-4-ylmethoxy)-cyclohexanol als gelblichen Feststoff. C18H23NO3 (301.39), MS (ESI): 302 (M + H+).

### 4-((1R,3S)-3-Allyloxy-cyclohexyloxymethyl)-5-methyl-2-m-tolyl-oxazol 5

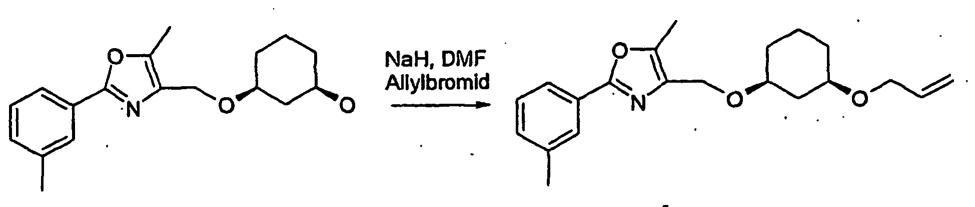

Zu einer Lösung von 2.2 g 3-((1R,3S)-cis-5-Methyl-2-m-tolyl-oxazol-4-ylmethoxy)-cyclohexanol in 30 ml Dimethylformamid werden bei Raumtemp. 470 mg 60-proz. Natriumhydrid-Suspension gegeben und 20 min bei Raumtemperatur gerührt. Anschließend werden 1,36 ml Allylbromid addiert man rührt bei 40 °C bis zum vollständigen Umsatz, eventuell werden weiteres Natriumhydrid und Allybromid zugegeben. Bei vollständigen Umsatz (LC-MS Kontrolle) werden 100 ml Ethylacetat und 150 ml ges NaCI-Lösung zugegeben. Die organische Phase wird über Magnesiumsulfat getrocknet, die Lösungsmittel im Vakuum entfernt und der Rückstand durch Flash-Chromatographie an Kieselgel (n-Heptan/Ethylacetat = 3:1) gereinigt. Man erhält 2.3 g 4-((1R,3S)-3-Allyloxy-cyclohexyloxymethyl)-5-methyl-2-m-tolyl-oxazol 5 als farbloses Öl. C21 H27N03 (341.45), MS (ESI): 342 (M + H+).

### (1R',3S')-3-[3-(5-Methyl-2-m-tolyl-oxazol-4-ylmethoxy)-cyclohexyloxy]-propane-1,2-diol

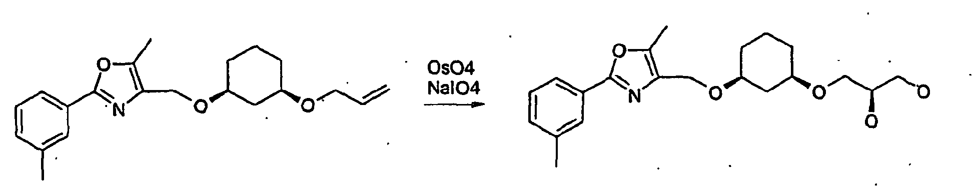

Zu 2,8 g 4-((1R,35)-3-Allyloxy-cydohexyloxymethyl)-5-methyl-2-m-tolyl-oxazol in 9 ml Aceton/Wasser 10:1 gab man bei 0°C 225 mg DABCO, 1,4 g wasserfreies N-Methylmorpholin-N-oxid und 350 µl Osmiumtetroxid 2,5% in tert Butanol. Nach 24 h Rühren bei Raumtemp. gab man 2,4 g Natriummetabisulfrt zu und verdünnte nach 10 min mit 25 ml CH2Cl2. Man filtrierte und entfernte das Lösungsmittel im Vakuum. Der Rückstand wird durch Flash-Chromatographie an Kieselgel (n-Heptan/Ethylacetat = 1:3) gereinigt. Man erhält 2.5 g 2R-(1R',3S')-3-[3-(5-Mathyl-2-m-tolyl-oxazol-4-ylmethoxy)-cyclohexyloxy]-propan-1,2-diol als farbloses Öl. C₂₁H₂₉NO₅ (375.47), MS (ESI): 376 (M + H⁺).

### 2S-(1S',3R')-1-(tert-Butyl-dimethyl-silanyloxy)-3-[3-(5-methyl-2-m-tolyl-oxazol-4-ylmethoxy)-cyclohexyloxy]-propan-2-ol

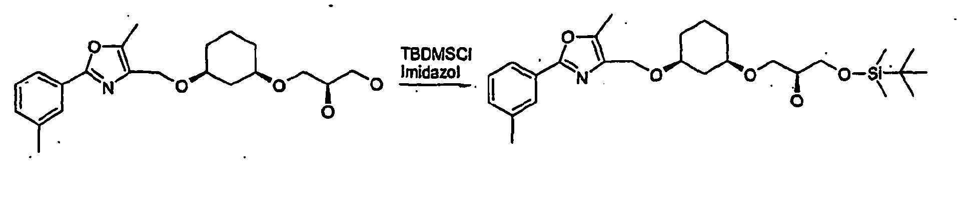

Zu 2,5 g 2R-(1R',3S')-3-[3-(5-Methyl-2-m-tolyl-oxazol-4-ylmethoxy)-cyclohexyloxy]-propane-1,2-diol in 30 ml Dichlormethan gibt man bei 0°C 500 mg Imidazol, 1,02 g tert.Butyldimethylsilylchlorid und 50 mg Tetrabutylammoniumiodid. Man läßt über 18 h auf Raumtemp. kommen und gießt die Mischung auf Eis. Man extrahiert mit Dichlormethan, trocknet über Natriumsulfat, filtriert und entfernt das Lösungsmittel im Vakuum. Der Rückstand wird durch Flash-Chromatographie an Kieselgel (n-Heptan/Ethylacetat = 2:1 → 1:2) gereinigt. Man erhält 2S-(1S',3R')-1-(tert-Butyl-dimethyl-silanyloxy)-3-[3-(5-methyl-2-m-tolyl-oxazol-4-ylmethoxy)-cyclohexyloxy]-propan-2-ol als farbloses Öl. C₂₇H₄₃NO₅Si (489.73), MS (ESI): 490 (M + H⁺).

### 2S'-(1S,3R)4-{3-[3-(tert-Butyl-dimethyl-silanyloxy)-2-prop-2-ynyloxy-propoxy]-cyclohexyloxymethyl}-5-methyl-2-m-tolyl-oxazol

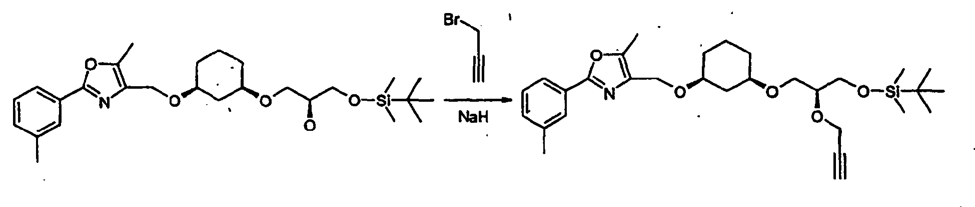

Zu 245 mg 2S-(1S',3R')-1-(tert-Butyl-dimethyl-silanyloxy)-3-[3-(5-methyl-2-m-totyl-oxazol-4-ylmethoxy)-cyclohexyloxy]-propan-2-ol in 3 ml Dimethylformamid gibt man 25 mg 60-proz. Natriumhydrid-Suspension und rührt 20 min bei Raumtemperatur. Anschließend werden 200 mg Propargylbromid addiert und man rührt bei Raumtemp bis zum vollständigen Umsatz. Man nimmt in ges NaCl-Lösung/Ethylacetat auf, trocknet die organische Phase über Natriumsulfat, filtriert und entfernt das Lösungsmittel im Vakuum. Man erhält 25'-(1S,3R)4-{3-[3-(tert-Butyl-dimethyl-silanyloxy)-2-prop-2-ynyloxy-propoxy]-cyclohexyloxymethyl}-5-methyl-2-m-tolyl-oxazol als farbloses Öl. C₃₀H₄₅NO₅Si (527.78), MS (ESI): 528 (M + H⁺).

### (1R',3S')-2R-3-[3-(5-Methyl-2-m-tolyl-oxazol-4-ylmethoxy)-cyclohexyloxy]-2-prop-2-ynyloxy-propan-1-ol

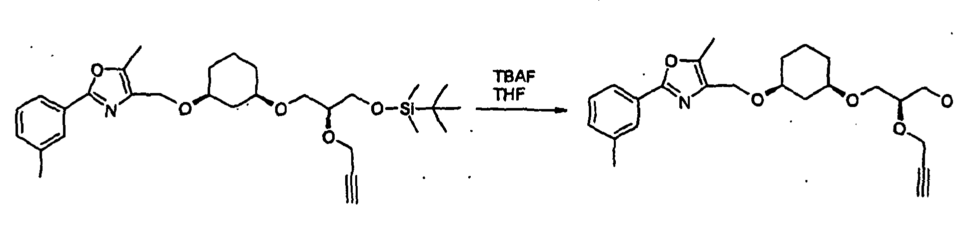

Zu 200 mg 2S'-(1S,3R)4-{3-[3-(tert-Butyl-dimethyl-silanyloxy)-2-prop-2-ynyloxy-propoxy]-cyclohexyloxymethyl}-5-methyl-2-m-tolyl-oxazol in 2 ml Tetrahydrofuran gibt man 2 ml Tetrabutylammoniumfluorid 1 M in Tetrahydrofuran und rührt 2 h bei Raumtemp. Man nimmt in ges NaCI-Lösung/Ethylacetat auf und trocknet die organische Phase über Natriumsulfat, filtriert, entfernt das Lösungsmittel im Vakuum. Man erhält (1R',3S')-2R-3-[3-(5-Methyl-2-m-tolyl-oxazol-4-ylmethoxy)-cyclohexyloxy]-2-prop-2-ynyloxy-propan-1-ol als farbloses Öl. C₂₄H₃₁NO₅ (413.53), MS (ESI): 414 (M + H⁺).

### 2R-(1R',3S')-3-[3-(5-Methyl-2-m-tolyl-oxazol-4-ylmethoxy)-cyclohexyloxy]-2-propoxy-propan-1-ol

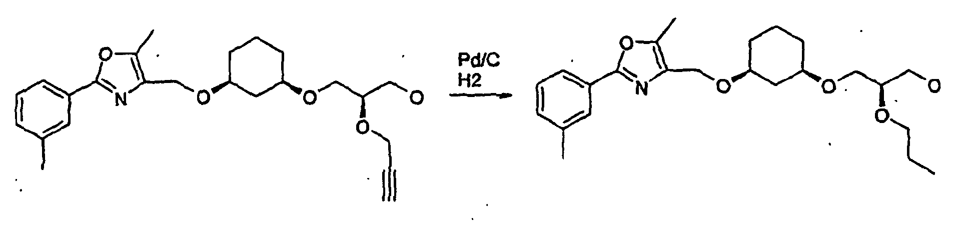

Zum Rohprodukt (1R',3S')-2R-3-[3-(5-Methyl-2-m-tolyl-oxazol-4-pmethoxy)-cyclohexyloxy]-2-prop-2-ynyloxy-propan-1-ol gelöst in 20 ml Methanol gibt man 50 mg Pd/C 10% und rührt unter 1 bar Wasserstoff für 3 h. Man filtriert vom Katalysator und entfernt das Lösungsmittel im Vakuum. Man erhält 2R-(1R',3S')-3-[3-(5-Methyl-2-m-tolyl-oxazol-4-ylmethoxy)-cyclohexyloxy]-2-propoxy-propan-1-ol als farbloses Öl. Der Rückstand wird durch Flash-Chromatographie an Kieselgel (n-HeptanlEthylacetat = 1:1) gereinigt. C₂₄H₃₅NO₅ (417.55), MS (ESI): 418 (M + H⁺).

### 2R-(1R',3S')-2-(2-propoxy)-3-[3-(5-methyl-2-m-tolyl-oxazol-4-ylmethoxy)-cyclohexyloxy]-propionsäure

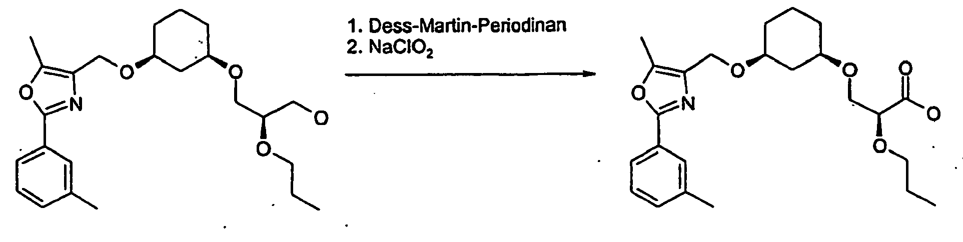

90 mg 2R-(1R',3S')-2-Propoxy-3-[3-(5-methyl-2-m-tolyl-oxazol-4-ylmethoxy)-cyclohexyloxy]-propan-1-ol werden in 1,5 ml Dichlormethan mit 180 mg Dess-Martin-Periodinan versetzt und 2 h bei Raumtemperatur gerührt. Anschließend werden 41 mg Na₂S₂O₃ in 3 ml 5 % NaHCO₃-Lösung zugesetzt und 10 min bei Raumtemperatur gerührt. Die organische Phase wird abgetrennt, über MgSO₄ getrocknet und eingeengt. Der Rückstand wird in 2 ml Acetonitril aufgenommen und mit 1,5 ml 0,65 M NaH₂PO₄-Lösung und 48 µl 35 % H₂O₂-Lösung versetzt. Bei 0°C werden 30 mg NaClO₂ in 2 ml Wasser über 1 h zugetropft. Die Mischung wird 3 h bei dieser Temperatur gerührt. Dann werden Na₂SO₃-Lösung, 10 % HCl und 10 ml CH₂Cl₂ zugegeben, die Phasen getrennt, die organische Phase über MgSO₄ getrocknet und eingeengt. Reinigung des Rückstandes per HPLC liefert 1,2 mg 2R-(1R',3S')-2-(2-methylpropoxy)-3-[3-(5-methyl-2-m-tolyl-oxazol-4-ylmethoxy)-cyclohexyloxy]-propionsäure.

### Beispiel 11:

### 2R-(1R',3S')-3-[3-(5-Methyl-2-m-tolyl-oxazol-4-ylmethoxy)-cyclohexyloxy]-2-(4-trifluoromethyl-benzyloxy)-propansäure

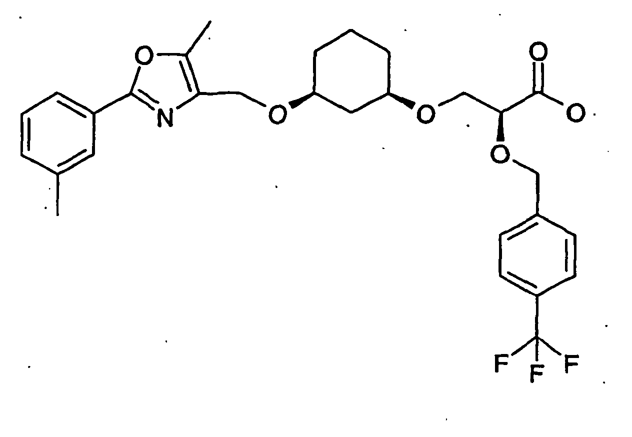

Angepaßt an die Reaktionsbedingungen des Beispiels 10 erhält man aus 2S-(1R',3S')-1-(tert-Butyl-dimethyl-silanyloxy)-3-[3-(5-methyl-2-m-tolyl-oxazol-4-ylmethoxy)-cyclohexyloxy]-propan-2-ol und 4-Trifluoromethylbenzylbromid 2R-(1R',3S')-3-[3-(5-Methyl-2-m-tolyl-oxazol-4-ylmethoxy)-cyclohexyloxy]-2-(4-trifluoromethyl-benzyloxy)-propionsäure.

### Beispiel 12:

### 3-[(1R,3S)-3-(5-Methyl-2-m-tolyl-oxazol-4-ylmethoxy)-cyclohexyloxy]-2-methoxypropionsäure

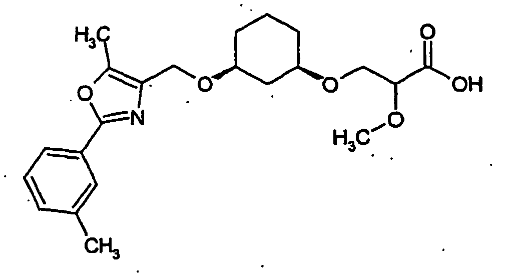

Angepaßt an die Reaktionsbedingungen des Beispiels 10 erhält man aus 1-(tert-Butyl-dimethyl-silanyloxy)-3-[(1R,3S)-3-(5-methyl-2-m-tolyl-oxazol-4-ylmethoxy)-cyclohexyloxy]-propan-2-ol und Methyliodid 3-[(1R,3S)-3-(5-Methyl-2-m-tolyl-oxazol-4-ylmethoxy)-cyclohexyloxy]-2-methoxypropionsäure.

### Beispiel 13:

### 3-[(1R,3S)-3-(5-Methyl-2-m-tolyl-oxazol-4-ylmethoxy)-cyclohexyloxy]-2-(4-trifluoromethyl-benzyloxy)-propansäure

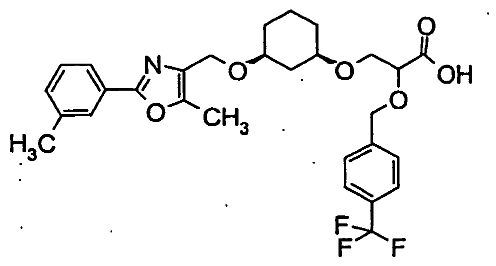

Angepaßt an die Reaktionsbedingungen des Beispiels 10 erhält man aus 1-(tert-Butyl-dimethyl-silanyloxy)-3-[(1R,3S)-3-(5-methyl-2-m-tolyl-oxazol-4-ylmethoxy)-cyclohexyloxy]-propan-2-ol und 4-Trifluoromethylbenzylbromid 3-[(1R,35)-3-(5-Methyl-2-m-tolyl-oxazol-4-ylmethoxy)-cyclohexyloxy]-2-(4-trifluoromethyl-benzyloxy)-propansäure.

### Beispiel 14:

### 3-[(1R,3S)-3-(5-Methyl-2-m-tolyl-oxazol-4-ylmethoxy)-cyclohexyloxy]-2-(3-tritluoromethyl-benzyloxy)-propansäure

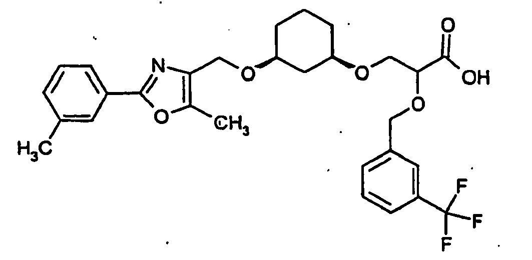

Angepaßt an die Reaktionsbedingungen des Beispiels 10 erhält man aus 1-(tert-Butyl-dimethyl-silanyloxy)-3-[(1R,35)-3-(5-methyl-2-m-tolyl-oxazol-4-ylmethoxy)-cyclohexyloxy]-propan-2-ol und und 3-Trifluöromethylbenzylbromid 3-[(1R,3S)-3-(5-Methyl-2-m-tolyl-oxazol-4-ylmethoxy)-cyclohexyloxy]-2-(3-trifluoromethyl-benzyloxy)-propansäure.

### Beispiel 15:

### 3-[(1R,3S)-3-(5-Methyl-2-m-tolyl-oxazol-4-ylmethoxy)-cyclohexyloxy]-2-(3-methoxy-benzyloxy)-propansäure

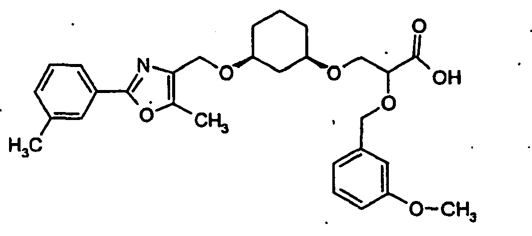

Angepaßt an die Reaktionsbedingungen des Beispiels 10 erhält man aus 1-(tert-Butyl-dimethyl-silanyloxy)-3-[(1R,3S)-3-(5-methyl-2-m-tolyl-oxazol-4-ylmethoxy)-cyclohexyloxy]-propan-2-ol und 3-Methoxybenzylbromid 3-[(1R,3S)-3-(5-Methyl-2-m-tolyl-oxazol-4-ylmethoxy)-cyclohexyloxy]-2-(3-methoxy-benzyloxy)-propansäure.

### Beispiel 16:

### 3-[(1R,3S)-3-(5-Methyl-2-m-tolyl-oxazol-4-ylmethoxy)-cyclohexyloxy]-2-(2,5-dimethylbenzyloxy)-propansäure

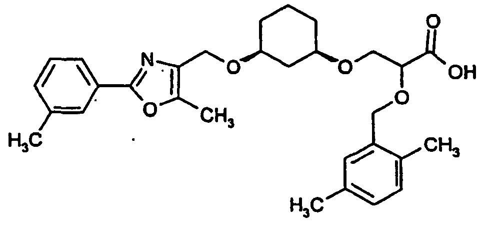

Angepaßt an die Reaktionsbedingungen des Beispiels 10 erhält man aus 1-(tert-Butyl-dimethyl-silanyloxy)-3-[(1R,3S)-3-(5-methyl-2-m-tolyl-oxazol-4-ylmethoxy)-cyclohexyloxy]-propan-2-ol und 2,5-dimethylbenzylbromid 3-[(1R,3S)-3-(5-Methyl-2-m-tolyl-oxazol-4-ylmethoxy)-cyclohexyloxy]-2-(2,5-dimethylbenzyloxy)-propansäure.

### Beispiel 17:

### 3-[(1R,3S)-3-(5-Methyl-2-m-tolyl-oxazol-4-ylmethoxy)-cyclohexyloxy]-2-(4-methylbenzyloxy)-propansäure

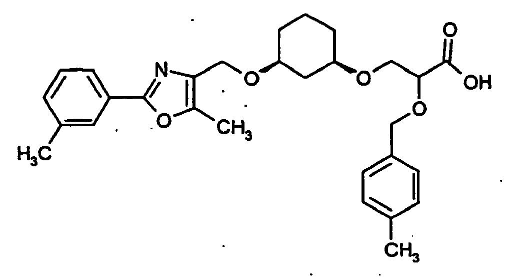

Angepaßt an die Reaktionsbedingungen des Beispiels 10 erhält man aus 1-(tert-Butyl-dimethyl-silanyloxy)-3-[(1R,3S)-3-(5-methyl-2-m-tolyl-oxazol-4-ylmethoxy)-cyclohexyloxy]-propan-2-ol und 4-Methylbenzylbromid 3-[(1R,3S)-3-(5-Methyl-2-m-tolyl-oxazol-4-ylmethoxy)-cyclohexyloxy]-2-(4-methylbenzyloxy)-propansäure.

### Beispiel 18:

### 3-[(1R,3S)-3-(5-Methyl-2-m-tolyl-oxazol-4-ylmethoxy)-cyclohexyloxy]-2-(4-tert-butylbenzyloxy)-propansäure

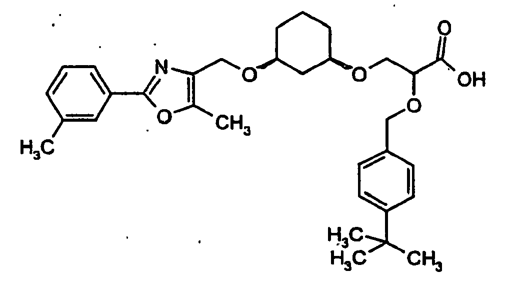

Angepaßt an die Reaktionsbedingungen des Beispiels 10 erhält man aus 1-(tert-Butyl-dimethyl-silanyloxy)-3-[(1R,3S)-3-(5-methyl-2-m-tolyl-oxazol-4-ylmethoxy)-cyclohexyloxy]-propan-2-ol und 4-tert-Butylbenzylbromid 3-[(1R,3S)-3-(5-Methyl-2-m-tolyl-oxazol-4-ylmethoxy)-cyclohexyioxy]-2-(4-tert-buty benzyloxy)-propansäure.

### Beispiel 19:

### 3-[(1R,3S)-3-(5-Methyl-2-m-tolyl-oxazol-4-ylmethoxy)-cyclohexyloxy]-2-(2-trifluoromethyl-benzyloxy)-propansäure

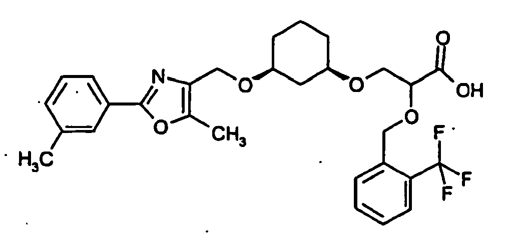

Angepaßt an die Reaktionsbedingungen des Beispiels 10 erhält man aus 1-(tert-Butyl-dimethyl-silanyloxy)-3-[(1R,3S)-3-(5-methyl-2-m-tolyl-oxazol-4-ylmethoxy)-cyclohexyloxy]-propan-2-ol und 2-Trifluoromethylbenzylbromid 3-[(1R,3S)-3-(5-Methyl-2-m-tolyl-oxazol-4-ylmethoxy)-cyclohexyloxy]-2-(2-trifluoromethyl-benzyloxy)-propansäure.

### Beispiel 20:

### 3-[(1R,3S)-3-(5-Methyl-2-m-tolyl-oxazol-4-ylmethoxy)-cyclohexyloxy]-2-(2-chlorthien-5-ylmethoxy)-propionsäure

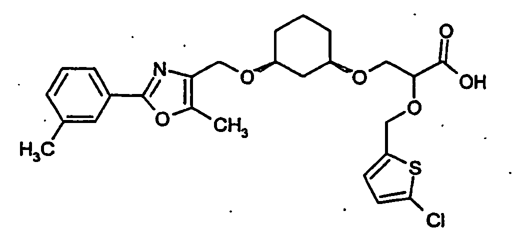

Angepaßt an die Reaktionsbedingungen des Beispiels 10 erhält man aus 1-(tert-Butyl-dimethyl-silanyloxy)-3-[(1R,3S)-3-(5-methyl-2-m-tolyl-oxazol-4-ylmethoxy)-cyclohexyloxy]-propan-2-ol und 2-chlorthien-5-ylmethylchlorid 3-[(1R,3S)-3-(5-Methyl-2-m-tolyl-oxazol-4-ylmethoxy)-cyclohexyloxy]-2-(2-chlorthien-5-ylmethoxy)-propionsäure.

### Beispiel 21:

### 3-[(1R,3S)-3-(5-Methyl-2-m-tolyl-oxazol-4-ylmethoxy)-cyclohexyloxy]-2-(2-butinoxy)-propionsäure

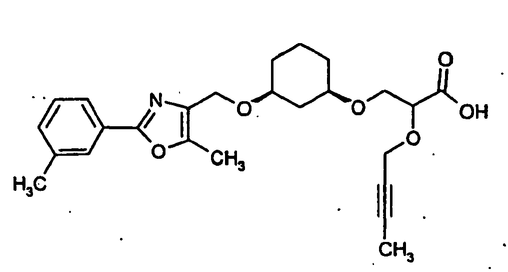

Angepaßt an die Reaktionsbedingungen des Beispiels 10 erhält man aus 1-(tert-Butyl-dimethyl-silanyloxy)-3-[(1R,3S)-3-(5-methyl-2-m-tolyl-oxazol-4-ylmethoxy)-cyclohexyloxy]-propan-2-ol und 2-Butinylbromid 3-[(1R,3S)-3-(5-Methyl-2-m-tolyl-oxazol-4-ylmethoxy)-cyclohexyloxy]-2-(2-butinoxy)-propionsäure.

### Beispiel 22:

### 3-[(1R,3S)-3-(5-Methyl-2-m-tolyl-oxazol-4-ylmethoxy)-cyclohexyloxy]-2-(2-propinoxy)-propionsäure

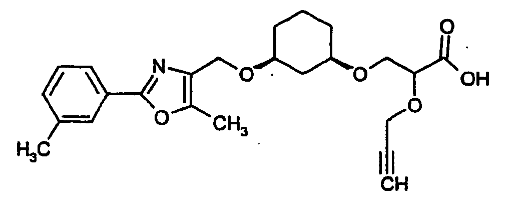

Angepaßt an die Reaktionsbedingungen des Beispiels 10 erhält man aus 1-(tert-Butyl-dimethyl-silanyloxy)-3-[(1R,3S)-3-(5-methyl-2-m-tolyl-oxazol-4-ylmethoxy)-cyclohexyloxy]-propan-2-ol und 2-Propinylbromid 3-[(1R,3S)-3-(5-Methyl-2-m-tolyl-oxazol-4-ylmethoxy)-cyclohexyloxy]-2-(2-propinoxy)-propionsäure.

### Beispiel 23:

### 3-[(1R,3S)-3-(5-Methyl-2-m-tolyl-oxazol-4-ylmethoxy)-cyclohexyloxy]-2-(2-pentinoxy)-propionsäure

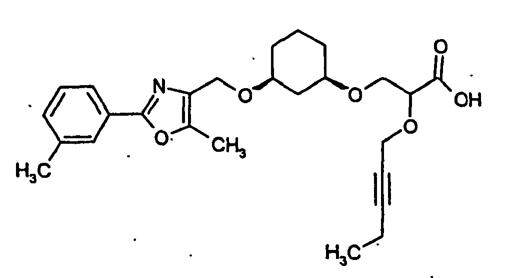

Angepaßt an die Reaktionsbedingungen des Beispiels 10 erhält man aus 1-(tert-Butyl-dimethyl-silanyloxy)-3-[(1R,3S)-3-(5-methyl-2-m-tolyl-oxazol-4-ylmethoxy)-cyclohexyloxy]-propan-2-ol und 2-Pentinylbromid 3-[(1R,3S)-3-(5-Methyl-2-m-tolyl-oxazol-4-ylmethoxy)-cyclohexyloxy]-2-(2-pentinoxy)-propionsäure.

### Beispiel 24:

### 3-[(1R,3S)-3-(5-Methyl-2-m-tolyl-oxazol-4-ylmethoxy)-cyclohexyloxy]-2-(2-propenoxy)-propionsäure

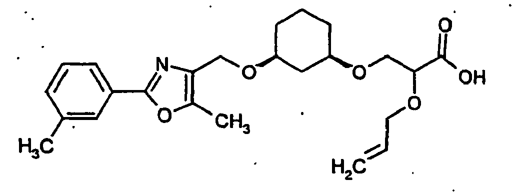

Angepaßt an die Reaktionsbedingungen des Beispiels 10 erhält man aus 1-(tert-Butyl-dimethyl-silanyloxy)-3-[(1R,3S)-3-(5-methyl-2-m-tolyl-oxazol-4-ylmethoxy)-cyclohexyloxy]-propan-2-ol und Allylbromid 3-[(1R,3S)-3-(5-Methyl-2-m-tolyl-oxazol-4-ylmethoxy)-cyclohexyloxy]-2-(2-propenoxy)-propionsäure.

### Beispiel 25:

### 3-[(1R,3S)-3-(5-Methyl-2-m-tolyl-oxazol-4-ylmethoxy)-cyclohexyloxy]-2-(3-phenyl-2-propenoxy)-propionsäure

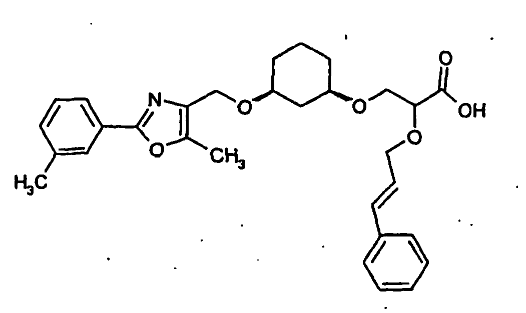

Angepaßt an die Reaktionsbedingungen des Beispiels 10 erhält man aus 1-(tert-Butyl-dimethyl-silanyloxy)-3-[(1R,3S)-3-(5-methyl-2-m-tolyl-oxazol-4-ylmethoxy)-cyclohexyloxy]-propan-2-ol und 3-Phenyl-2-propenylbromid 3-[(1R,3S)-3-(5-Methyl-2-mtolyl-oxazol-4-ylmethoxy)-cyclohexyloxy]-2-(3-phenyl-2-propenoxy)-propionsäure.

### Beispiel 26:

### 3-[(1R,3S)-3-(5-Methyl-2-m-tolyl-oxazol-4-ylmethoxy)-cyclohexyloxy]-2-(2-methyl-2-propenoxy)-propionsäure

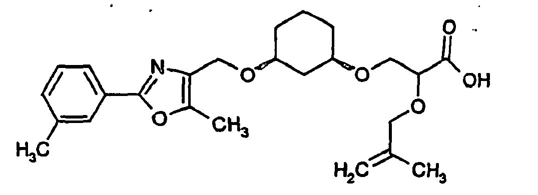

Angepaßt an die Reaktionsbedingungen des Beispiels 10 erhält man aus 1-(tert-Butyl-dimethyl-silanyloxy)-3-[(1R,3S)-3-(5-methyl-2-m-tolyl-oxazol-4-ylmethoxy)-cyclohexyloxy]-propan-2-ol und Isobutenylbromid 3-[(1R,3S)-3-(5-Methyl-2-m-tolyl-oxazol-4-ylmethoxy)-cyclohexyloxy]-2-(2-methyl-2-propenoxy)-propionsäure.

### Beispiel 27:

### 3-[(1R,3S)-3-(5-Methyl-2-m-tolyl-oxazol-4-ylmethoxy)-cyclohexyloxy]-2-benzyloxypropionsäure

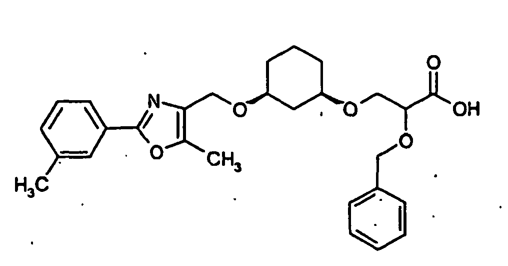

Angepaßt an die Reaktionsbedingungen des Beispiels 10 erhält man aus 1-(tert-Butyl-dimethyl-silanyloxy)-3-[(1R,3S)-3-(5-methyl-2-m-tolyl-oxazol-4-ylmethoxy)-cyclohexyloxy]-propan-2-ol und Benzylbromid 3-[(1R,3S)-3-(5-Methyl-2-m-tolyl-oxazol-4-ylmethoxy)-cyclohexyloxy]-2-benzyloxypropionsäure.

### Beispiel 28:

### Benzyl-[(1S,3R)-3-allyloxycyclohex-1-yl]-ether

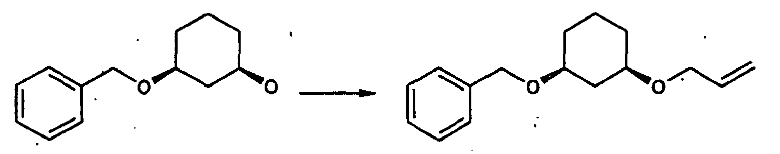

12 g Natriumhydrid (55-65%) werden unter Argonatmosphäre in 200 ml abs DMF vorgelegt und 10 min bei RT gerührt. 54 g Benzyl-[(1S,3R)-3-hydoxycyclohex-1-yl]-ether in 70 ml DMF werden zugetropft unter Eiskühlung. Die Mischung wird 90 min bei RT gerührt. 50 g Allybromid werden unter Eiskühlung langsam zugetropft. Nach vollendeter Zugabe wird 1 h bei 45 °C gerührt. Lt. DC ist die Reaktion vollständig. Die Reaktionslösung wird mit 15 ml iPrOH gequencht, mit Wasser und ges. NaCl-Lösung verdünnt und mit MTBE extrahiert. Die vereinigten organischen Phasen werden mit Wasser und NaCl-Lösung gewaschen, über MgSO4 getrocknet und eingeengt. Man erhält 60 g Benzyl-[(1S,3R)-3-allyloxycyclohex-1-yl]-ether als hellbelges Öl. C16H22O2 (246,35), MS (ESI): 247 (M + H⁺).

### Benzoesäure-[(1S,3R)-3-allyloxycyclohex-1-yl]-ester

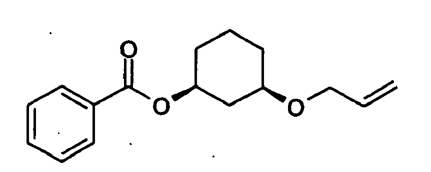

Analog zu Benzyl-[(1S,3R)-3-allyloxycyclohex-1-yl]-ether
erhält man aus Benzoesäure-[(1S,3R)-3-hydoxycyclohex-1-yl]-ester und Allylbromid Benzoesäure-[(1S,3R)-3-allyloxycyclohex-1-yl]-ester. C16H20O3 (260,37), MS (ESI): 261 (M + H⁺).

### Allyl-[(1R,3S)-3-trityloxymethylcyclohex-1-yl]-ether

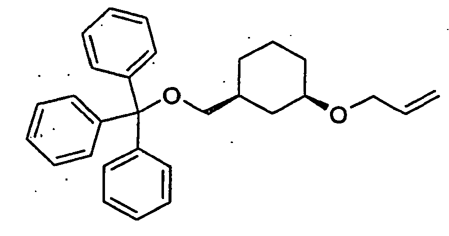

Analog zu Benzyl-[(1S,3R)-3-allyloxycyclohex-1-yl]-ether
erhält man aus (1R,3S)-3-trityloxymethylcyclohexanol und Allylbromid Allyl-[(1R,3S)-3-trityloxymethylcyclohex-1-yl]-ether.

### (2R/2S)-3-[(1S,3R)-3-Benzyloxycyclohexyloxy]-propan-1,2-diol

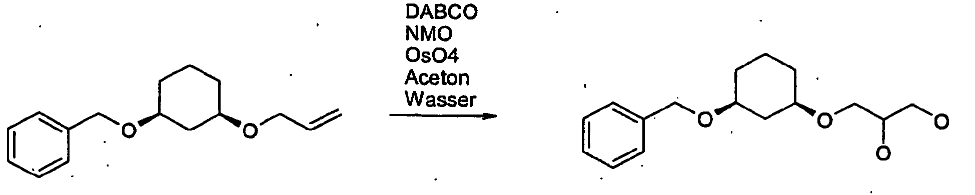

Zu 60 g Benzyl-[(1S,3R)-3-allyloxycyclohex-1-yl]-ether in 200 ml Aceton/Wasser (10:1) werden bei 0°C nacheinander 6,8 g DABCO, 42,8 g NMO und 8,0 ml Osmiumtetroxidlösung (2,5% in tert-Butanol) gegeben. Die Lösung wird über Nacht bei RT gerührt. Nach 24 h werden 5 ml Osmiumtetroxidlösung nachdosiert und die Lösung über Nacht bei RT weitergerührt. Danach ist die Reaktion vollständig. Natriumsulfit-Lösung und Wasser werden zugegeben, und das Gemisch wird mit Dichlormethan extrahiert. Die organische Phase wird über Na2SO4 getrocknet und eingeengt, wobei 65 g (2R/2S)-3-[(1S,3R)-3-benzyloxycyclohexyloxy]-opropan-1,2-diol als braunes Öl erhalten werden. C16H24O4 (280,37), MS (ESI): 281 (M + H⁺).

### (2R/2S)-3-[(1R,3S)-1-Benzoyloxycyclohex-3-yloxy]-propan-1,2-diol

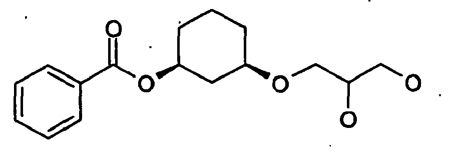

Analog zu (2R/2S)-3-[(1S,3R)-3-Benzyloxycyclohexyloxy]-propan-1,2-diol
erhält man aus Benzoesäure-[(1S,3R)-3-hydoxycyclohex-1-yl]-ester, NMO, DABCO und Osmiumtetroxid (2R/2S)-3-[(1R,3S)-1-Benzoyloxycyclohex-3-yloxy]-propan-1,2-diol. C16H22O5 (294,35), MS (ESI): 295 (M + H⁺).

### (2R/2S)-3-[(1R,3S)-3-Trityloxymethylcyclohexyloxy]-propan-1,2-diol

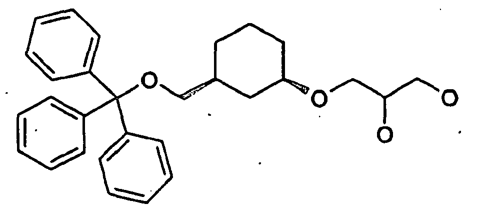

Analog zu (2R/2S)-3-[(1S,3R)-3-Benzyloxycyclohexyloxy]-propan-1,2-diol
erhält man aus Allyl-[(1R,3S)-3-trityloxymethylcyclohex-1-yl]-ether, NMO, DABCO und Osmiumtetroxid ((2R/2S)-3-[(1R,3S)-3-Trityloxymethylcyclohexyloxy]-propan-1,2-diol.

### (2R/2S)-3-[(1S,3R)-3-Benzyloxycyclohexyloxy]-2-hydroxypropyl-tert-butyldiphenylsilylether

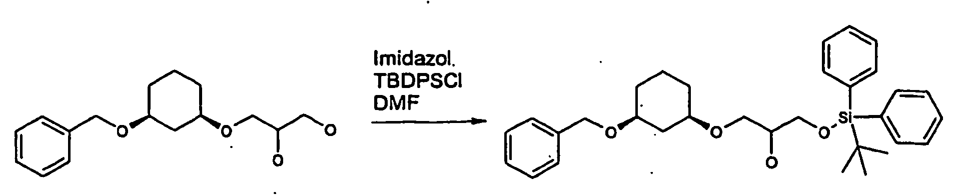

20 g (2R/2S)-3-[(1S,3R)-3-benzyloxycyclohexyloxy]-propan-1,2-diol werden in 100 ml DMF und gelöst und mit 12 g Imidazol und anschließend mit 20,6 g TBDPSCI versetzt und bei RT gerührt. Nach 3 h ist die Reaktion vollständig. Verdünnen der Lösung mit Wasser/ges. NaCl-Lösung, Extraktion mit MTBE und Trocknen der organischen Phase über MgSO4 und Einengen liefern 35 g (2R/2S)-3-[(1S,3R)-3-benzyloxycyclohexyloxy]-2-hydroxypropyl-tert-butyldiphenylsilylether als gelbes Öl. C32H42O4Si (518,78), MS (ESI): 519 (M + H⁺).

### (2R/2S)-3-[(1R,3S)-1-Benzoyloxycyclohex-1-yloxy]-2-hydroxypropyl-tert-butyldimethylsilytether

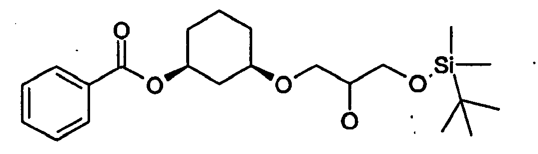

Analog zu (2R/2S)-3-[(1S,3R)-3-benzoyloxycyclohex-1-yloxy]-2-hydroxypropyl-tert-butyldiphenylsilylether erhält man aus (2R/2S)-3-[(1R,3S)-1-Benzoyloxycyclohex-3-yloxy]-propan-1,2-diol, Imidazol und TBDMSCI (2R/2S)-3-[(1S,3R)-1-Benzoyloxycyclohex-1-yloxy]-2-hydroxypropyl-tert-butyldimethylsilylether.
C22H3605Si (408.62), MS (ESI): 409 (M + H⁺).

### (2R/2S)-3-[(1R,3S)-3-Trityloxymethylcyclohexyloxy]-2-hydroxypropyl-tert-butyldiphenylsilylether

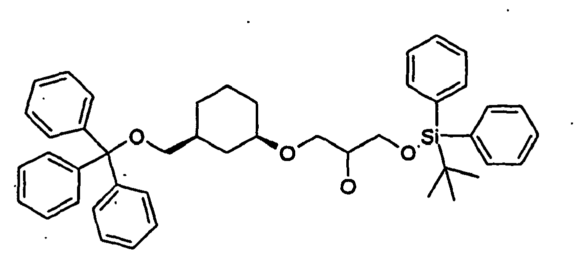

Analog zu (2R/2S)-3-[(1S,3R)-3-Benzyloxycyclohexyloxy]-2-hydroxypropyl-tert-butyldiphenylsilylether erhält man aus (2R/2S)-3-[(1R,3S)-3-Trityloxymethylcyclohexyloxy]-propan-1,2-diol, Imidazol und TBDPSCI (2R/2S)-3-[(1R,3S)-3-Trityloxymethylcyclohexyloxy]-2-hydroxypropyl-tert-butyldiphenylsilylether.

### (2R/2S)-3-[(1S,3R)-3-Benzyloxycyclohexyloxy]-2-methoxypropyl-tert-butyldiphenylsilylether

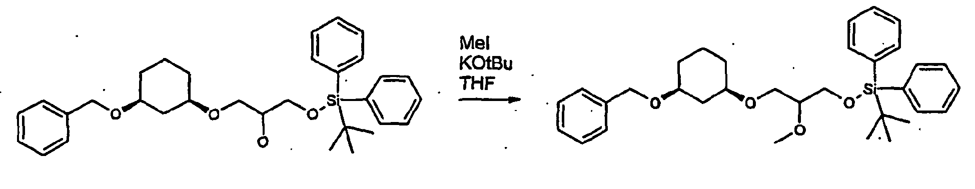

10 g (2R/2S)-3-[(1S,3R)-3-benzyloaycyclohexyloxy]-2-hydroxypropyl-tert-butyldiphenylsilylether werden mit 8,5 g lodmethan in 100 ml THF gelöst. Bei RT werden 3,3 g KOtBu zugesetzt und die Suspension 1 h bei RT gerührt. Anschließend werden ges. NH4Cl-Lösung und Wasser zugesetzt, und die Lösung wird mit MTBE extrahiert. Die vereinigten organsichen Phasen werden über MgSO4 getrocknet und eingeengt. Der Rückstand wird an Kieselgel chromatographiert (Heptan/Ethylacetat 5:1 -> 1:1), wobei 7,2 g (2R/2S)-3-[(1S,3R)-3-benzyloxycyclohexyloxy]-2-methoxypropyl-tert-butyldiphenylsilylether als hellgelbes Öl erhalten werden.

### (2R/2S)-3-[(1S,3R)-3-Benzyloxycyclohexyloxy]-2-ethoxypropyl-tert-butyldiphenylsilylether

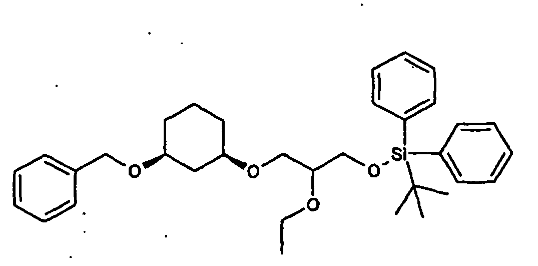

Analog zu (2R/2S)-3-[(1S,3R)-3-Benzyloxycyclohexyloxy]-2-methoxypropyl-tert-butyldiphenylsilylether erhält man aus (2R/2S)-3-[(1S,3R)-3-Benzyloxycyclohexyloxy]-2-hydroxypropyl-tert-butyldiphenylsilylether und Iodethan (2R/2S)-3-[(1S,3R)-3-Benzyloxycyclohexyloxy]-2-ethoxypropyl-tert-butyldiphenylsilylether.

### (2R/2S)-3-[(1S,3R)-3-Benzyloxycyclohexyloxy]-2-allyloxypropyl-tert-butyldiphenylsilylether

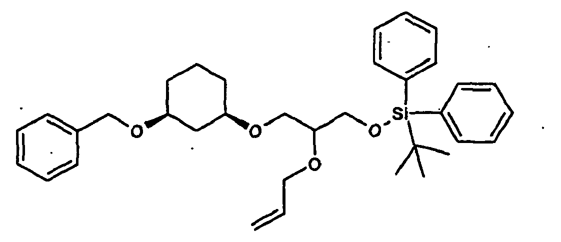

Analog zu (2R/2S)-3-[(1S,3R)-3-Benzyloxycyclohexyloxy]-2-methoxypropyl-tert-butyldiphenylsilylether erhält man aus (2R/2S)-3-[(1S,3R)-3-Benzyloxycyclohexyloxy]-2-hydroxypropyl-tert-butyldiphenylsilylether und Allylbromid (2R/2S)-3-[(1S,3R)-3-Benzyloxycyclohexyloxy]-2-allyloxypropyl-tert-butyldiphenylsilylether.

### (2R/2S)-3-[(1S,3R)-3-Benzyloxycyclohexyloxy]-2-(2-methyl-2-propenloxy)-propyl-tert-butyldiphenylsilylether

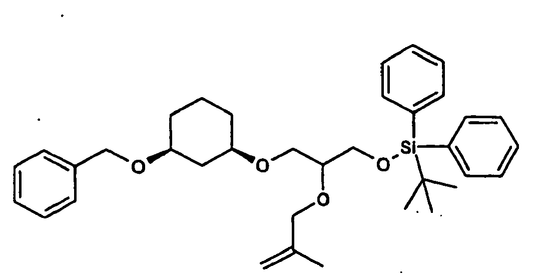

Analog zu (2R/2S)-3-[(1S,3R)-3-Benzyloxycyclohexyloxy]-2-methoxypropyl-tert-butyldiphenylsilylether erhält man aus (2R/2S)-3-[(1S,3R)-3-Benzyloxycyclohexyloxy]-2-hydroxypropyl-tert-butyldiphenylsilylether und Isobutenylbromid (2R/2S)-3-[(1S,3R)-3-Benzyloxycyclohexyloxy]-2-(2-methyl-2-propenloxy)-propyl-tert-butyldiphenylsilylether.

### (2R/2S)-3-[(1R,3S)-3-Benzoyloxycyclohex-1-yloxy]-2-(4-trifluormethylbenzyloxy)-propyl-tert-butyldimethylsilylether

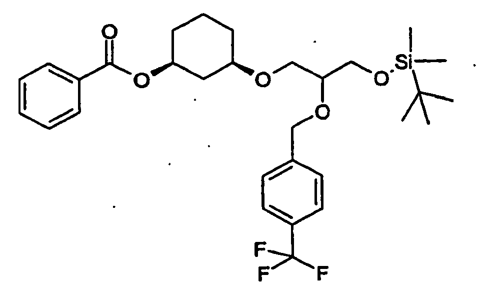

Analog zu (2R/2S)-3-[(1S,3R)-3-Benzoyloxycyclohex-1-yloxy]-2-(4-trifluormethylbenzyloxy)-tert-butyldimethylsilylether erhält man aus (2R/2S)-3-[(1R,3S)-1-Benzoyloxycyclohex-3-yloxy]-2-hydroxypropyl-tert-butyldiphenylsilylether und 4-Trifluomnethylbenzylbromid (2R/2S)-3-[(1R,3S)-3-Benzoyloxycyclohex-1-yloxy]-2-(4-trifluormethylbenzyloxy)-propyl-tert-butyldimethylsilytether.

### (2R/2S)-3-[(1R,3S)-3-Trityloxymethylcyclohexyloxy]-2-(4-trifluormethylbenzyloxy)-propyl-tert-butyldiphenylsilylether

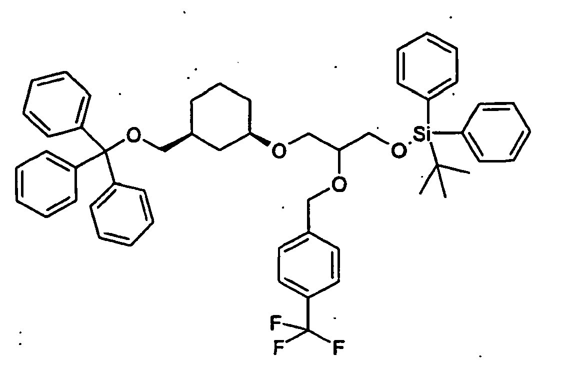

Analog zu (2R/2S)-3-[(1S,3R)-3-Benzyloxycyclohexyloxy]-2-methoxypropyl-tert-butyldiphenylsilylether erhält man aus (2R/2S)-3-[(1R,3S)-3-Trityloxymethylcyclohexyloxy]-2-hydroxypropyl-tert-butyldiphenylsilylether und 4-Trifluormethylbenzylbromid (2R/2S)-3-[(1R,3S)-3-Trityloxymethylcyclohexyloxy]-2-(4-trifluormethylbenzyloxy)-propyl-tert-butyldiphenylsilylether.

### (2R/2S)-3-[(1S,3R)-3-Hydroxycyclohexyloxy]-2-methoxypropyl-tert-butyldiphenylsilylether

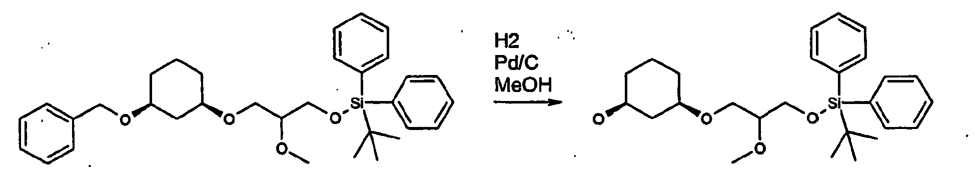

7,2 g (2R/2S)-3-[(1S,3R)-3-benzyloxycyclohexyloxy]-2-methoxypropyl-tert-butyldiphenylsilylether werden in 40 ml Methanol gelöst, mit 4 g Pd/C (10%) versetzt und bei RT und 5 bar Überdruck 15 h hydriert. Der Katalysator wird abfiltriert, der Rückstand mit Dichlormethan gewaschen, und das Filtrat wird eingeengt, wobei 6,0 g (2R/2S)-3-[(1S,3R)-3-Hydroxycyclohexyloxy]-2-methoxypropyl-tert-butyldiphenylsilylether als farbloses Öl erhalten werden. C26H38O4Si (442,68), MS (ESI): 443 (M + H⁺).

### (2R/2S)-3-[(1S,3R)-3-Hydroxycyclohexyloxy]-2-ethoxypropyl-tert-butyldiphenylsilylether

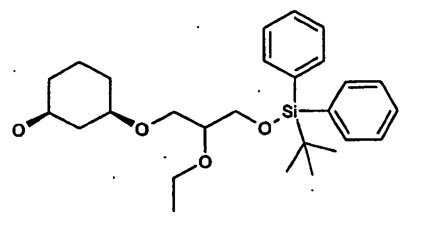

Analog zu (2R/2S)-3-[(1S,3R)-3-Hydroxycyclohexyloxy]-2-methoxypropyl-tert-butyldiphenylsilylether erhält man aus (2R/2S)-3-[(1S,3R)-3-Benzyloxycyclohexyloxy]-2-ethoxypropyl-tert-butyldiphenylsilylether (2R/2S)-3-[(1S,3R)-3-Hydroxycyclohexyloxy]-2-ethoxypropyl-tert-butyldiphenylsilylether.
C27H40O4Si (456,70), MS (ESI): 457 (M + H⁺).

### (2R/2S)-3-[(1R,3S)-3-Hydroxymethylcyclohexyloxy]-2-(4-trifluormethylbenzyloxy)-propyl-tert-butyldiphenylsilylether

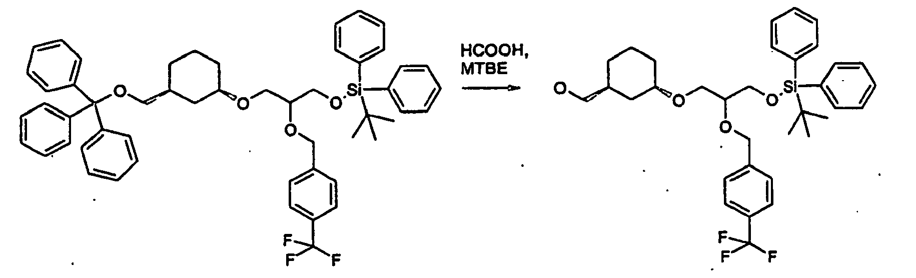

3,5 g (2R/2S)-3-[(1R,3S)-3-Trityloxymethylcyclohexyloxy]-2-(4-trifluormethylbenzyloxy)-propyl-tert-butyldiphenylsilylether werden in 100 ml MTBE gelöst, mit 58 g Ameisensäure versetzt und bei RT gerührt. Die Lösung wird eingeengt, der Rückstand an Kieselgel chromatographiert, wobei 170 mg (2R/2S)-3-[(1R,3S)-3-Hydroxymethylcyclohexyloxy]-2-(4-trifluormethylbenzyloxy)-propyl-tert-butyldiphenylsilylether als farbloses Öl erhalten werden.

### (2R/2S)-3-[(1S,3R)-3-Hydroxycyclohexyloxy]-2-propoxypropyl-tert-butyldiphenylsilylether

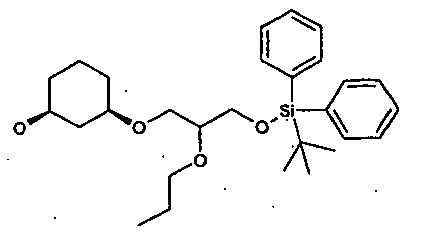

Analog zu (2R/2S)-3-[(1S,3R)-3-Hydroxycyclohexyloxy]-2-methoxypropyl-tert-butyldiphenylsilylether erhält man aus (2R/2S)-3-[(1S,3R)-3-Benzyloxycyclohexyloxy]-2-propoxypropyl-tert-butyldiphenylsilylether (2R/2S)-3-[(1S,3R)-3-Hydroxycyclohexyloxy]-2-propoxypropyl-tert-butyldiphenylsilylether.
C28H4204Si (470,73), MS (ESI): 471 (M + H⁺).

### (2R/2S)-3-[(1S,3R)-3-Hydroxycyclohexyloxy]-2-isobutoxypropyl-tert-butyldiphenylsilylether

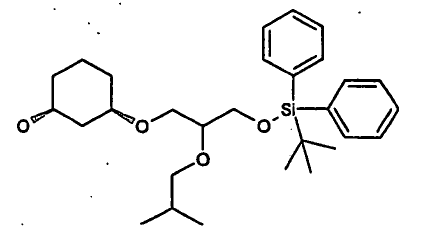

Analog zu (2R/2S)-3-[(1S,3R)-3-Hydroxycyclohexyloxy]-2-methoxypropyl-tert-butyldiphenylsilylether erhält man aus (2R/2S)-3-[(1S,3R)-3-Benzyloxycyclohexyloxy]-2-isobutoxypropyl-tert-butyldiphenylsilylether (2R/2S)-3-[(1S,3R)-3-Hydroxycyclohexyloxy]-2-isobutoxypropyl-tert-butyldiphenylsilylether.
C29H4404Si (484,76), MS (ESI): 485 (M + H⁺).

### (2R/2S)-3-[(1R,3S)-1-Hydroxycyclohex-1-yloxy]-2-(4-trifluormethylbenzyloxy)-tert-butyldimethylsilylether

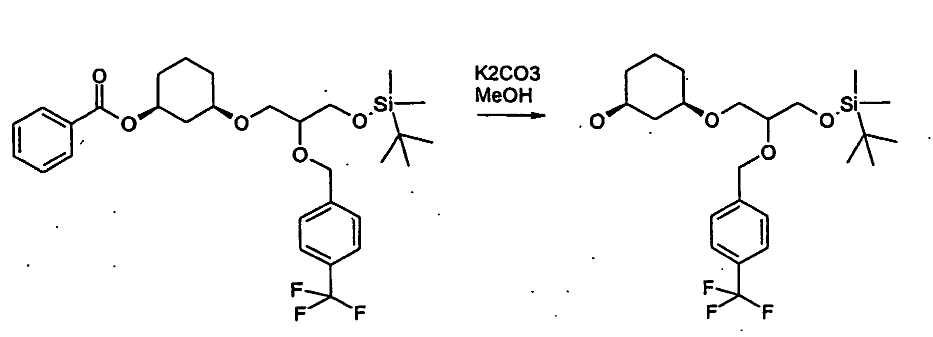

4,6 g (2R/2S)-3-[(1S,3R)-1-Benzoyloxycyclohex-3-yloxy]-2-(4-trifluormethoxybenzyloxy)-tert-butyldimethylsilylether werden in 20 ml Methanol gelöst, mit 2,2 g K2CO3 versetzt und bei RT 72 h gerührt. K2CO3 wird abfiltriert, der Rückstand mit Methanol gewaschen, und das Filtrat wird eingeengt, wobei 0,85 g (2R/2S)-3-[(1R,3S)-3-Hydroxycyclohexyloxy]-2-(4-trifluormethylbenzyloxy)-tert-butyldimethylsilylether als farbloses Öl erhalten werden. C23H37F304Si (462,63), MS (ESI): 463 (M + H⁺).

### 2-Methoxy-3-[(1R,3S)-3-(5-methyl-2-phenyl-oxazol-4-ylmethoxy)-cyclohexyloxy]-propansäure

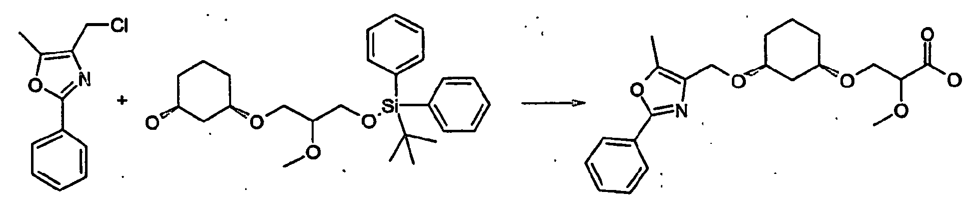

100 mg (2R/2S)-3-[(1S,3R)-3-Hydroxycyclohexyloxy]-2-methoxypropyl-tert-butyldiphenylsilylether werden in 2 ml THF gelöst und nacheinander mit 83 mg 2-Phenyl-5-methyloxazol-4-ylmethylchlorid und 51 mg KOtBu versetzt. Bei RT wird die Reaktionsmischung über Nacht geschüttelt. 214 mg TBAF werden zugeben und die Suspension über Nacht stehen lassen. Nach Zugabe von Wasser und MBTE wird die organische Phase abgetrennt und eingeengt. Der Rückstand wird in Aceton aufgenommen und mit 1 ml 1,92 M Jones Reagenz versetzt. Die Reaktionslösung wird über Nacht geschüttelt. Die Lösung wird mit 3 ml Wasser verdünnt und auf eine Extraktionskartusche (Kieselgur, für 20 ml wäßrige Phasen) gegossen. Anschließend wird mit Ethylacetat eluiert, die erhaltene Lösung wird eingeengt und per HPLC gereinigt. Es werden 5 mg 2-Methoxy-3-[(1R,3S)-3-(5-methyl-2-phenyl-oxazol-4-ylmethoxy)-cyclohexyloxy]-propansäure als gelbes Öl erhalten.

### Beispiel 29:

### 2-Methoxy-3-{(1R,3S)-3-[5-methyl-2-(3-trifluoromethyl-phenyl)-oxazol-4-ylmethoxy]-cyclohexyloxy}-propionsäure

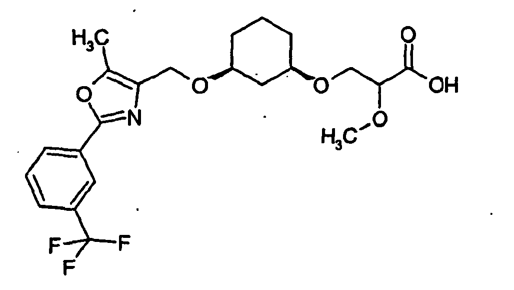

Angepaßt an die Reaktionsbedingungen des Beispiels 28 erhält man aus (2R/2S)-3-[(1 S,3R)-3-Hydroxycyclohexyloxy]-2-methoxypropyl-tert-butyldiphenylsilylether und 5-Methyl-2-(3-trifluoromethylphenyl)-oxazol-4-ylmethyliodid 2-Methoxy-3-{(1R,3S)-3-[5-methyl-2-(3-trifluoromethyl-phenyl)-oxazol-4-ylmethoxy]-cyclohexyloxy}-propionsäure.

### Beispiel 30:

### 2-Methoxy-3-{(1R,3S)-3-[5-methyl-2-(3-trifluoromethoxy-phenyl)-oxazol-4-ylmethoxy]-cyclohexyloxy}-propionsäure

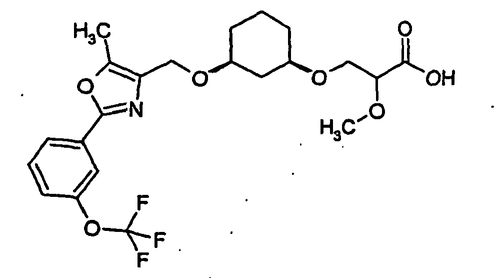

Angepaßt an die Reaktionsbedingungen des Beispiels 28 erhält man aus (2R/2S)-3-[(1S,3R)-3-Hydroxycyclohexyloxy]-2-methoxypropyl-tert-butyldiphenylsilylether und 5-Methyl-2-(3-trifluoromethoxy-phenyl)-oxazol-4-ylmethyliodid 2-Methoxy-3-{(1R,3S)-3-[5-methyl-2-(3-trifluoromethoxy-phenyl)-oxazol-4-ylmethoxy]-cyclohexyloxy}-propionsäure

### Beispiel 31:

### 2-Methoxy-3-{(1R,3S)-3-[5-methyl-2-(4-trifluoromethyl-phenyl)-oxazol-4-ylmethoxy]-cyclohexyloxy}-propionsäure

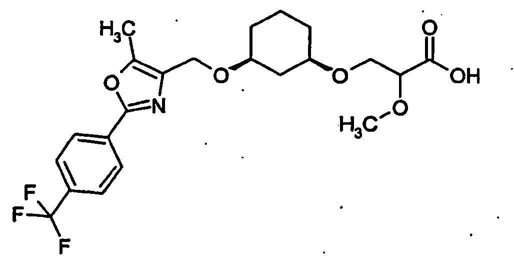

Angepaßt an die Reaktionsbedingungen des Beispiels 28 erhält man aus (2R/2S)-3-[(1S,3R)-3-Hydroxycyclohexyloxy]-2-methoxypropyl-tert-butyldiphenylsliylether und 5-Methyl-2-(4-trifluoromethylphenyl)-oxazol-4-ylmethyliodid 2-Methoxy-3-{(1R,3S)-3-[5-methyl-2-(4-trifluoromethyl-phenyl)-oxazol-4-ylmethoxy]-cyclohexyloxy}-propionsäure.

### Beispiel 32:

### 2-Methoxy-3-{(1R,3S)-3-[5-methyl-2-(4-methyl-phenyl)-oxazol-4-ylmethoxy]-cyclohexyloxy}-propionsäure

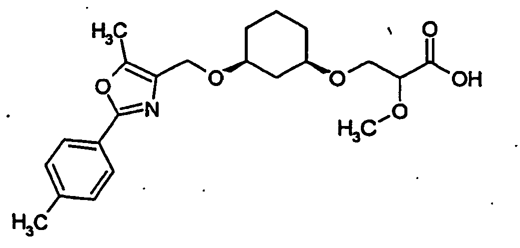

Angepaßt an die Reaktionsbedingungen des Beispiels 28 erhält man aus (2R/2S)-3-[(1 S,3R)-3-Hydroxycyclohexyloxy]-2-methoxypropyl-tert-butyldiphenylsilylether und 5-Methyl-2-(4-methylphenyl)-oxazol-4-ylmethyliodid 2-Methoxy-3-{(1R,3S)-3-[5-methyl-2-(4-methyl-phenyl)-oxazol-4-ylmethoxy]-cyclohexyloxy}-propionsäure

### Beispiel 33:

### 2-Methoxy-3-{(1R,3S)-3-[5-methyl-2-(2,6-dimethyl-phenyl)-oxazol-4-ylmethoxy]-cyclohexyloxy}-propionsäure

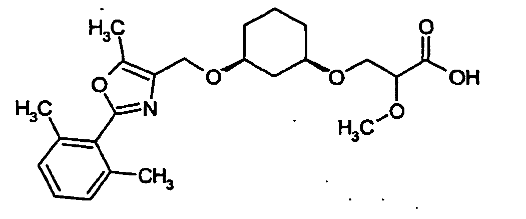

Angepaßt an die Reaktionsbedingungen des Beispiels 28 erhält man aus (2R/2S)-3-[(1S,3R)-3-Hydroxycyclohexyloxy]-2-methoxypropyl-tert-butyldiphenylsilylether und 5-Methyl-2-(2,6-dimethylphenyl)-oxazol-4-ylmethyliodid 2-Methoxy-3-{(1R,3S)-3-[5-methyl-2-(2,6-dimethyl-phenyl)-oxazol-4-ylmethoxy]-cyclohexyloxy}-propionsäure.

### Beispiel 34:

### 2-Methoxy-3-{(1R,3S)-3-[5-methyl-2-(2-trifluoromethyl-phenyl)-oxazol-4-ylmethoxy]-cyclohexyloxy}-propionsäure

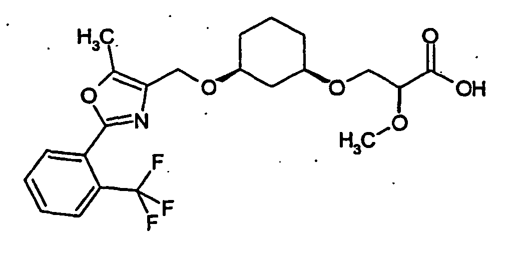

Angepaßt an die Reaktionsbedingungen des Beispiels 28 erhält man aus (2R/2S)-3-[(1S,3R)-3-Hydroxycyclohexyloxy]-2-methoxypropyl-tert-butyldiphenylsilylether und 5-Methyl-2-(2-trifluoromethylphenyl)-oxazol-4-ylmethyliodid 2-Methoxy-3-{(1R,3S)-3-[5-methyl-2-(2-trifluoromethyl-phenyl)-oxazol-4-ylmethoxy]-cyclohexyloxy}-propionsäure.

### Beispiel 35:

### 2-Methoxy-3-{(1R,3S)-3-[5-methyl-2-(3-naphthyl)-oxazol-4-ylmethoxy]-cyclohexyloxy}-propionsäure

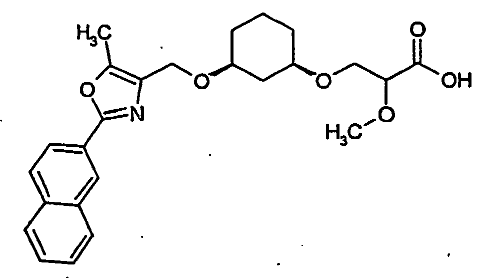

Angepaßt an die Reaktionsbedingungen des Beispiels 28 erhält man aus (2R/2S)-3-[(1S,3R)-3-Hydroxycyclohexyloxy]-2-methoxypropyl-tert-butyldiphenylsilylether und 5-Methyl-2-(2-naphthyl)-oxazol-4-ylmethyliodid 2-Methoxy-3-{(1R,3S)-3-[5-methyl-2-(3-naphthyl)-oxazol-4-ylmethoxy]-cyclohexyloxy}-propionsäure.

### Beispiel 36:

### 2-Methoxy-3-{(1R,3S)-3-[5-methyl-2-(2,4-dimethyl-phenyl)-oxazol-4-ylmethoxy]-cyclohexyloxy}-propionsäure

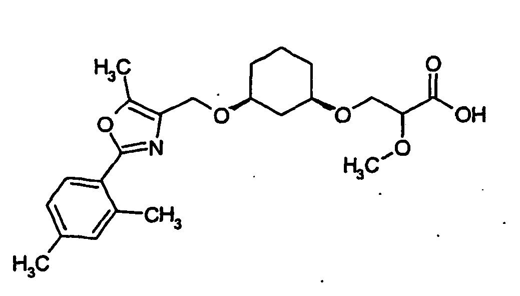

Angepaßt an die Reaktionsbedingungen des Beispiels 28 erhält man aus (2R/2S)-3-[(1S,3R)-3-Hydroxycyclohexyloxy]-2-methoxypropyl-tert-butyldiphenylsilylether und 5-Methyl-2-(2,4-dimethylphenyl)-oxazol-4-ylmethyliodid 2-Methoxy-3-{(1R,3S)-3-[5-methyl-2-(2,4-dimethyl-phenyl)-oxazol-4-ylmethoxy]-cyclohexyloxy}-propionsäure.

### Beispiel 37:

### 2-Methoxy-3-{(1R,3S)-3-[5-methyl-2-(4-biphenyl)-oxazol-4-ylmethoxy]-cyclohexyloxy}-propionsäure

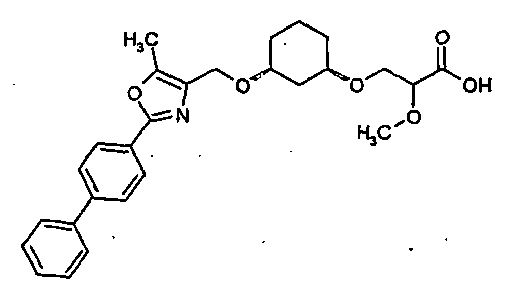

Angepaßt an die Reaktionsbedingungen des Beispiels 28 erhält man aus (2R/2S)-3-[(1S,3R)-3-Hydroxycyclohexyloxy]-2-methoxypropyl-tert-butyldiphenylsilylether und 5-Methyl-2-(4-biphenyl)-oxazol-4-ylmethyliodid 2-Methoxy-3-{(1R,3S)-3-[5-methyl-2-(4-biphenyl)-oxazol-4-ylmethoxy]-cyclohexyloxy}-propionsäure.

### Beispiel 38:

### 2-Methoxy-3-{(1R,3S)-3-[5-ethyl-2-(3-methoxy-phenyl)-oxazol-4-ylmethoxy]-cyclohexyloxy}-propionsäure

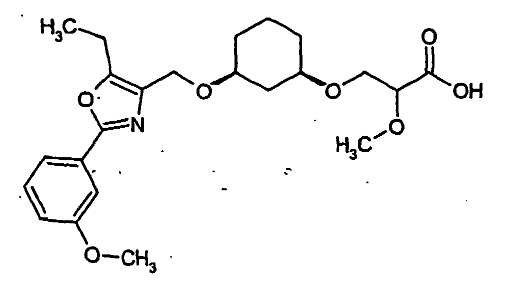

Angepaßt an die Reaktionsbedingungen des Beispiels 28 erhält man aus (2R/2S)-3-[(1S,3R)-3-Hydroxycyclohexyloxy]-2-methoxypropyl-tert-butyldiphenylsilylether und 5-Ethyl-2-(3-methoxyphenyl)-oxazol-4-ylmethyliodid 2-Methoxy-3-{(1R,3S)-3-[5-ethyl-2-(3-methoxyphenyl)-oxazol-4-ylmethoxy]-cyclohexyloxy}-propionsäure.

### Beispiel 39:

### 2-Methoxy-3-{(1R,3S)-3-[5-ethyl-2-(4-methylphenyl)-oxazol-4-ylmethoxy]-cyclohexyloxy}-propionsäure

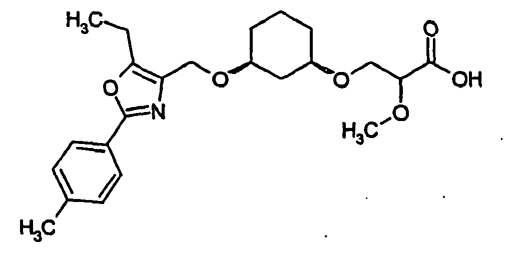

Angepaßt an die Reaktionsbedingungen des Beispiels 28 erhält man aus (2R/2S)-3-[(1S,3R)-3-Hydroxycyclohexyloxy]-2-methoxypropyl-tert-butyldiphenylsilylether und 5-Ethyl-2-(4-methylphenyl)-oxazol-4-ylmethyliodid 2-Methoxy-3-{(1R,3S)-3-[5-ethyl-2-(4-methylphenyl)-oxazol-4-ylmethoxy]-cyclohexyloxy}-propionsäure.

### Beispiel 40:

### 2-Methoxy-3-{(1R,3S)-3-[5-ethyl-2-(2-trifluoromethylphenyl)-oxazol-4-ylmethoxy]-cyclohexyloxy}-propionsäure

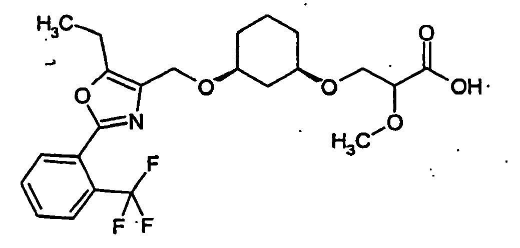

Angepaßt an die Reaktionsbedingungen des Beispiels 28 erhält man aus (2R/2S)-3-[(1S,3R)-3-Hydroxycyclohexyloxy]-2-methoxypropyl-tert-butyldiphenylsilylether und 5-Ethyl-2-(2-trifluoromethylphenyl)-oxazol-4-ylmethyliodid 2-Methoxy-3-{(1R,3S)-3-[5-ethyl-2-(2-trifluoromethylphenyl)-oxazol-4-ylmethoxy]-cyclohexyloxy}-propionsäure.

### Beispiel 41:

### 2-Methoxy-3-{(1R,3S)-3-[5-ethyl-2-(2,6-dimethylphenyl)-oxazol-4-ylmethoxy]-cyclohexyloxy}-propionsäure

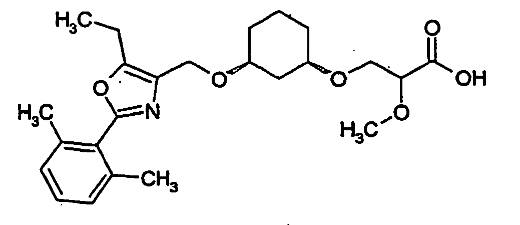

Angepaßt an die Reaktionsbedingungen des Beispiels 28 erhält man aus (2R/2S)-3-[(1S,3R)-3-Hydroxycyclohexyloxy]-2-methoxypropyl-tert-butyldiphenylsilylether und 5-Ethyl-2-(2,6-methylphenyl)-oxazol-4-ylmethyliodid 2-Methoxy-3-{(1R,3S)-3-[5-ethyl-2-(2,6-dimethylphenyl)-oxazol-4-ylmethoxy]-cyclohexyloxy}-propionsäure.

### Beispiel 42:

### 3-[(1R,3S)-3-(5-Methyl-2-phenyl-oxazol-4-ylmethoxy)-cyclohexyloxy]-2-propoxy-propionsäure

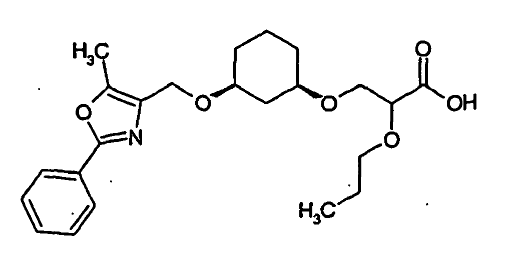

Angepaßt an die Reaktionsbedingungen des Beispiels 28 erhält man aus (2R/2S)-3-[(1S,3R)-3-Hydroxycyclohexyloxy]-2-propoxypropyl-tert-butyldiphenylsilylether und 5-Methyl-2-phenyl-oxazol-4-ylmethyliodid 3-[(1R,3S)-3-(5-Methyl-2-phenyl-oxazol-4-ylmethoxy)-cyclohexyloxy]-2-propoxypropionsäure.

### Beispiel 43:

### 3-[(1R,3S)-3-(5-Methyl-2-(3-tolyl)-oxazol-4-ylmethoxy)-cyclohexyloxy]-2-propoxypropionsäure

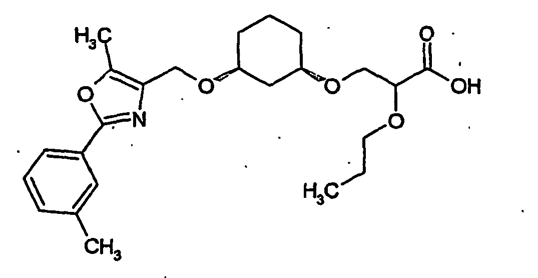

Angepaßt an die Reaktionsbedingungen des Beispiels 28 erhält man aus (2R/2S)-3-[(1S,3R)-3-Hydroxycyclohexyloxy]-2-propoxypropyl-tert-butyldiphenylsilylether und 5-Methyl-2-(3-tolyl)-oxazol-ylmethyliodid 3-[(1R,3S)-3-(5-Methyl-2-(3-tolyl)-oxazol-4-ylmethoxy)-cyclohexyloxy]-2-propoxy-propionsäure.

### Beispiel 44:

### 3-{(1R,3S)-3-[5-Methyl-2-(3-trifluoromethoxy-phenyl)-oxazol-4-ylmethoxy]-cyclohexyloxy}-2-propoxy-propionsäure

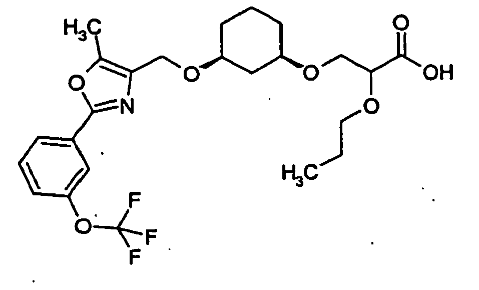

Angepaßt an die Reaktionsbedingungen des Beispiels 28 erhält man aus (2R/2S)-3-[(1S,3R)-3-Hydroxycyclohexyloxy]-2-propoxypropyl-tert-butyldiphenylsilylether 2-(3-Trifluoromethoxyphenyl)-oxazol-4-ylmethyliodid 3-{(1R,3S)-3-[5-Methyl-2-(3-trifluoromethoxy-phenyl)-oxazol-4-ylmethoxy]-cyclohexyloxy}-2-propoxy-propionsäure.

### Beispiel 45:

### 3-{(1R,3S)-3-[5-Methyl-2-(3-methoxy-phenyl)-oxazol-4-ylmethoxy]-cyclohexyloxy}-2-propoxy-propionsäure

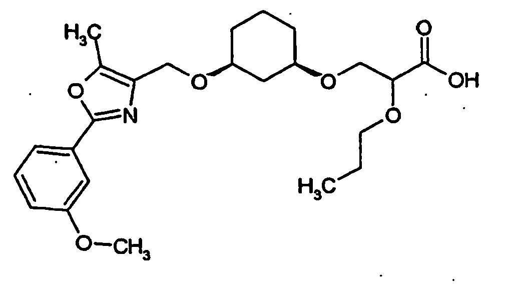

Angepaßt an die Reaktionsbedingungen des Beispiels 28 erhält man aus (2R/2S)-3-[(1S,3R)-3-Hydroxycyclohexyloxy]-2-propoxypropyl-tert-butyldiphenylsilylether und 2-(3-Methoxyphenyl)-oxazol-4-ylmethyliodid 3-{(1R,3S)-3-[5-Methyl-2-(3-methoxyphenyl)-oxazol-4-ylmethoxy]-cyclohexyloxy}-2-propoxy-propionsäure.

### Beispiel 46:

### 3-{(1R,3S)-3-[5-Methyl-2-(4-trifluormethylphenyl)-oxazol-4-ylmethoxy]-cyclohexyloxy}-2-propoxy-propionsäure

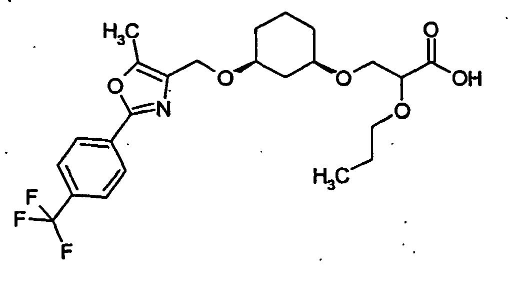

Angepaßt an die Reaktionsbedingungen des Beispiels 28 erhält man aus (2R/2S)-3-[(1S,3R)-3-Hydroxycyclohexyloxy]-2-propoxypropyl-tert-butyldiphenylsilylether und 2-(4-trifluormethylphenyl)-oxazol-4-ylmethyliodid 3-{(1R,3S)-3-[5-Methyl-2-(4-trifluormethylphenyl)-oxazol-4-ylmethoxy]-cyclohexyloxy}-2-propoxy-propionsäure.

### Beispiel 47:

### 3-{(1R,3S)-3-[5-Methyl-2-(4-methylphenyl)-oxazol-4-ylmethoxy]-cyclohexyloxy}-2-propoxy-propionsäure

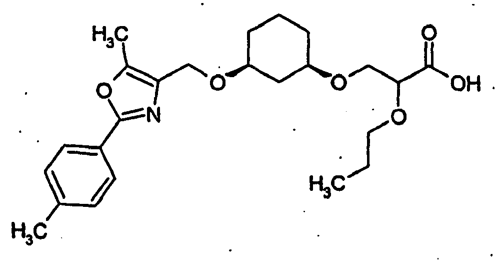

Angepaßt an die Reaktionsbedingungen des Beispiels 28 erhält man aus (2R/2S)-3-[(1 S,3R)-3-Hydroxycyclohexyloxy]-2-propoxypropyl-tert-butyldiphenylsilylether und 2-(4-Methylphenyl)-oxazol-4-ylmethyliodid 3-{(1R,3S)-3-[5-Methyl-2-(4-methylphenyl)-öxazol-4-ylmethoxy]-cyclohexyloxy}-2-propoxy-propionsäure.

### Beispiel 48:

### 3-{(1R,3S)-3-[5-Methyl-2-(4-trifluormethoxy-phenyl)-oxazol-4-ylmethoxy]-cyclohexyloxy}-2-propoxy-propionsäure

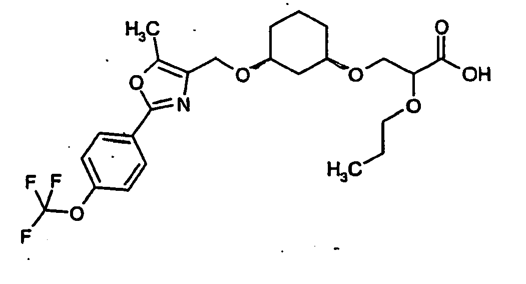

Angepaßt an die Reaktionsbedingungen des Beispiels 28 erhält man aus (2R/2S)-3-[(1S,3R)-3-Hydroxycyclohexyloxy]-2-propoxypropyl-tert-butyldiphenylsilylether und 2-(4-Trifluormethoxyphenyl)-oxazol-4-ylmethyliodid 3-{(1R,3S)-3-[5-Methyl-2-(4-trifluormethoxy-phenyl)-oxazol-4-ylmethoxy]-cyclohexyloxy}-2-propoxy-propionsäure.

### Beispiel 49:

### 3-{(1R,3S)-3-[5-Methyl-2-(4-isopropylphenyl)-oxazol-4-ylmethoxy]-cyclohexyloxy}-2-propoxy-propionsäure

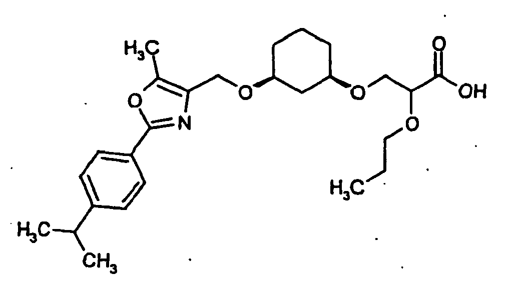

Angepaßt an die Reaktionsbedingungen des Beispiels 28 erhält man aus (2R/2S)-3-[(1S,3R)-3-Hydroxycyclohexyloxy]-2-propoxypropyl-tert-butyldiphenylsilylether und 2-(4-isopropylphenyl)-oxazol-4-ylmethyliodid 3-{(1R,3S)-3-[5-Methyl-2-(4-isopropylphenyl)-oxazol-4-ylmethoxy]-cyclohexyloxy}-2-propoxy-propionsäure.

### Beispiel 50:

### 3-{(1R,3S)-3-[5-Methyl-2-(2-trifluormethylphenyl)-oxazol-4-ylmethoxy]-cyclohexyloxy}-2-propoxy-propionsäure

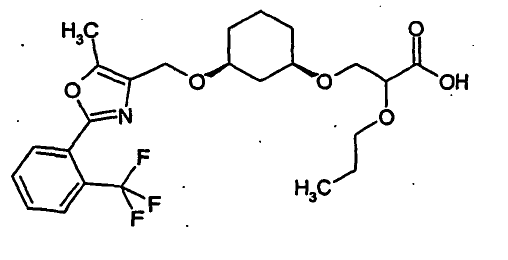

Angepaßt an die Reaktionsbedingungen des Beispiels 28 erhält man aus (2R/2S)-3-[(1S,3R)-3-Hydroxycyclohexyloxy]-2-propoxypropyl-tert-butyldiphenylsilylether und 2-(2-trifluormethylphenyl)-oxazol-4-ylmethyliodid 3-{(1R,3S)-3-[5-Methyl-2-(2-trifluormethylphenyl)-oxazol-4-ylmethoxy]-cyclohexyloxy}-2-propoxy-propionsäure.

### Beispiel 51:

### 3-{(1R,3S)-3-[5-Methyl-2-(2-naphthylphenyl)-oxazol-4-ylmethoxy]-cyclohexyloxy}-2-propoxy-propionsäure

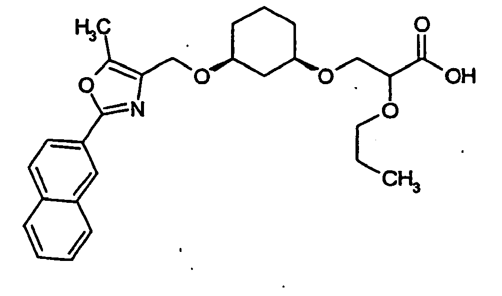

Angepaßt an die Reaktionsbedingungen des Beispiels 28 erhält man aus (2R/2S)-3-[(1S,3R)-3-Hydroxycyclohexyloxy]-2-propoxypropyl-tert-butyldiphenylsilylether und 2-(2-naphthyl)-oxazol-4-ylmethyliodid 3-{(1R,3S)-3-[5-Methyl-2-(2-naphthylphenyl)-oxazol-4-ylmethoxy]-cyclohexyloxy}-2-propoxy-propionsäure.

### Beispiel 52:

### 3-{(1R,3S)-3-[5-Methyl-2-(3-trifluormethylphenyl)-oxazol-4-ylmethoxy]-cyclohexyloxy}-2-propoxy-propionsäure

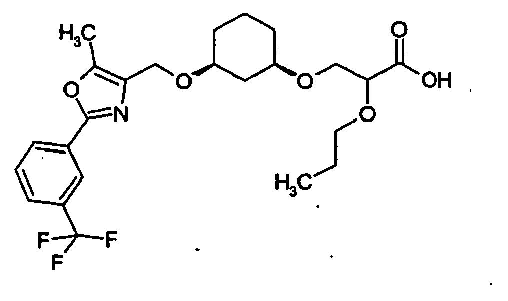

Angepaßt an die Reaktionsbedingungen des Beispiels 28 erhält man aus (2R/2S)-3-[(1S,3R)-3-Hydroxycyclohexyloxy]-2-propoxypropyl-tert-butyldiphenylsilylether und 2-(3-Trifluormethylphenyl)-oxazol-4-ylmethyliodid 3-{(1R,3S)-3-[5-Methyl-2-(3-trifluormethylphenyl)-oxazol-4-ylmethoxy]-cyclohexyloxy}-2-propoxy-propionsäure.

### Beispiel 53:

### 3-{(1R,3S)-3-[5-Ethyl-2-(3-methoxy-phenyl)-oxazol-4-ylmethoxy]-cyclohexyloxy}-2-propoxy-propionsäure

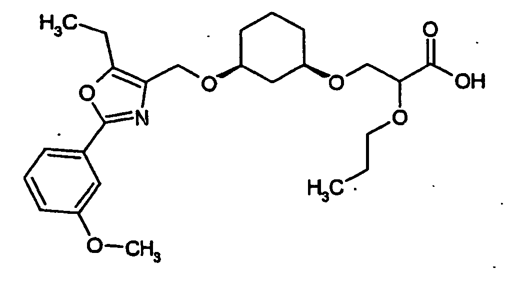

Angepaßt an die Reaktionsbedingungen des Beispiels 28 erhält man aus (2R/2S)-3-[(1S,3R)-3-Hydroxycyclohexyloxy]-2-propoxypropyl-tert-butyldiphenylsilylether und 5-Ethyl-2-(3-methoxy-phenyl)-oxazol-4-ylmethyliodid 3-{(1R,3S)-3-[5-Ethyl-2-(3-methoxy-phenyl)-oxazol-4-ylmethoxy]-cyclohexyloxy}-2-propoxy-propionsäure.

### Beispiel 54:

### 3-{(1R,3S)-3-[5-Ethyl-2-(4-methylphenyl)-oxazol-4-ylmethoxy]-cyclohexyloxy}-2-propoxy-propionsäure

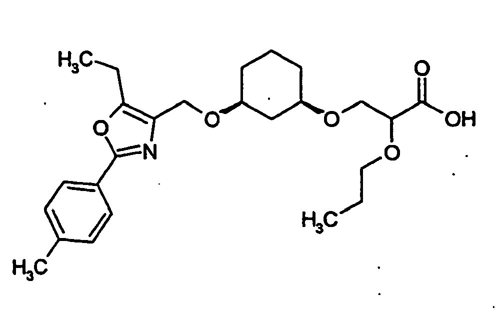

Angepaßt an die Reaktionsbedingungen des Beispiels 28 erhält man aus (2R/2S)-3-[(1S,3R)-3-Hydroxycyclohexyloxy]-2-propoxypropyl-tert-butyldiphenylsilylether und 5-Ethyl-2-(4-methylphenyl)-oxazol-4-ylmethyliodid 3-{(1R,3S)-3-[5-Ethyl-2-(4-methylphenyl)-oxazol-4-ylmethoxy]-cyclohexyloxy}-2-propoxy-propionsäure.

### Beispiel 55:

### 3-{(1R,3S)-3-[5-Ethyl-2-(2-trifluormethylphenyl)-oxazol-4-ylmethoxy]-cyclohexyloxy}-2-propoxy-propionsäure

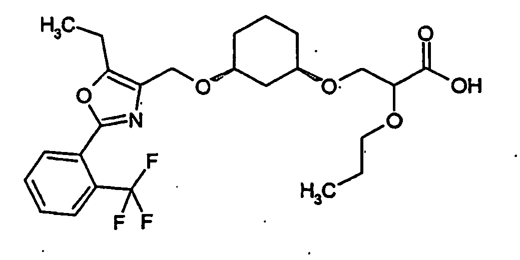

Angepaßt an die Reaktionsbedingungen des Beispiels 28 erhält man aus (2R/2S)-3-[(1S,3R)-3-Hydroxycyclohexyloxy]-2-propoxypropyl-tert-butyldiphenylsilylether und 5-Ethyl-2-(2-trifluormethylphenyl)-oxazol-4-ylmethyliodid 3-{(1R,3S)-3-[5-Ethyl-2-(2-trifluormethylphenyl)-oxazol-4-ylmethoxy]-cyclohexyloxy}-2-propoxy-propionsäure.

### Beispiel 56:

### 3-{(1R,3S)-3-[5-Ethyl-2-(2,6-dimethylphenyl)-oxazol-4-ylmethoxy]-cyclohexyloxy}-2-propoxy-propionsäure

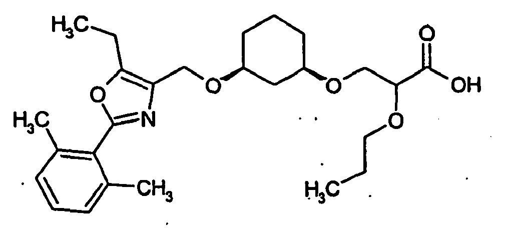

Angepaßt an die Reaktionsbedingungen des Beispiels 28 erhält man aus (2R/2S)-3-[(1 S,3R)-3-Hydroxycyclohexyloxy]-2-propoxypropyl-tert-butyldiphenylsilylether und 5-Ethyl-2-(2,6-dimethylphenyl)-oxazol-4-ylmethyliodid 3-{(1R,3S)-3-[5-Ethyl-2-(2,6-dimethylphenyl)-oxazol-4-ylmethoxy]-cyclohexyloxy}-2-propoxy-propionsäure.

### Beispiel 57:

### 2-Ethoxy-3-{(1R,3S)-3-[5-methyl-2-(3-trifluoromethyl-phenyl)-oxazol-4-ylmethoxy]-cyclohexyloxy}-propionsäure

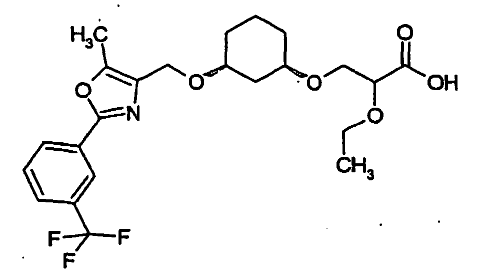

Angepaßt an die Reaktionsbedingungen des Beispiels 28 erhält man aus (2R/2S)-3-[(1S,3R)-3-Benzyloxycyclohexyloxy]-2-ethoxypropyl-tert-butyldiphenylsilylether und 5-methyl-2-(3-trifluoromethyl-phenyl)-oxazol-4-ylmethyliodid 2-Ethoxy-3-{(1R,3S)-3-[5-methyl-2-(3-trifluoromethyl-phenyl)-oxazol-4-ylmethoxy]-cyclohexyloxy}-propionsäure.

### Beispiel 58:

### 2-Ethoxy-3-{(1R,3S)-3-[5-methyl-2-(3-trifluoromethoxyphenyl)-oxazol-4-ylmethoxy]-cyclohexyloxy}-propionsäure

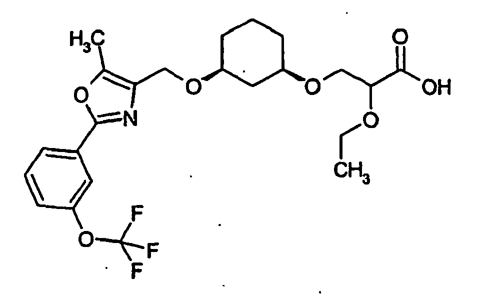

Angepaßt an die Reaktionsbedingungen des Beispiels 28 erhält man aus (2R/2S)-3-[(1S,3R)-3-Benzyloxycyclohexyloxy]-2-ethoxypropyl-tert-butyldiphenylsilylether und 5-methyl-2-(3-trifluoromethoxyphenyl)-oxazol-4-ylmethyliodid 2-Ethoxy-3-{(1R,3S)-3-[5-methyl-2-(3-trifluoromethoxyphenyl)-oxazol-4-ylmethoxy]-cyclohexyloxy}-propionsäure.

### Beispiel 59:

### 2-Ethoxy-3-{(1R,3S)-3-[5-methyl-2-(4-trifluoromethyl-phenyl)-oxazol-4-ylmethoxy]-cyclohexyloxy}-propionsäure

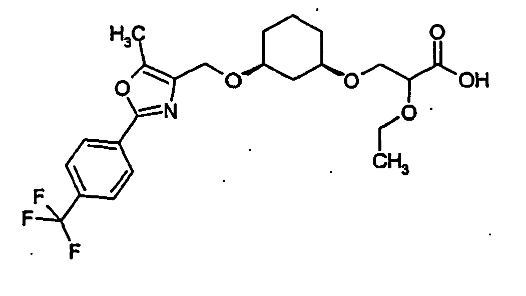

Angepaßt an die Reaktionsbedingungen des Beispiels 28 erhält man aus (2R/2S)-3-[(1S,3R)-3-Benzyloxycyclohexyloxy]-2-ethoxypropyl-tert-butyldiphenylsilylether und 5-methyl-2-(4-trifluoromethyl-phenyl)-oxazol-4-ylmethyliodid 2-Ethoxy-3-{(1R,3S)-3-[5-methyl-2-(4-trifluoromethyl-phenyl)-oxazol-4-ylmethoxy]-cyclohexyloxy}-propionsäure.

### Beispiel 60:

### 2-Ethoxy-3-{(1R,3S)-3-[5-methyl-2-(4-trifluoromethoxyphenyl)--oxazol-4-ylmethoxy]-cyclohexyloxy}-propionsäure

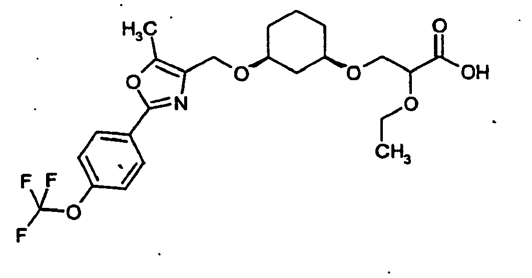

Angepaßt an die Reaktionsbedingungen des Beispiels 28 erhält man aus (2R/2S)-3-[(1S,3R)-3-Benzyloxycyclohexyloxy]-2-ethoxypropyl-tert-butyldiphenylsilylether und 5-methyl-2-(4-trifluoromethoxyphenyl)-oxazol-4-ylmethyliodid 2-Ethoxy-3-{(1R,3S)-3-[5-methyl-2-(4-trifluoromethoxyphenyl)-oxazol-4-ylmethoxy]-cyclohexyloxy}-propionsäure.

### Beispiel 61:

### 2-Ethoxy-3-{(1R,3S)-3-[5-methyl-2-(4-isopropylphenyl)-oxazol-4-ylmethoxy]-cyclohexyloxy}-propionsäure

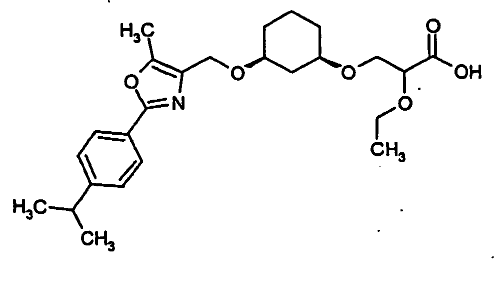

Angepaßt an die Reaktionsbedingungen des Beispiels 28 erhält man aus (2R/2S)-3-[(1S,3R)-3-Benzyloxycyclohexyloxy]-2-ethoxypropyl-tert-butyldiphenylsilylether und 5-methyl-2-(4-isopropylphenyl)-oxazol-4-ylmethyliodid 2-Ethoxy-3-{(1R,3S)-3-[5-methyl-2-(4-isopropylphenyl)-oxazol-4-ylmethoxy]-cyclohexyloxy}-propionsäure.

### Beispiel 62:

### 2-Ethoxy-3-{(1R,3S)-3-[5-methyl-2-(2-trifluoromethylphenyl)-oxazol-4-ylmethoxy]-cyclohexyloxy}-propionsäure

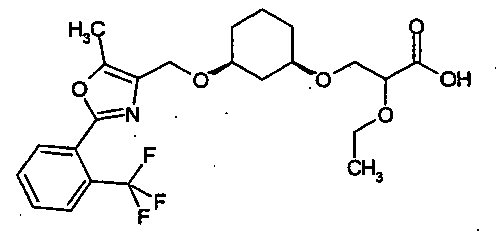

Angepaßt an die Reaktionsbedingungen des Beispiels 28 erhält man aus (2R/2S)-3-[(1S,3R)-3-Benzyloxycyclohexyloxy]-2-ethoxypropyl-tert-butyldiphenylsilylether und 5-methyl-2-(2-trifluoromethylphenyl)-oxazol-4-ylmethyliodid 2-Ethoxy-3-{(1R,3S)-3-[5-methyl-2-(2-trifluoromethylphenyl)-oxazol-4-ylmethoxy]-cyclohexyloxy}-propionsäure.

### Beispiel 63:

### 2-Ethoxy-3-{(1R,3S)-3-[5-methyl-2-(2,4-dimethylphenyl)-oxazol-4-ylmethoxy]-cyclohexyloxy}-propionsäure

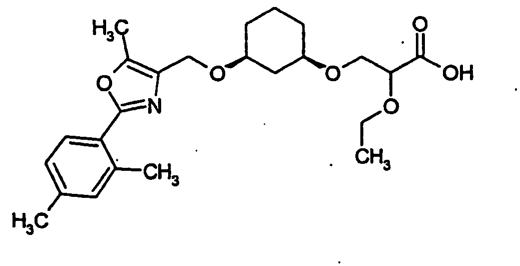

Angepaßt an die Reaktionsbedingungen des Beispiels 28 erhält man aus (2R/2S)-3-[(1S,3R)-3-Benzyloxycyclohexyloxy]-2-ethoxypropyl-tert-butyldiphenylsilylether und 5-methyl-2-(2,4-dimethylphenyl)-oxazol-4-ylmethyliodid 2-Ethoxy-3-{(1R,3S)-3-[5-methyl-2-(2,4-dimethylphenyl)-oxazol-4-ylmethoxy]-cyclohexyloxy}-propionsäure.

### Beispiel 64:

### 2-Ethoxy-3-{(1R,3S)-3-[5-methyl-2-(4-biphenyl)-oxazol-4-ylmethoxy]-cyclohexyloxy}-propionsäure

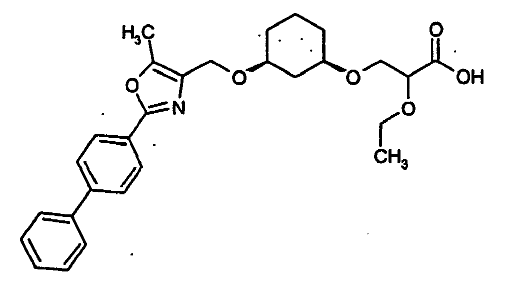

Angepaßt an die Reaktionsbedingungen des Beispiels 28 erhält man aus (2R/2S)-3-[(1S,3R)-3-Benzyloxycyclohexyloxy]-2-ethoxypropyl-tert-butyldiphenylsilylether und 5-methyl-2-(4-biphenyl)-oxazol-4-ylmethytiodid 2-Ethoxy-3-{(1R,3S)-3-[5-methyl-2-(4-biphenyl)-oxazol-4-ylmethoxy]-cyclohexyloxy}-propionsäure.

### Beispiel 65:

### 2-Ethoxy-3-[(1R,3S)-3-(5-ethyl-2-(2-tolyl)-oxazol-4-ylmethoxy)-cyclohexyloxy]-propionsäure

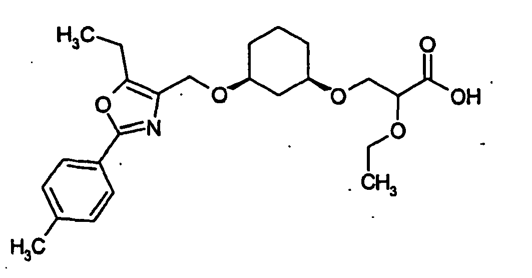

Angepaßt an die Reaktionsbedingungen des Beispiels 28 erhält man aus (2R/2S)-3-[(1S,3R)-3-Benzyloxycyclohexyloxy]-2-ethoxypropyl-tert-butyldiphenylsilylether und 5-methyl-2-(4-methylphenyl)-oxazol-4-ylmethyliodid 2-Ethoxy-3-[(1R,3S)-3-(5-ethyl-2-(2-tolyl)-oxazol-4-ylmethoxy)-cyclohexyloxy]-propionsäure.

### Beispiel 66:

### 2-Ethoxy-3-[(1R,3S)-3-(5-ethyl-2-(4-isopropylphenyl)-oxazol-4-ylmethoxy)-cyclohexyloxy]-propionsäure

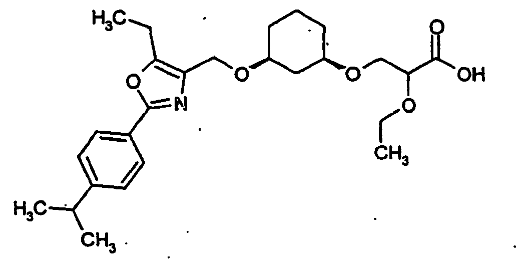

Angepaßt an die Reaktionsbedingungen des Beispiels 28 erhält man aus (2R/2S)-3-[(1S,3R)-3-Benzyloxycyclohexyloxy]-2-ethoxypropyl-tert-butyldiphenylsilylether und 5-methyl-2-(4-isopropylphenyl)-oxazol-4-ylmethyliodid 2-Ethoxy-3-[(1R,3S)-3-(5-ethyl-2-(4-isopropylphenyl)-oxazol-4-ylmethoxy)-cyclohexyloxy]-propionsäure.

### Beispiel 67:

### 2-Ethoxy-3-[(1R,3S)-3-(5-ethyl-2-(2-trifluormethylphenyl)-oxazol-4-ylmethoxy)-cyclohexyloxy]-propionsäure

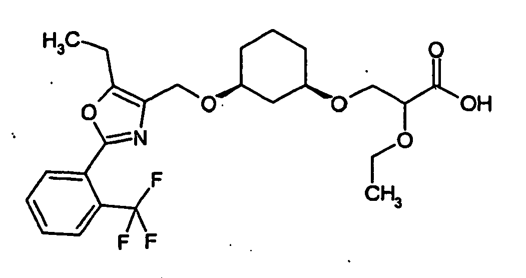

Angepaßt an die Reaktionsbedingungen des Beispiels 28 erhält man aus (2R/2S)-3-[(1 S,3R)-3-Benzyloxycyclohexyloxy]-2-ethoxypropyl-tert-butyldiphenylsilylether und 5-methyl-2-(2-trifluormethylphenyl)-oxazol-4-ylmethyliodid 2-Ethoxy-3-[(1R,3S)-3-(5-ethyl-2-(2-trifluormethylphenyl)-oxazol-4-ylmethoxy)-cyclohexyloxy]-propionsäure.

### Beispiel 68:

### 2-Ethoxy-3-[(1R,3S)-3-(5-ethyl-2-(2,4-dimethylphenyl)-oxazol-4-ylmethoxy)-cyclohexyloxy]-propionsäure

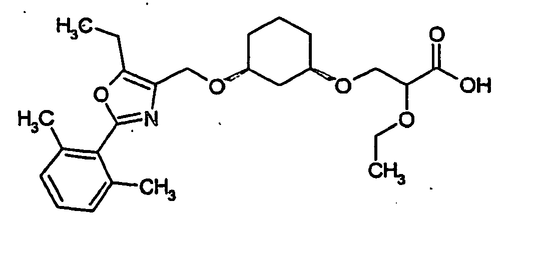

Angepaßt an die Reaktionsbedingungen des Beispiels 28 erhält man aus (2R/2S)-3-[(1 S,3R)-3-Benzyloxycyclohexyloxy]-2-ethoxypropyl-tert-butyldiphenylsilylether und 5-methyl-2-(2,4-dimethylphenyl)-oxazol-4-ylmethyliodid 2-Ethoxy-3-[(1R,3S)-3-(5-ethyl-2-(2,4-dimethylphenyl)-oxazol-4-ylmethoxy)-cyclohexyloxy]-propionsäure.

### Beispiel 69:

### 3-{(1R,3S)-3-[5-Ethyl-2-(4-isopropylphenyl)-oxazol-4-ylmethoxy]-cyclohexyloxy}-2-propoxy-propionsäure

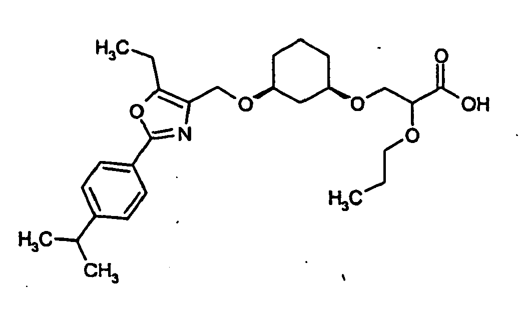

Angepaßt an die Reaktionsbedingungen des Beispiels 28 erhält man aus (2R/2S)-3-[(1S,3R)-3-Hydroxycyclohexyloxy]-2-propoxypropyl-tert-butyldiphenylsilylether und 5-Ethyl-2-(4- isopropylphenyl)-oxazol-4-ylmethyliodid 3-{(1R,3S)-3-[5-Ethyl-2-(4-isopropylphenyl)-oxazol-4-ylmethoxy]-cyclohexyloxy}-2-propoxy-propionsäure.

### Beispiel 70:

### 2-Ethoxy-3-{(1R,3S)-3-[5-methyl-2-phenyloxazol-4-ylmethoxy]-cyclohexyloxy}-propionsäure

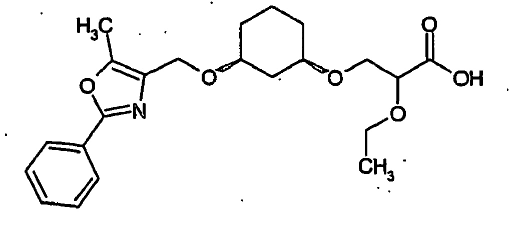

Angepaßt an die Reaktionsbedingungen des Beispiels 28 erhält man aus (2R/2S)-3-[(1S,3R)-3-Benzyloxycyclohexyloxy]-2-ethoxypropyl-tert-butyldiphenylsilylether und 5-methyl-2-phenyloxazol-4-ylmethyliodid 2-Ethoxy-3-{(1R,3S)-3-[5-methyl-2-phenyloxazol-4-ylmethoxy]-cyclohexyloxy}-propionsäure.

### Beispiel 71:

### 2-Ethoxy-3-{(1R,3S)-3-[5-methyl-2-(3-methylphenyl)-oxazol-4-ylmethoxy]-cyclohexyloxy}-propionsäure

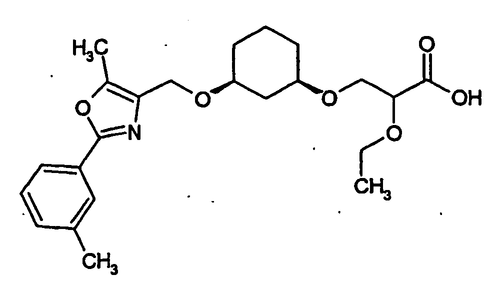

Angepaßt an die Reaktionsbedingungen des Beispiels 28 erhält man aus (2R/2S)-3-[(1S,3R)-3-Benzyloxycyclohexyloxy]-2-ethoxypropyl-tert-butyldiphenylsilylether und 5-methyl-2-(3-methylphenyl)-oxazol-4-ylmethyliodid 2-Ethoxy-3-{(1R,3S)-3-[5-methyl-2-(3-methylphenyl)-oxazol-4-ylmethoxy]-cyclohexyloxy}-propionsäure.

### Beispiel 72:

### 2-Ethoxy-3-{(1R,3S)-3-[5-methyl-2-(3-methoxyphenyl)-oxazol-4-ylmethoxy]-cyclohexyloxy}-propionsäure

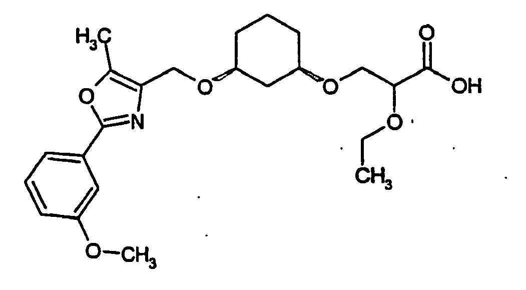

Angepaßt an die Reaktionsbedingungen des Beispiels 28 erhält man aus (2R/2S)-3-[(1S,3R)-3-Benzyloxycyclohexyloxy]-2-ethoxypropyl-tert-butyldiphenylsilylether und 5-methyl-2-(3-methoxyphenyl)-oxazol-4-ylmethyliodid 2-Ethoxy-3-{(1R,3S)-3-[5-methyl-2-(3-methoxyphenyl)-oxazol-4-ylmethoxy]-cyclohexyloxy}-propionsäure.

### Beispiel 73:

### 2-Ethoxy-3-{(1R,3S)-3-[5-methyl-2-(4-methylphenyl)-oxazol-4-ylmethoxy]-cyclohexyloxy}-propionsäure

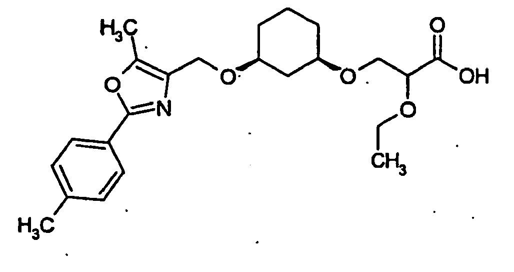

Angepaßt an die Reaktionsbedingungen des Beispiels 28 erhält man aus (2R/2S)-3-[(1S,3R)-3-Benzyloxycyclohexyloxy]-2-ethoxypropyl-tert-butyldiphenylsilylether und 5-methyl-2-(4-methylphenyl)-oxazol-4-ylmethyliodid 2-Ethoxy-3-{(1R,3S)-3-[5-methyl-2-(4-methylphenyl)-oxazol-4-ylmethoxy]-cyclohexyloxy}-propionsäure.

### Beispiel 74:

### 2-Ethoxy-3-{(1R,3S)3-[5-methyl-2-(2-naphthyl)-oxazol-4-ylmethoxy]-cyclohexyloxy}-propionsäure

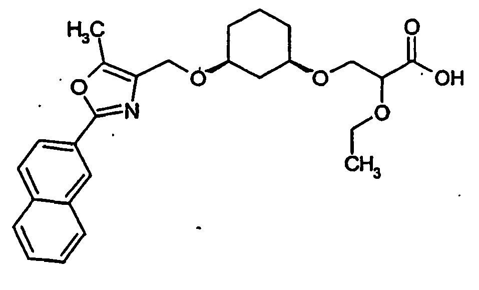

Angepaßt an die Reaktionsbedingungen des Beispiels 28 erhält man aus (2R/2S)-3-[(1S,3R)-3-Benzyloxycyclohexyloxy]-2-ethoxypropyl-tert-butyldiphenylsilylether und 5-methyl-2-(2-naphthyl)-oxazol-4-ylmethyliodid 2-Ethoxy-3-{(1R,3S)-3-[5-methyl-2-(2-naphthyl)-oxazol-4-ylmethoxy]-cyclohexyloxy}-propionsäure.

### Beispiel 75:

### 3-{(1R,3S)-3-[5-Ethyl-2-(3-methoxyphenyl)-oxazol-4-ylmethoxy]-cyclohexyloxy}-2-propoxy-propionsäure

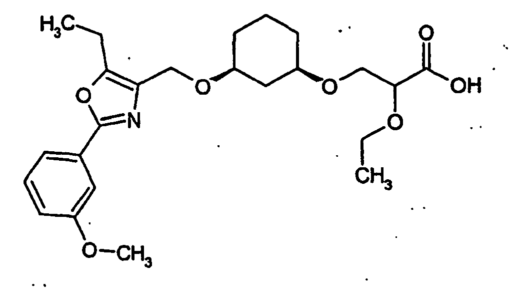

Angepaßt an die Reaktionsbedingungen des Beispiels 28 erhält man aus (2R/2S)-3-[(1S,3R)-3-Hydroxycyclohexyloxy]-2-propoxypropyl-tert-butyldiphenylsilylether und 5-Ethyl-2-(3-methoxyphenyl)-oxazol-4-ylmethyliodid 3-{(1R,3S)-3-[5-Ethyl-2-(3-methoxyphenyl)-oxazol-4-ylmethoxy]-cyclohexyloxy}-2-propoxy-propionsäure.

### Beispiel 76:

### (R)-3-[(1R,3S)-3-(5-Methyl-2-m-tolyl-oxazol-4-ylmethoxy)-cyclohexyloxy]-2-(4-trifluoromethyl-benzyloxy)-propionsäure

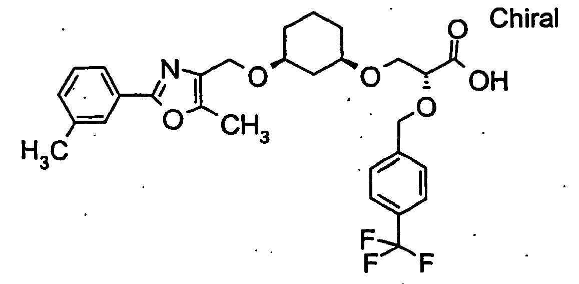

Durch Trennung von 3-[(1R,3S)-3-(5-Methyl-2-m-tolyl-oxazol-4-ylmethoxy)-cyclohexyloxy]-2-(4-trifluoroniethyl-benzyloxy)-propansäure (Beispiel 13) per chiraler HPLC erhält man neben enantiomerenreiner (S)-3-[(1R,3S)-3-(5-Methyl-2-m-tolyl-oxazol-4-ylmethoxy)-cyclohexyloxy]-2-(4-trifluoromethyl-benzyloxy)-propionsäure (Beispiel 10) (R)-3-[(1R,3S)-3-(5-Methyl-2-m-tolyl-oxazol-4-ylmethoxy)-cyclohexyloxy]-2-(4-trifluoromethyl-benzyloxy)-propionsäure.

### Beispiel 77:

### (R)-3-[(1R,3S)-3-(5-Methyl-2-(3-trifluormethylphenyl)-oxazol-4-ylmethoxy)-cyclohexyloxy]-2-(4-trifluoromethylbenzyloxy)-propionsäure

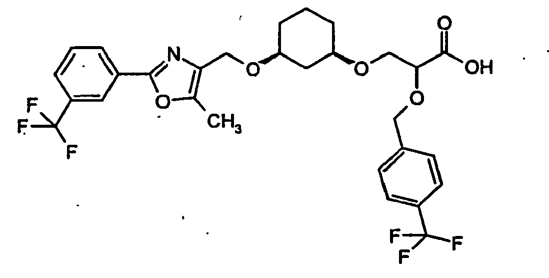

Angepaßt an die Reaktionsbedingungen des Beispiels 28 erhält man aus (2R/2S)-3-[(1R,3S)-1-Hydroxycyclohex-1-yloxy]-2-(4-trfluormethylbenzyloxy)-tert-butyldimethylsilylether und 5-methyl-2-(3-trifluormethylphenyl)-oxazol-4-ylmethyliodid (R)-3-[(1R,3S)3-(5-Methyl-2-(3-trifluormethylphenyl)-oxazol-4-ylmethoxy)-cyclohexyloxy]-2-(4-trifluoromethylbenzyloxy)-propionsäure.

### Beispiel 78:

### (R)-3-[(R,3S)-3-(5-Methyl-2-(4-trifluormethoxyphenyl)oxazol-4-ylmethoxy)-cyclohexytoxy]-2-(4-trifluoromethylbenzyloxy)-propionsäure

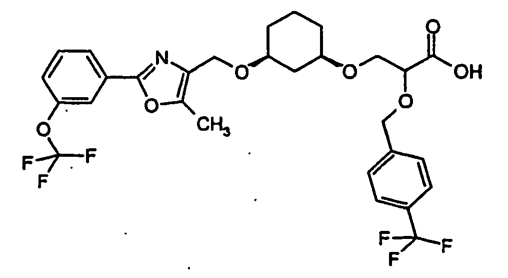

Angepaßt an die Reaktionsbedingungen des Beispiels 28 erhält man aus (2R/2S)-3-[(1R,3S)-1-Hydroxycyclohex-1-yloxy]-2-(4-trifluormethylbenzyloxy)-tert-butyldimethylsilylether und 5-methyl-2-(3-trifluormethoxyphenyl)-oxazol-4-ylmethyliodid (R)-3-[(R,3S)-3-(5-Methyl-2-(4-trifluormethoxyphenyl)-oxazol-4-ylmethoxy)-cyclohexyloxy]-2-(4-trifluoromethylbenzyloxy)-propionsäure.

### Beispiel 79:

### (R)-3-[(1R,3S)-3-(5-Methyl-2-(4-isopropylphenyl)-oxazol-4-ylmethoxy)-cyclohexyloxy]-2-(4-trifluoromethylbenzyloxy)-propionsäure

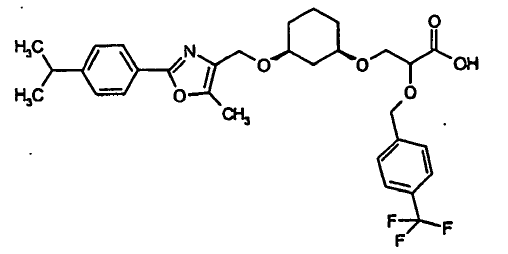

Angepaßt an die Reaktionsbedingungen des Beispiels 28 erhält man aus (2R/2S)-3-[(1R,3S)-1-Hydroxycyclohex-1-yloxy]-2-(4-trifluormethylbenzyloxy)-tert-butyldimethylsilylether und 5-methyl-2-(4-isopropylphenyl)-oxazol-4-ylmethyliodid (R)-3-[(1R,3S)-3-(5-Methyl-2-(4-isopropylphenyl)-oxazol-4-ylmethoxy)-cyclohexyloxy]-2-(4-trifluoromethylbenzyloxy)-propionsäure.

### Beispiel 80:

### (R)-3-[(1R,3S)-3-(5-Methyl-2-(2-naphthyl)-oxazol-4-ylmethoxy)-cyclohexyloxy]-2-(4-trifluoromethylbenzyloxy)-propionsäure

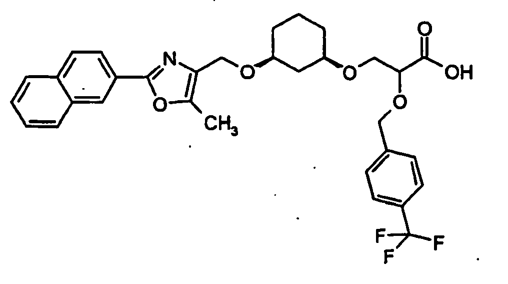

Angepaßt an die Reaktionsbedingungen des Beispiels 28 erhält man aus (2R/2S)-3-[(1R,3S)-1-Hydroxycyclohex-1-yloxy]-2-(4-trifluormethylbenzyloxy)-tert-butyldimethylsilylether und 5-methyl-2-(2-naphthyl)-oxazol-4-ylmethyliodid (R)-3-[(1R,3S)-3-(5-Methyl-2-(2-naphthyl)-oxazol-4-ylmethoxy)-cyclohexyloxy]-2-(4-trifluoromethylbenzyloxy)-propionsäure.

### Beispiel 81:

### (R)-3-[(1R,3S)-3-(5-Methyl-2-(4-methylphenyl)-oxazol-4-ylmethoxy)-cyclohexyloxy]-2-(4-trifluoromethylbenzyloxy)-propionsäure

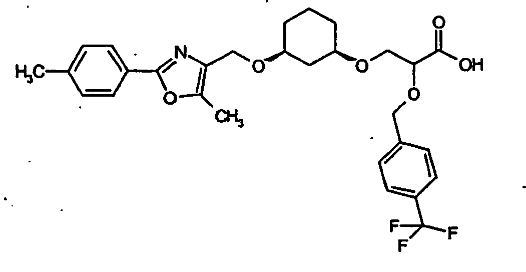

Angepaßt an die Reaktionsbedingungen des Beispiels 28 erhält man aus (2R/2S)-3-[(1R,3S)-1-Hydroxycyclohex-1-yloxy]-2-(4-trifluormethylbenzyloxy)-tert-butyldimethylsilylether und 5-methyl-2-(4-methylphenyl)-oxazol-4-ylmethyliodid (R)-3-[(1R,3S)-3-(5-Methyl-2-(4-methylphenyl)-oxazol-4-ylmethoxy)-cyclohexyloxy]-2-(4-trifluoromethylbenzyloxy)-propionsäure.

### Beispiel 82:

### (R)-3-[(1R,3S)-3-(5-Methyl-2-(4-trifluormethoxyphenyl)-oxazol-4-ylmethoxy)-cyclohexyloxy]-2-(4-trifluoromethylbenzyloxy)-propionsäure

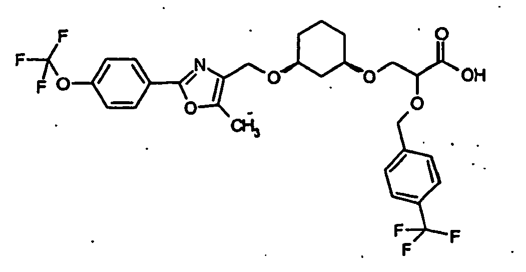

Angepaßt an die Reaktionsbedingungen des Beispiels 28 erhält man aus (2R/2S)-3-[(1R,3S)-1-Hydroxycyclohex-1-yloxy]-2-(4-trifluormethylbenzyloxy)-tert-butyldimethylsilylether und 5-methyl-2-(4-trifluormethoxyphenyl)-oxazol-4-ylmethyliodid (R)-3-[(1R,3S)-3-(5-Methyl-2-(4-trifluormethoxyphenyl)-oxazol-4-ylmethoxy)-cyclohexyloxy]-2-(4-trifluoromethylbenzyloxy)-propionsäure.

### Beispiel 83:

### (R)-3-[(1R,3S)-3-(5-Methyl-2-(3-trifluormethylphenyl)-oxazol-4-ylmethoxy)-cyclohexyloxy]-2-(4-trifluoromethylbenzyloxy)-propionsäure

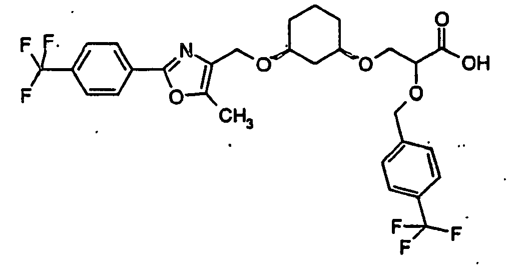

Angepaßt an die Reaktionsbedingungen des Beispiels 28 erhält man aus (2R/2S)-3-[(1R,3S)-1-Hydroxycyclohex-yloxy]-2-(4-trifluormethylbenzyloxy)-tert-butyldimiethylsilylether und 5-methyl-2-(3-trifluormethylphenyl)-oxazol-4-ylmethyliodid (R)3-[(1R,35)-3-(5-Methyl-2-(3-trifluormethylphenyl)-oxazol-4-ylmethoxy)-cyclohexyloxy]-2-(4-trifluoromethylbenzyloxy)-propionsäure.

### Beispiel 84:

### (R)3-[(1R,3S)-3-(5-Methyl-2-(3-methoxyphenyl)-oxazol-4-ylmethoxy)-cyclohexyloxy]-2-(4-trifluoromethylbenzyloxy)-propionsäure

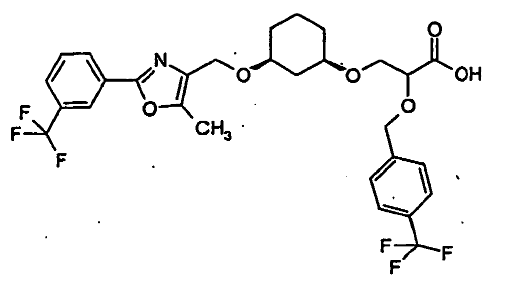

Angepaßt an die Reaktionsbedingungen des Beispiels 28 erhält man aus (2R/2S)-3-[(1R,3S)-1-Hydroxycyclohex-9-yloxy]-2-(4-trifluormethylbenzyloxy)-tert-butyldimethylsilylether und 5-methyl-2-(3-methoxyphenyl)-oxazol-4-ylmethyliodid (R)-3-[(1R,3S)-3-(5-Methyl-2-(3-methoxyphenyl)-oxazol-4-ylmethoxy)-cyclohexyloxy]-2-(4-trifluoromethylbenzyloxy)-propionsäure.

### Beispiel 85:

### 3-{(1R,3S)-3-[5-Methyl-2-(3-trifluormethylphenyl)-oxazol-4-ylmethoxy]-cyclohexyloxy}-2-isobutoxy-propionsäure

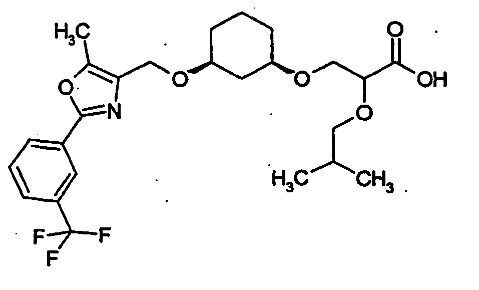

Angepaßt an die Reaktionsbedingungen des Beispiels 28 erhält man aus (2R/2S)-3-[(1S,3R)-3-Hydroxycyclohexyloxy]-2-isobutoxypropyl-tert-butyldiphenylsilylether und 2-(3-trifluormethylphenyl)-oxazol-4-ylmethyliodid 3-{(1R,3S)-3-[5-Methyl-2-(3-trifluormethylphenyl)-oxazol-4-ylmethoxy]-cyclohexyloxy}-2-isobutoxy-propionsäure.

### Beispiel 86:

### 3-{(1R,3S)-3-[5-Methyl-2-(3-methoxyphenyl)-oxazol-4-ylmethoxy]-cyclohexyloxy}-2-isobutoxypropionsäure

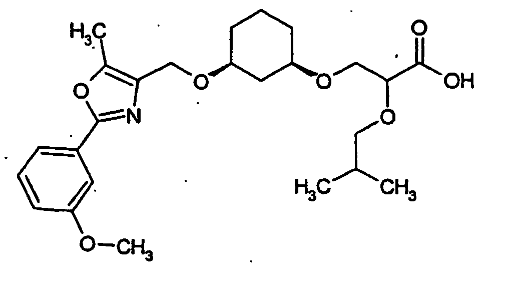

Angepaßt an die Reaktionsbedingungen des Beispiels 28 erhält man aus (2R/2S)-3-[(1S,3R)-3-Hydroxycyclohexyloxy]-2-isobutoxypropyl-tert-butyldiphenylsilylether und 2-(3-methoxyphenyl)-oxazol-4-ylmethyliodid 3-{(1R,3S)-3-[5-Methyl-2-(3-methoxyphenyl)-oxazol-4-ylmethoxy]-cyclohexyloxy}-2-isobutoxypropionsäure.

### Beispiel 87:

### 3-{(1R,3S)-3-[5-Methyl-2-(4-methylphenyl)-oxazol-4-ylmethoxy]-cyclohexyloxy}-2-isobutoxy-propionsäure

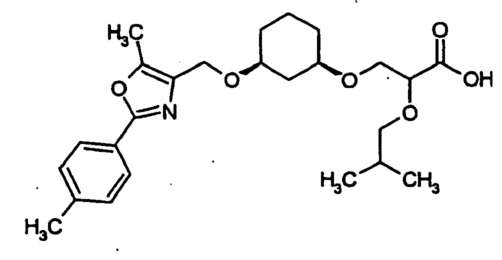

Angepaßt an die Reaktionsbedingungen des Beispiels 28 erhält man aus (2R/2S)-3-[(1S,3R)-3-Hydroxycyclohexyloxy]-2-isobutoxypropyl-tert-butyldiphenylsilylether und 2-(4-methylphenyl)-oxazol-4-ylmethyliodid 3-{(1R,3S)-3-[5-Methyl-2-(4-methylphenyl)-oxazol-4-ylmethoxy]-cyclohexyloxy}-2-isobutoxy-propionsäure.

### Beispiel 88:

### 3-{(1R,3S)-3-[5-Methyl-2-(4-isopropylphenyl)-oxazol-4-ylmethoxy]-cyclohexyloxy}-2-isobutoxy-propionsäure

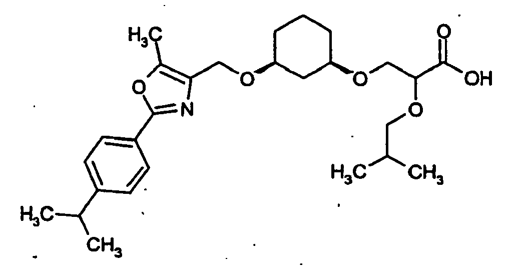

Angepaßt an die Reaktionsbedingungen des Beispiels 28 erhält man aus 2R/2S)-3-[(1S,3R)-3-Hydroxycyclohexyloxy]-2-isobutoxypropyl-tert-butyldiphenylsilylether und 2-(4-isopropylphenyl)-oxazol-4-ylmethyliodid 3-{(1R,3S)-3-[5-Methyl-2-(4-isopropylphenyl)-oxazol-4-ylmethoxy]-cyclohexyloxy}-2-isobutoxy-propionsäure.

### Beispiel 89:

### (2R/2S)-3-[(1R,3S)-3-(5-Methyl-2-m-tolyl-oxazol-4-ylmethoxymethyl)-cyclohexyloxy]-2-(4-trifluoromethylbenzyloxy)-propionsäure

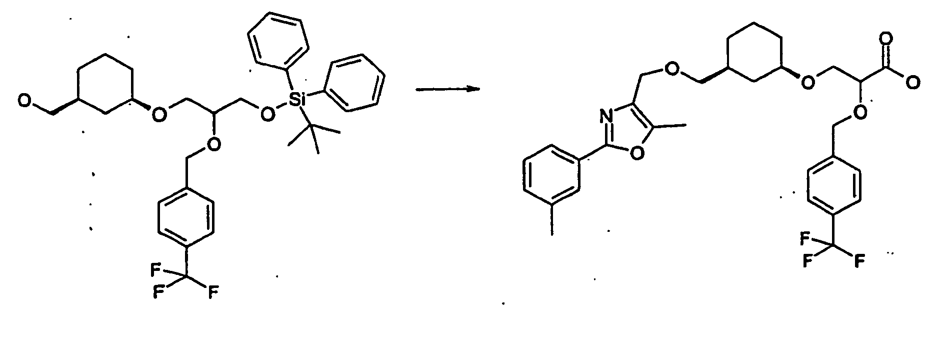

333 mg (2R/2S)3-[(1R,3S)-3-Hydroxymethylcyclohexyloxy]-2-(4-trifluormethylbenzyloxy)-propyl-tert-butyldiphenylsilylether werden in MTBE gelöst und nacheinander mit 125 mg Kalium-tert-butylat und mit 347 mg 5-Methyl-2-(3-methylphenyl)-oxazol-4-ylmethyliodid versetzt und bei RT gerührt. Nach vollständiger Umsetzung werden Wasser und MTBE zugesetzt, die organische Phase wird abgetrennt, über MgSO4 getrocknet und eingeengt. Der Rückstand wird an Kieselgel chromatographiert (Flash-Master, Heptan/Ethylacetat 1:0 -> 1:1 -> 0:1). Die produkthaltigen Fraktionen werden eingeengt, der Rückstand (420 mg) in 10 ml THF gelöst und mit 174 mg TBAF versetzt. Nach 72 h Rühren werden Wasser und MTBE zugesetzt, die organische Phase wird abgetrennt, mit NaCl-Lösung gewaschen, über MgSO4 getrocknet und eingeengt. Der Rückstand wird in 2 ml Aceton gelöst, mit 0,5 ml 1,9M Jones Reagenz versetzt und über Nacht bei RT gerührt. Nach Zugabe von Wasser und MTBE wird die organische Phase abgetrennt, über MgSO4 getrocknet und eingeengt. Der Rückstand wird per HPLC gereinigt, wobei 200 mg (2R/2S)-3-[(1R,3S)-3-(5-Methyl-2-m-tolyl-oxazol-4-ylmethoxymethyl)-cyclohexyloxy]-2-(4-trifluoromethylbenzyloxy)-propionsäure erhalten werden. C30H34F3NO6 (561,6), MS (ESI): 562 (M + H+).

### (S)-3-[(1R,3S)-3-(5-Methyl-2-m-tolyl-oxazol-4-ylmethoxymethyl)-cyclohexyloxy]-2-(4-trifluoromethyl-benzyloxy)-propionsäure

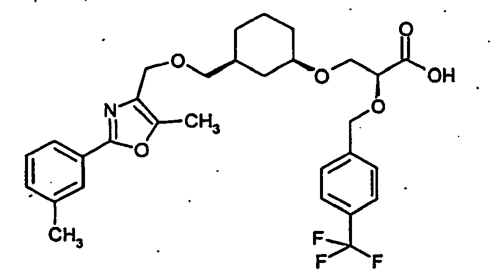

Durch Trennung von (2R/2S)-3-[(1R,3S)-3-(5-Methyl-2-m-tolyl-oxazol-4-ylmethoxymethyl)-cyclohexyloxy]-2-(4-trifluoromethylbenzyloxy)-propionsäure per chiraler HPLC erhält man (2S)-3-[(1R,3S)-3-(5-Methyl-2-m-tolyl-oxazol-4-ylmethoxymethyl)-cyclohexyloxy]-2-(4-trifluoromethylbenzyloxy)-propionsäure. C30H34F3NO6 (561,6), MS (ESI): 562 (M + H+).

### Beispiel 90:

### (R)-3-[(1R,3S)-3-(5-Methyl-2-m-tolyl-oxazol-4-ylmethoxymethyl)-cyclohexyloxy]-2-(4-trifluoromethyl-benzyloxy)-propionsäure

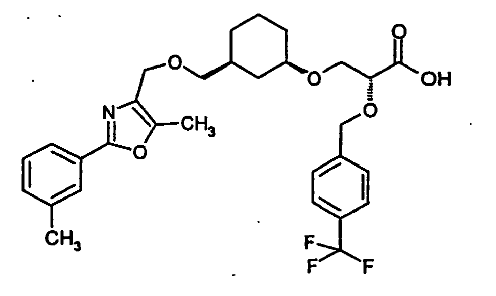

Durch Trennung von (2R/2S)-3-[(1R,3S)3-(5-Methyl-2-m-tolyl-oxazol-4-ylmethoxymethyl)-cyclohexyloxy]-2-(4-trifluoromethylbenzyloxy)-propionsäure per chiraler HPLC erhält man (2R)3-[(1R,3S)-3-(5-Methyl-2-m-tolyl-oxazol-4-ylmethoxymethyl)-cyclohexyloxy]-2-(4-trifluoromethylbenzyloxy)-propionsäure. C30H34F3NO6 (561,6), MS (ESI): 562 (M + H+).

### Beispiel 91:

### 2-[(1R,3S)-3-(5-Methyl-2-m-tolyl-oxazol-4-ylmethoxy)-cyclohexyloxymethyl]-3-(3-trifluoromethyl-phenyl)-propionsäure

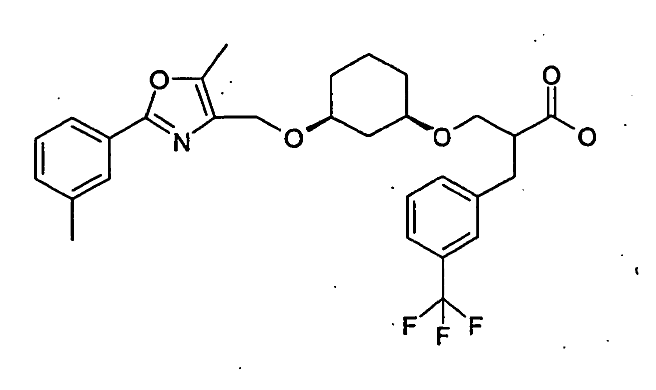

Analog zu Beipiel 9 erhält man aus ((1R,3S)-2-[3-(5-Methyl-2-m-tolyl-oxazol-4-ylmethoxy)-cyclohexyloxymethyl]-acrylsäureethylester und 3-Brombenzotrifluorid 2-[(1R,3S)-3-(5-Methyl-2-m-tolyl-oxazol-4-ylmethoxy)-cyclohexyloxymethyl]-3-(3-trifluoromethyl-phenyl)-propionsäure.

### Massenspektroskopische Charakterisierung der Beispiele:

| Beispiel | Summenformel | Monoisotop. Masse | gefunden per MS (ESI) als (M + H⁺) |
|---|---|---|---|
| 1 | C23H30FNO5 | 419.49 | 420 |
| 2 | C24H32FNO5 | 433,52 | 434 |
| 3 | C21H26FN05 | 391,44 | 392 |
| 4 | C22H28FNO5 | 405,47 | 406 |
| 5 | C23H29NO5 | 399.49 | 400 |
| 6 | C31H40N2O5 | 520.67 | 521 |
| 7 | C30H38N2O5 | 506.65 | 507 |
| 8 | C23H31N06 | 417.51 | 418 |
| 9 | C29H34FNO5 | 495.60 | 496 |
| 10 | C24H33NO6 | 431,53 | 432 |
| 11 | C29H32F3NO6 | 547,58 | 548 |
| 12 | C22H29NO6 | 403,48 | 404 |
| 13 | C29H32F3NO6 | 547,58 | 548 |
| 14 | C29H32F3NO6 | 547,58 | 548 |
| 15 | C29H35NO7 | 509,61 | 510 |
| 16 | C30H37NO6 | 507,63 | 508 |
| 17 | C29H35NO6 | 493,61 | 494 |
| 18 | C32H41 NO6 | 535,69 | 536 |
| 19 | C29H32F3NO6 | 547,58 | 548 |
| 20 | C26H30ClNO6S | 520,05 | 521 |
| 21 | C25H31 N06 | 441,53 | 442 |
| 22 | C24H29NO6 | 427,50 | 428 |
| 23 | C26H33NO6 | 455,56 | 456 |
| 24 | C24H31NO6 | 429,52 | 430 |
| 25 | C30H35NO6 | 505,62 | 506 |
| 26 | C25H33NO6 | 443,55 | 444 |
| 27 | C28H33NO6 | 479,58 | 480 |
| 28 | C21H27NO6 | 389,45 | 390 |
| 29 | C22H26F3NO6 | 457,45 | 458 |
| 30 | C22H26F3NO7 | 473,45 | 474 |
| 31 | C22H26F3NO6 | 457,45 | 458 |
| 32 | C22H29NO6 | 403,48 | 404 |
| 33 | C23H31NO6 | 417,51 | 418 |
| 34 | C22H26F3NO6 | 457,45 | 458 |
| 35 | C25H29NO6 | 439,51 | 440 |
| 36 | C23H31NO6 | 417,51 | 418 |
| 37 | C27H31NO6 | 465,55 | 466 |
| 38 | C23H31 N07 | 433,51 | 434 |
| 39 | C23H31NO6 | 417,51 | 418 |
| 40 | C23H28F3NO6 | 471,48 | 472 |
| 41 | C24H33N06 | 431,53 | 432 |
| 42 | C23H31NO6 | 417,51 | 418 |
| 43 | C24H33NO6 | 431,53 | 432 |
| 44 | C24H30F3NO7 | 501,50 | 502 |
| 45 | C24H33N07 | 447,53 | 448 |
| 46 | C24H30F3NO6 | 485,51 | 486 |
| 47 | C24H33NO6 | 431,53 | 432 |
| 48 | C24H30F3NO7 | 501,50 | 502 |
| 49 | C26H37NO6 | 459,59 | 460 |
| 50 | C24H30F3NO6 | 485,51 | 486 |
| 51 | C27H33NO6 | 467,57 | 468 |
| 52 | C24H30F3NO6 | 485,51 | 486 |
| 53 | C25H35NO7 | 461,56 | 462 |
| 54 | C25H35NO6 | 445,56 | 446 |
| 55 | C25H32F3NO6 | 499,53 | 500 |
| 56 | C26H37NO6 | 459,59 | 460 |
| 57 | C23H28F3NO6 | 471,48 | 472 |
| 58 | C23H28F3NO7 | 487,48 | 488 |
| 59 | C23H28F3NO6 | 471,48 | 472 |
| 60 | C23H28F3NO7 | 487,48 | 488 |
| 61 | C25H35NO6 | 445,56 | 446 |
| 62 | C23H28F3NO6 | 471,48 | 472 |
| 63 | C24H33NO6 | 431,53 | 432 |
| 64 | C28H33NO6 | 479,58 | 480 |
| 65 | C24H33NO6 | 431,53 | 432 |
| 66 | C26H37NO6 | 459,59 | 460 |
| 67 | C24H30F3NO6 | 485,51 | 486 |
| 68 | C25H35NO6 | 445,56 | 446 |
| 69 | C27H39NO6 | 473,62 | 474 |
| 70 | C22H29NO6 | 403,48 | 404 |
| 71 | C23H31NO6 | 417,51 | 418 |
| 72 | C23H31NO7 | 433,51 | 434 |
| 73 | C23H31NO6 | 417,51 | 418 |
| 74 | C26H31N06 | 453,54 | 454 |
| 75 | C24H33NO7 | 447,53 | 448 |
| 76 | C29H32F3NO6 | 547,58 | 548 |
| 77 | C29H29F6NO6 | 601,55 | 602 |
| 78 | C29H29F6NO7 | 617,55 | 618 |
| 79 | C31H36F3NO6 | 575,63 | 576 |
| 80 | C32H32F3NO6 | 583,61 | 584 |
| 81 | C29H32F3NO6 | 547,58 | 548 |
| 82 | C29H29F6NO7 | 617,55 | 618 |
| 83 | C29H29F6NO6 | 601,55 | 602 |
| 84 | C29H32F3NO7 | 563,58 | 564 |
| 85 | C25H32F3NO6 | 499,53 | 500 |
| 86 | C25H35NO7 | 461,56 | 462 |
| 87 | C25H35NO6 | 445,56 | 446 |
| 88 | C27H39NO6 | 473,62 | 474 |
| 89 | C30H34F3NO6 | 561,60 | 562 |
| 90 | C30H34F3NO6 | 561,60 | 562 |
| 91 | C29H32F3NO5 | 531.58 | 532 |

## Patentansprüche

1. Verbindungen der Formel I 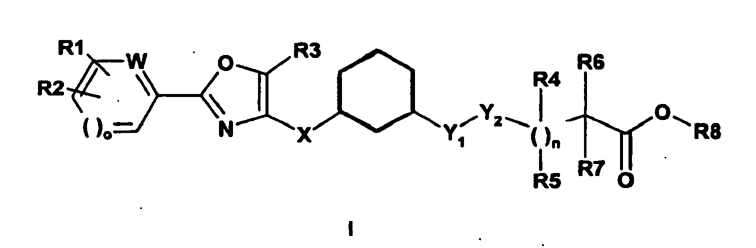 worin bedeuten:
R1, R2 unabhängig voneinander H, F, Cl, Br, CF₃, OCF₃, (C1-C6)-Alkyl, O-(C1-C6)-Alkyl, SCF₃, SF₅, OCF₂-CHF₂, (C6-C10)-Aryl, (C6-C10)-Aryloxy, OH, NO₂; oder
R1 und R2 zusammengenommen mit dem Phenyl-, Pyridin-, 1-H-Pyrrol-, Thiophen- oder Furan-Ring kondensiertes, teilweise oder nicht gesättigtes bicyclisches (C6-C10)-Aryl, (C5-C11)-Heteroaryl;
R3 H, (C1-C6)-Alkyl, (C3-C8)-Cycloalkyl, (C1-C3)-Alkyl-(C3-C8)-Cycloalkyl, Phenyl, (C1-C3)-Alkyl-Phenyl, (C5-C6)-Heteroaryl, (C1-C3)-Alkyl-(C5-C6)-Heteroaryl oder (C1-C3)-Alkyl, das durch F ganz oder teilweise substituiert ist;
o 0 oder 1;
W CH, N, falls o = 1;
W O, S, NR9, falls o = 0;
X (C1-C6)-Alkandiyl, wobei in der Alkandiylgruppe ein oder mehrere Kohlenstoffatome durch Sauerstoffatome ersetzt sein können;
Y1 O;
Y2 CR12R13, SO, SO2;
n 0 - 2;
R4 H, F, (C1-C6)-Alkyl;
R5 H, F, (C1-C6)-Alkyl;
R6 H, (C1-C6)-Alkyl; oder F, falls n ungleich 0;
R7 H, (C1-C6)-Alkyl, (C2-C6)-Alkenyl, (C2-C6)-Alkinyl, O-(C1-C6)-Alkyl, O-(C2-C6)-Alkenyl, O-(C2-C6)-Alkinyl, (C3-C8)-Cycloalkyl, Phenyl, (C5-C11)-Heteroaryl, O-(C3-C8)-Cycloalkyl, O-Phenyl, die substituiert sein können durch OH, NR10R11, (C1-C6)-Alkyl. (C2-C6)-Alkenyl, (C2-C6)-Alkinyl, (C3-C8)-Cycloalkyl, Phenyl, (C5-C11)-Heteroaryl, und wobei (C3-C8)-Cycloalkyl, Phenyl, (C5-C11)-Heteroaryl zusätzlich substituiert sein können durch (C1-C6)-Alkyl gegebenenfalls ganz oder teilweise substituiert durch F, O-(C1-C6)-Alkyl gegebenenfalls ganz oder teilweise substituiert durch F, Cl, Br, J, OH, NR10R11, CO-(C1-C6)-Alkyl, CO-(C6-C10)-Aryl, CO-(C1-C6)-Alkyl-(C6-C10)-Aryl, CO-(C5-C11)-Heteroaryl, C(O)-O-(C1-C6)-Alkyl, C(O)-O-(C1-C6)-Alkyl-(C6-C10)-Aryl, C(O)-O-(C6-C10)-Aryl, C(O)-O-(C5-C11)-Heteroaryl, S02-(C1-C6)-Alkyl, SO2-(C1-C6)Alkyl-(C6-C10)-Aryl, SO2-(C1-C6)-Alkyl-SO2-(C1-C6)-alkyl, SO2-(C6-C10)-Aryl, SO2-(C5-C11)-Heteroaryl;
R6 und R7 zusammen mit dem sie tragenden C-Atom (C3-C8)-Cycloalkyl;
R8 H, (C1-C6)-Alkyl;
R9 H, (C1-C6)-Alkyl, das gegebenenfalls substituiert ist durch Phenyl;
R10 H, (C1-C6)-Alkyl, das gegebenenfalls substituiert ist durch Phenyl;
R11 H, (C1-C6)-Alkyl, das gegebenenfalls substituiert ist durch Phenyl;
R12 H, (C1-C6)-Alkyl;
R13 H, (C1-C6)-Alkyl;
sowie deren physiologisch verträgliche Salze.

2. Verbindungen der Formel I gemäß Anspruch 1, worin bedeuten:
X (C1-C6)-Alkandiyl, wobei in der Alkandiylgruppe das C1- oder C2-Kohlenstoffatom (zum Cyclohexandiyl-Ring) durch Sauerstoffatom ersetzt ist;
sowie deren physiologisch verträgliche Salze.

3. Verbindungen der Formel I gemäß einem oder mehreren der Ansprüche 1 bis 2, worin bedeuten
R1, R2 unabhängig voneinander H, F, CF3, (C1-C6)-Alkyl, O-(C1-C6)-Alkyl, Phenyl, oder
R1 und R2 zusammengenommen mit dem Phenylring = Naphthyl;
R3 (C1-C6)-Alkyl;
W CH, falls o = 1;
X (CH2)O, CH2-O-CH2;
Y1 O;
Y2 CH2;
n 0, 1;
R4 H;
R5 H;
R6 . H;
R7 H, (C1-C6)-Alkyl, O-(C1-C6)-Alkyl, (C1-C6)-Alkyl-O-(C1-C6)-Alkyl, O-(C2-C6)-Alkenyl, O-(C2-C6)-Alkinyl, CH2NR10R11, wobei Alkyl, O-Alkyl und Alkenyl substituiert sein kann durch Phenyl oder (C5-C6)-Heteroaryl, die wiederum substituiert sein können durch (C1-C6)-Alkyl. O-(C1-C6)-Alkyl, CF3;
R6 und R7 zusammen mit dem sie tragenden C-Atom (C3-C6)-Cycloalkyl;
R8 H;
R10 (C1-C6)-Alkyl;
R11 (C1-C6)-Alkyl, das durch Phenyl substituiert ist;
sowie deren physiologisch verträgliche Salze.

4. Arzneimittel enthaltend eine oder mehrere der Verbindungen der Formel I gemäß einem oder mehreren der Ansprüche 1 bis 3.

5. Arzneimittel enthaltend eine oder mehrere der Verbindungen der Formel I gemäß einem oder mehreren der Ansprüche 1 bis 3 und einen oder mehrere Wirkstoffe, die günstige Wirkungen auf Stoffwechselstörungen oder damit assozierte Erkrankungen haben.

6. Arzneimittel enthaltend eine oder mehrere der Verbindungen der Formel I gemäß einem oder mehreren der Ansprüche 1 bis 3 und einen oder mehrere Antidiabetika.

7. Arzneimittel enthaltend eine oder mehrere der Verbindungen der Formel I gemäß einem oder mehreren der Ansprüche 1 bis 3 und einen oder mehrere Lipidmodulatoren.

8. Verwendung der Verbindungen der Formel I gemäß einem oder mehreren der Ansprüche 1 bis 3 zur Herstellung eines Arzneimittels zur Behandlung und/oder Prävention von Störungen des Fettsäurestoffwechsels und Glucoseverwertungsstörungen.

9. Verwendung der Verbindungen der Formel I gemäß einem oder mehreren der Ansprüche 1 bis 3 zur Herstellung eines Arzneimittels zur Behandlung und/oder Prävention von Störungen, bei denen Insulin Resistenz eine Rolle spielt.

10. Verwendung der Verbindungen der Formel I gemäß einem oder mehreren der Ansprüche 1 bis 3 zur Herstellung eines Arzneimittels zur Behandlung und/oder Prävention von Diabetes mellitus und der damit verbundenen Folgeerkrankungen.

11. Verwendung der Verbindungen der Formel I gemäß einem oder mehreren der Ansprüche 1 bis 3 zur Herstellung eines Arzneimittels zur Behandlung und/oder Prävention von Dyslipidämien und deren Folgen.

12. Verwendung der Verbindungen der Formel I gemäß einem oder mehreren der Ansprüche 1 bis 3 zur Herstellung eines Arzneimittels zur Behandlung und/oder Prävention von Zuständen, die mit dem Metabolischen Syndrom assoziert sind.

13. Verwendung der Verbindungen gemäß einem oder mehreren der Ansprüche 1 bis 3 in Kombination mit mindestens einem weiteren Wirkstoff zur Herstellung eines Arzneimittels zur Behandlung und/oder Prävention von Störungen des Fettsäurestoffwechsels und Glucoseverwertungsstörungen.

14. Verwendung der Verbindungen gemäß einem oder mehreren der Ansprüche 1 bis 3 in Kombination mit mindestens einem weiteren Wirkstoff zur Herstellung eines Arzneimittels zur Behandlung und/oder Prävention von Störungen, bei denen Insulin Resistenz eine Rolle spielt.

15. Verfahren zur Herstellung eines Arzneimittels enthaltend eine oder mehrere der Verbindungen gemäß einem oder mehreren der Ansprüche 1 bis 3, **dadurch gekennzeichnet, dass** der Wirkstoff mit einem pharmazeutisch geeigneten Träger vermischt wird und diese Mischung in eine für die Verabreichung geeignete Form gebracht wird.

## Claims

1. A compound of the formula I 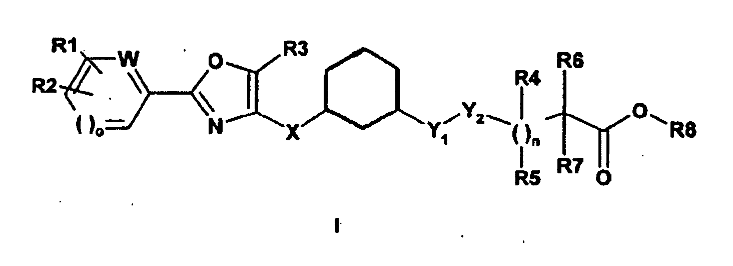 in which:
R1, R2 independently of one another are H, F, Cl, Br, CF₃, OCF₃, (C1-C6)-alkyl, O-(C1-C6)-alkyl, SCF₃, SF₅, OCF₂-CHF₂, (C6-C10)-aryl, (C6-C10)-aryloxy, OH, NO₂; or
R1 and R2 together with the phenyl, pyridine, 1H-pyrrole, thiophene or furan ring form fused, partially or unsaturated bicyclic (C6-C10)-aryl, (C5-C11)-heteroaryl;
R3 is H, (C1-C6)-alkyl, (C3-C8)cycloalkyl, (C1-C3)-alkyl-(C3-C8)cycloalkyl, phenyl, (C1-C3)-alkyl-phenyl, (C5-C6)-heteroaryl, (C1-C3)-alkyl-(C5-C6)-heteroaryl or (Cl-C3)-alkyl which is fully or partially substituted by F;
O is 0 or 1;
W is CH or N if o = 1;
W is O, S or NR9 if o = 0;
X is (C1-C6)-alkanediyl, where in the alkanediyl group one or more carbon atoms may be replaced by oxygen atoms;
Y1 is O;
Y2 is CR12R13, SO, SO2;
n is 0-2;
R4 is H, F, (C1-C6)-alkyl;
R5 is H, F, (C1-C6)-alkyl;
R6 is H, (C1-C6)-alkyl; or F if n is not 0;
R7 is H, (C1-C6)-alkyl, (C2-C6)-alkenyl, (C2-C6)-alkynyl, O-(C1-C6)-alkyl, O-(C2-C6)-alkenyl, O-(C2-C6)-alkynyl, (C3-C8)-cycloalkyl, phenyl, (C5-C11)-heteroaryl, O-(C3-C8)-cycloalkyl, O-phenyl, which may be substituted by OH, NR10R11, (C1-C6)-alkyl, (C2-C6)-alkenyl, (C2-C6)-alkynyl, (C3-C8)-cycloalkyl, phenyl, (C5-C11)-heteroaryl, and where (C3-C8)-cycloalkyl, phenyl, (C5-C11)-heteroaryl may additionally be substituted by (C1-C6)-alkyl, optionally fully or partially substituted by F, O-(C1-C6)-alkyl, optionally fully or partially substituted by F, Cl, Br, I, OH, NR10R11, CO-(C1-C6)-alkyl, CO-(C6-C10)-aryl, CO-(C1-C6)-alkyl-(C6-C10)-aryl, CO-(C5-C11)-heteroaryl, C(O)-O-(C1-C6)-alkyl, C(O)-O-(C1-C6)-alkyl-(C6-C10)-aryl, C(O)-O-(C6-C10)-aryl, C(O)-O-(C5-C11)-heteroaryl, SO2-(C1-C6)-alkyl, SO2-(C1-C6)-alkyl-(C6-C10)-aryl, SO2-(C1-C6)-alkyl-SO2-(C1-C6)-alkyl, SO2-(C6-C10)-aryl, SO2-(C5-C11)-heteroaryl;
R6 and R7 together with the carbon atom that carries them are (C3-C8)-cycloalkyl;
R8 is H, (C1-C6)-alkyl;
R9 is H, (C1-C6)-alkyl, which is unsubstituted or substituted by phenyl;
R10 is H, (C1-C6)-alkyl, which is unsubstituted or substituted by phenyl;
R11 is H, (C1-C6)-alkyl, which is unsubstituted or substituted by phenyl;
R12 is H, (C1-C6)-alkyl;
R13 is H, (C1-C6)-alkyl;
and its physiologically acceptable salts.

2. A compound of the formula I as claimed in claim 1 in which:
X is (C1-C6)-alkanediyl, where in the alkanediyl group the C1 or C2 carbon atom (to cyclohexanediyl ring) is replaced by an oxygen atom;
and its physiologically acceptable salts.

3. A compound of the formula I as claimed in one or more of claims 1 to 2, in which
R1, R2 independently of one another are H, F, CF3, (C1-C6)-alkyl, O-(C1-C6)-alkyl, phenyl, or
R1 and R2 together with the phenyl ring = naphthyl;
R3 is (C1-C6)-alkyl;
W is CH if o = 1;
X is (CH2)O, CH2-O-CH2;
Y1 is O;
Y2 is CH2;
n is 0, 1;
R4 is H;
R5 is H;
R6 is H;
R7 is H, (C1-C6)-alkyl, O-(C1-C6)-alkyl, (C1-C6)-alkyl-O-(C1-C6)-alkyl , O-(C2-C6) -alkenyl, O-(C2-C6)-alkynyl, CH2NR10R11, where alkyl, O-alkyl and alkenyl may be substituted by phenyl or (C5-C6)-heteroaryl, which in turn may be substituted by (C1-C6)-alkyl, O-(C1-C6)-alkyl, CF3;
R6 and R7 together with the carbon atom that carries them are (C3-C6)-cycloalkyl;
R8 is H;
R10 is (C1-C6)-alkyl;
R11 is (C1-C6)-alkyl which is substituted by phenyl;
and its physiologically acceptable salts.

4. A pharmaceutical, comprising one or more compounds of the formula I as claimed in one or more of claims 1 to 3.

5. A pharmaceutical, comprising one or more compounds of the formula I as claimed in one or more of claims 1 to 3 and one or more active compounds having favorable effects on metabolic disorders or diseases associated therewith.

6. A pharmaceutical, comprising one or more compounds of the formula I as claimed in one or more of claims 1 to 3 and one or more antidiabetics.

7. A pharmaceutical, comprising one or more compounds of the formula I as claimed in one or more of claims 1 to 3 and one or more lipid modulators.

8. The use of the compounds of the formula I as claimed in one or more of claims 1 to 3 for preparing a pharmaceutical for the treatment and/or prevention of disorders of the fatty acid metabolism and glucose utilization disorders.

9. The use of the compounds of the formula I as claimed in one or more of claims 1 to 3 for preparing a pharmaceutical for the treatment and/or prevention of disorders where insulin resistance is involved.

10. The use of the compounds of the formula I as claimed in one or more of claims 1 to 3 for preparing a pharmaceutical for the treatment and/or prevention of diabetes mellitus and its sequelae.

11. The use of the compounds of the formula I as claimed in one or more of claims 1 to 3 for preparing a pharmaceutical for the treatment and/or prevention of dyslipidemias and their sequelae.

12. The use of the compounds of the formula I as claimed in one or more of claims 1 to 3 for preparing a pharmaceutical for the treatment and/or prevention of states associated with metabolic syndrome.

13. The use of the compounds as claimed in one or more of claims 1 to 3 in combination with at least one further active compound for preparing a pharmaceutical for the treatment and/or prevention of disorders of the fatty acid metabolism and glucose utilization disorders.

14. The use of the compounds as claimed in one or more of claims 1 to 3 in combination with at least one further active compound for preparing a pharmaceutical for the treatment and/or prevention of disorders in which insulin resistance is involved.

15. A process for preparing a pharmaceutical comprising one or more of the compounds as claimed in one or more of claims 1 to 3, which comprises mixing the active compound with a pharmaceutically acceptable carrier and bringing this mixture into a form suitable for administration.

## Revendications

1. Composés de formule I 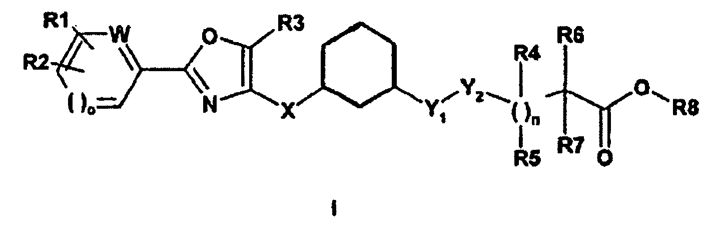 dans laquelle
R1, R2 signifient, indépendamment l'un de l'autre, H, F, Cl, Br, CF₃, OCF₃, (C₁-C₆) -alkyle, O-(C₁-C₆)-alkyle, SCF₃, SF₅, OCF₂-CHF₂, (C₆-C₁₀)-aryle, (C₆-C₁₀)-aryloxy, OH, NO₂ ; ou
R1 et R2 pris ensemble avec le cycle phényle, pyridine, 1-H-pyrrole, thiophène ou furanne, signifient (C₆-C₁₀)-aryle, (C₅-C₁₁)-hétéroaryle bicyclique condensé, partiellement saturé ou non saturé ;
R3 signifie H, (C₁-C₆)-alkyle, (C₃-C₈) -cycloalkyle, (C₁-C₃) -alkyl- (C₃-C₈) -cycloalkyle, phényle, (C₁-C₃)-alkyl-phényle, (C₅-C₆) -hétéroaryle, (C₁-C₃)-alkyl-(C₅-C₆)-hétéroaryle ou (C₁-C₃)-alkyle, qui est totalement ou partiellement substitué par F ;
o vaut 0 ou 1 ;
W signifie CH, N, si o = 1 ;
W signifie O, S, NR9, si o = 0 ;
X signifie (C₁-C₆)-alcanediyle, où dans le groupe alcanediyle, un ou plusieurs atomes de carbone peuvent être remplacés par des atomes d'oxygène ;
Y1 signifie O ;
Y2 signifie CR12R13, SO, SO₂ ;
n vaut 0-2 ;
R4 signifie H, F, (C₁-C₆)-alkyle ;
R5 signifie H, F, (C₁-C₆)-alkyle ;
R6 signifie H, (C₁-C₆)-alkyle ; ou F, si n est différent de 0 ;
R7 signifie H, (C₁-C₆) -alkyle, (C₂-C₆)-alcényle, (C₂-C₆)-alcynyle, O-(C₁-C₆)-alkyle, O-(C₂-C₆)-alcényle, O-(C₂-C₆) -alcynyle, (C₃-C₈)-cycloalkyle, phényle, (C₅-C₁₁)-hétéroaryle, O-(C₃-C₈)-cycloalkyle, O-phényle, qui peuvent être substitués par OH, NR10R11, (C₁-C₆) -alkyle, (C₂-C₆) -alcényle, (C₂-C₆)-alcynyle, (C₃-C₈) -cycloalkyle, phényle, (C₅-C₁₁)-hétéroaryle, et où (C₃-C₈)-cycloalkyle, phényle, (C₅-C₁₁) -hétéroaryle peuvent en outre être substitués par (C₁-C₆) -alkyle le cas échéant substitué totalement ou partiellement par F, O-(C₁-C₆)-alkyle le cas échéant substitué totalement ou partiellement par F, Cl, Br, I, OH, NR10R11, CO-(C₁-C₆)-alkyle, CO-(C₆-C₁₀)-aryle, CO-(C₁-C₆)-alkyl-(C₆-C₁₀)-aryle, CO-(C₅-C₁₁)-hétéroaryle, C(O)-O-(C₁-C₆)-alkyle, C(O)-O-(C₁-C₆)-alkyl-(C₆-C₁₀)-aryle, C(O)-O-(C₆-C₁₀)-aryle, C(O)-O-(C₅-C₁₁)-hétéroaryle, SO₂-(C₁-C₆)-alkyle, SO₂-(C₁-C₆)-alkyl-(C₆-C₁₀)-aryle, SO₂-(C₁-C₆)-alkyl-SO₂-(C₁-C₆)-alkyle, SO₂-(C₆-C₁₀)-aryle, SO₂-(C₅-C₁)-hétéroaryle ;
R6 et R7 signifient, ensemble avec l'atome de C qui les porte, (C₃-C₈)-cycloalkyle ;
R8 signifie H, (C₁-C₆)-alkyle ;
R9 signifie H, (C₁-C₆)-alkyle, qui est le cas échéant substitué par phényle ;
R10 signifie H, (C₁-C₆)-alkyle-, qui est le cas échéant substitué par phényle ;
R11 signifie H, (C₁-C₆)-alkyle, qui est le cas échéant substitué par phényle ;
R12 signifie H, (C₁-C₆)-alkyle ;
R13 signifie H, (C₁-C₆)-alkyle ;
ainsi que leurs sels physiologiquement acceptables.

2. Composés de formule I selon la revendication 1, dans laquelle
X signifie (C₁-C₆)-alcanediyle, où dans le groupe alcanediyle, l'atome de carbone C₁ ou C₂ (par rapport au cycle cyclohexanediyle) est remplacé par un atome d'oxygène ;
ainsi que leurs sels physiologiquement acceptables.

3. Composés de formule I selon l'une ou plusieurs des revendications 1 à 2, dans laquelle
R1, R2 signifient, indépendamment l'un de l'autre H, F, CF₃, (C₁-C₆)-alkyle, O-(C₁-C₆)-alkyle, phényle, ou
R1 et R2 ensemble avec le cycle phényle, signifient naphtyle ;
R3 signifie (C₁-C₆)-alkyle ;
W signifie CH, si o = 1 ;
X signifie (CH₂)O, CH₂-O-CH₂ ;
Y1 signifie O ;
Y2 signifie CH₂ ;
n vaut 0, 1 ;
R4 signifie H ;
R5 signifie H ;
R6 signifie H ;
R7 signifie H, (C₁-C₆)-alkyle, O-(C₁-C₆)-alkyle, (C₁-C₆)-alkyl-O-(C₁-C₆) -alkyle, O- (C₂-C₆)-alcényle, O-(C₂-C₆)-alcynyle, CH₂NR10R11, où alkyle, O-alkyle et alcényle peuvent être substitués par phényle ou (C₅-C₆)-hétéroaryle, qui peuvent être substitués à leur tour par (C₁-C₆)-alkyle, O-(C₁-C₆)-alkyle, CF₃ ;
R6 et R7 signifient, ensemble avec l'atome de C qui les porte, (C₃-C₆)-cycloalkyle ;
R8 signifie H ;
R10 signifie (C₁-C₆)-alkyle ;
R11 signifie (C₁-C₆)-alkyle, qui est substitué par phényle ;
ainsi que leurs sels physiologiquement acceptables.

4. Médicament contenant un ou plusieurs des composés de formule I selon l'une ou plusieurs des revendications 1 à 3.

5. Médicament contenant un ou plusieurs des composés de formule I selon l'une ou plusieurs des revendications 1 à 3 et une ou plusieurs substances actives, qui ont des effets favorables sur les troubles du métabolisme ou les maladies qui y sont associées.

6. Médicament contenant un ou plusieurs des composés de formule I selon l'une ou plusieurs des revendications 1 à 3 et un ou plusieurs antidiabétiques.

7. Médicament contenant un ou plusieurs des composés de formule I selon l'une ou plusieurs des revendications 1 à 3 et un ou plusieurs modulateurs de lipides.

8. Utilisation des composés de formule I selon l'une ou plusieurs des revendications 1 à 3 pour la préparation d'un médicament destiné au traitement et/ou à la prévention de troubles du métabolisme des acides gras et de troubles de l'utilisation du glucose.

9. Utilisation des composés de formule I selon l'une ou plusieurs des revendications 1 à 3 pour la préparation d'un médicament destiné au traitement et/ou à la prévention de troubles dans lesquels la résistance à l'insuline joue un rôle.

10. Utilisation des composés de formule I selon l'une ou plusieurs des revendications 1 à 3 pour la préparation d'un médicament destiné au traitement et/ou à la prévention du diabète sucré et des maladies secondaires qui y sont liées.

11. Utilisation des composés de formule I selon l'une ou plusieurs des revendications 1 à 3 pour la préparation d'un médicament destiné au traitement et/ou à la prévention de dyslipidémies et de leurs conséquences.

12. Utilisation des composés de formule I selon l'une ou plusieurs des revendications 1 à 3 pour la préparation d'un médicament destiné au traitement et/ou à la prévention d'états qui sont associés au syndrome métabolique.

13. Utilisation des composés selon l'une ou plusieurs des revendications 1 à 3 en combinaison avec au moins une autre substance active pour la préparation d'un médicament destiné au traitement et/ou à la prévention de troubles du métabolisme des acides gras et de troubles de l'utilisation du glucose.

14. Utilisation des composés selon l'une ou plusieurs des revendications 1 à 3 en combinaison avec au moins une autre substance active pour la préparation d'un médicament destiné au traitement et/ou à la prévention de troubles dans lesquels la résistance à l'insuline joue un rôle.

15. Procédé pour la préparation d'un médicament contenant un ou plusieurs des composés selon l'une ou plusieurs des revendications 1 à 3, **caractérisé en ce que** la substance active est mélangée avec un support pharmaceutiquement approprié et ce mélange est amené dans une forme appropriée pour l'administration.
